(19) Europäisches Patentamt
European Patent Office
Office européen des brevets

(11) Publication number: **0 409 634 A2**

(12) **EUROPEAN PATENT APPLICATION**

(21) Application number: **90307935.8**

(22) Date of filing: **19.07.90**

(51) Int. Cl.[5]: **C07D 213/26**, C09K 19/34, C07D 213/06, C07D 213/64, C07D 213/30, C07D 237/08, C09K 19/12, C09K 19/18, C09K 19/14, C07D 237/14, C07D 213/65

(30) Priority: **20.07.89 JP 188492/89**
**20.07.89 JP 188493/89**
**20.07.89 JP 188494/89**
**20.07.89 JP 188495/89**
**28.09.89 JP 253502/89**
**28.09.89 JP 253503/89**
**06.11.89 JP 289267/89**
**22.11.89 JP 303849/89**
**22.11.89 JP 303850/89**
**22.11.89 JP 303852/89**
**22.11.89 JP 303853/89**
**27.11.89 JP 307070/89**
**13.12.89 JP 323505/89**

(43) Date of publication of application:
**23.01.91 Bulletin 91/04**

(84) Designated Contracting States:
**CH DE FR GB IT LI NL**

(71) Applicant: **SANYO CHEMICAL INDUSTRIES LTD.**
**11-1, Ichinohashinomoto-cho**
**Higashiyama-ku, Kyoto-shi**
**Kyoto 605(JP)**

(72) Inventor: **Satoh, Masahiro**
**32, Fukakusa-ikenouti-cho, Fushimi-ku**
**Kyoto, 612(JP)**
Inventor: **Watanabe, Tetsuya**
**10-1, Imakumano-minamihiyoshi-cho**
**Higashiyama-ku, Kyoto-shi, 605(JP)**
Inventor: **Yoshio, Kunikiyo**
**2-8-13, Shinohara-cho**
**Ohmihachiman-shi, Shiga-ken, 523(JP)**
Inventor: **Kishiki, Hiroshi**
**2-12-2-204, Kamihamuro**
**Takatsuki-shi, Osaka-fu, 569(JP)**
Inventor: **Sugita, Naoko**
**12-44, Higashinominamiinoue-cho,**
**Yamashina-ku**
**Kyoto, 607(JP)**

(74) Representative: **Marlow, Nicholas Simon et al**
**Reddie & Grose 16, Theobalds Roadoad**
**London WC1X 8PL(GB)**

(54) **Liquid crystal compounds and optically active compounds.**

(57) A compound usefull for liquid crystal component which is represented by the following formulas (1), (2), (3), or (4) ;

$R_1$ -$X_1$ -$A_1$ -( $A_2$ )$_n$ -$Y_1$ -$A_3$ -$X_2$ -$R_2$ (1)

$R_1$ -$X_1$ -( $A_3$ -$Y_1$ )$_n$ -$A_1$ -$A_2$ -$X_2$ -$R_2$ (2)

$R_3$ -$X_1$ -$A_4$ -$Y_1$ -$A_3$ -$X_2$ -$R_4$ (3)

$R_3$ -$X_1$ -$A_5$ -$Y_1$ -$A_1$ -$A_2$ -$X_2$ -$R_4$ (4)

wherein $R_1$ ,$R_2$ ,$R_3$ and $R_4$ are $C_{1-18}$ alkyl groups; $X_1$ and $X_2$ is -, O, S, -C≡C- or divarent groups such as COO, OCO, $CH_2$ O or $OCH_2$

EP 0 409 634 A2

; $Y_1$ is $-CH_2$ $CH_2$ - or $-C\equiv C-$ ; $A_1$ is 1,4-phenylene-group, – 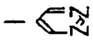 –, – 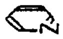 –, – 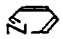 –, or – 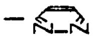 – ; $A_2$ , $A_3$ , $A_5$ are such as 1,4-phenylene-group; $A_4$ is – 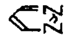 –, – 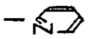 –, or – 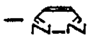 –.

# LIQUID CRYSTAL COMPOUNDS AND OPTICALLY ACTIVE COMPOUNDS

## Field of the Invention

The invention relates to a novel liquid crystalline compound useful in practice as display elements.

## Description of the Prior Art

Of liquid crystal diaplay elements those which are of TN(twisted nematic)-type are nowadays found typical in the art. This type of element is generally featured with a number of advantages such as lower driving potential and power consumption than competitive products. This type, however, is found inferior when compared with luminescent typed element (cathode ray tube, electroluminescent, plasma display, etc.) in term of response time. Even those which were specifically made improved with a twisted angele of 180 to 270° were found still unable to overcome the said shortcoming.

Notwithstanding the elaboration as aforementioned, none of TN-type element with a significantly faster response time has yet come realized.

Under such circumstance what is likely possible to realize a drastic improvement in term of response time is a novel display technology incorporating ferroelectric liquid crystal materials which is the focus of debate among controversial industrialists. (N.A. Clark et al ; Applied Phys. Lett., 36, 899 (1980)). The technology involves the utilization of chiral smectic phase more particularly phase C which is capable to provide ferroelectricity. It is generally known that not only chiral smectic C phase but also chiral smetic F-, G-, H-, I-, phases, etc. would provide ferroelectricity.

While those ferroelectric liquid crystal materials to be utilized as display elements must satisfy a plurality of requirements, no single kind of compound is at present found capable to satisfy such requirements but a plurality of liquid crystalline or non-liquid crystalline compounds must be inevitably combined together to obtain ferroelectric liquid crystal materials useful for the purpose intended. Unlike such ferroelectric liquid crystal composition which is solely composed of ferroelectric liquid crytalline compounds, Japanese Patent Laid Open Publication NO. 60-36003 disclosed a method to obtain a ferroelectric liquid crystal composition, wherein a basic substance consisting of those compounds and compositions which show non-chiral smectic phase of C,F,G,H,I, etc. is intermixed with single or a plurality of compounds with a ferroelectric liquid crystalline phase. In addition, another method to obtain a ferroelectric liquid crystal composition through intermixing of single or a plurality of compounds which are optically active but without ferroelectric liquid crystalline phase is widely known (Mol. Cryst. Liq. Cryst., 89, 327 (1982)).

In short, it can be said that a ferroelectric liquid crystal composition may be obtained through intermixing of basic substance with single or a plurality of compounds which shall have simply an optical activity, regardless of existence of ferroelectricity.

As the basic substance mentioned in the above, different kinds of compounds which show non-chiral smectic liquid crystalline phase such as smectic C phase may be generally made in use. In practice, however, liquid crystalline compounds or mixture showing smectic C phase over a wide range of temperature inclusive of room temperature are more preferable. Counted to the liquid crystalline mixture with smectic phase C are phenylbenzoate-, biphenyl-, phenylpyridine series and 5-alkyl-2-(4-alkoxylphenyl) pyrimidine as liquid crystalline compounds. Optically active 2-octanol derivative are typically known as optically active compounds.

It, however, remains still left open, whether those liquid crystal materials with chiral smectic C phase which are normally obtained through admixing optically active components are found superior with respect to ferroelectricity required for liquid crystal display. The reason why not is to be sought in the fact that liquid crystal display incorporating ferroelectricity is not made completed yet in technical point of view. In view of the foregoing there is found no other way to proceed with for the time being than to find out the desired one through screening of different kinds of new materials suitable for liquid crystal composition with smectic C phase.

It appears that the optically active 2-octanol derivatives, in general, would not be capable enough to enhance response time of liquid crystalline composition with chiral semctic C phase because of its relatively smaller dipole moment within melecule and that any optically active compounds that may be adaptable for this purpose would be a requisite for being able to utilize it advantageously in the practical field.

Summary of the Invention

The inventors' effort was concentrated to provide new types of liquid crystalline- and optically active compounds which may be advantageously utilized for composing liquid crystal with chiral smectic C phase conforming to the purpose intended, and this approach resulted in a fruitful success as is demonstrated by the present invention.

1) A compound usefull for liquid crystal component which is represented by the following formulas (1) or (2) ;

$R_1$ -$X_1$ -$A_1$ -( $A_2$ )$_n$ -$Y_1$ -$A_3$ -$X_2$ -$R_2$ . (1)

$R_1$ -$X_1$ -( $A_3$ -$Y_1$ )$_n$ -$A_1$ -$A_2$ -$X_2$ -$R_2$ (2)

wherein $R_1$ and $R_2$ denote the same or different alkyl-groups containing 1 to 18 carbon atoms (one or two -$CH_2$ - of said alkyl-groups may be substituted by those which are selected from the group consist of oxygen atoms, sulfur atoms and -CH = CH-, provided that 2 oxygen atoms or 2 sulfur atoms are not directly bonded together);

$X_1$ and $X_2$ denote direct single bond, -O-, -S-, -C≡C-, -COO-, -OCO-, -$CH_2$ O- or -$OCH_2$ -; $Y_1$ denotes -$CH_2$ $CH_2$ - or -C≡C-;

$A_1$ denotes 1,4-phenylene-group which may be substituted by 1 to 4 fluorine atoms or 1 to 2 chlorine atoms, 1 to 2 bromine atoms, 1 to 2 cyano- groups, 1 to 2 nitro- groups or trifluoromethyl- groups;

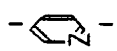, 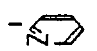, or 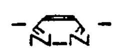 ;

$A_2$ denotes 1,4-phenylene group which may be substituted by 1 to 4 fluorine atoms or 1 to 2 chlorine atoms, 1 to 2 bromine atoms, 1 to 2 cyano-groups, 1 to 2 nitro-groups or trifluoromethyl-groups;

$A_3$ denotes 1,4-phenylene group which may be substituted by 1 to 4 fluorine atoms, 1 to 2 chlorine atoms, 1 to 2 bromine atoms, 1 to 2 cyano-groups, 1 to 2 nitro-groups or 1 to 2 trifluoromethyl-groups; 4,4'-biphenylene-group (of which 1 or 2 CH-groups may be substituted by nitrogen atoms) or 2,6-naphthylene-group; and n is equal to 0 or 1.

2) A compound usefull for liquid crystal component or optically active component which is represented by the following formulas (3) or (4) ;

$R_3$ -$X_1$ -$A_4$ -$Y_1$ -$A_3$ -$X_2$ -$R_4$ (3)

$R_3$ -$X_1$ -$A_5$ -$Y_1$ -$A_1$ -$A_2$ -$X_2$ -$R_4$ (4)

wherein $R_3$ and $R_4$ denote the same or different alkyl-groups containing 1 to 18 carbon atoms at least one of $R_3$ or $R_4$ should have asymmetric carbon atoms, and one or two -CH $_2$ - of said alkyl-groups may be substituted by those which are selected from the group consist of oxygen atoms, sulfur atoms and -CH = CH-, provided that 2 oxygen atoms or 2 sulfur atoms are not directly bonded together);

$X_1$ and $X_2$ denote direct single bond, -O-, -S-, -C≡C-, -COO-, -OCO-, -$CH_2$ O- or -$OCH_2$ -; $Y_1$ denotes -$CH_2$ $CH_2$ - or -C≡C-;

$A_1$ denotes 1,4-phenylene-group which may be substituted by 1 to 4 fluorine atoms or 1 to 2 chlorine atoms, bromine atom, cyano-group, 1 to 2 nitro- groups or trifluoromethyl- groups;

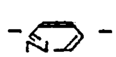, or 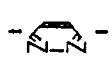 ;

$A_2$ , and $A_5$ denote 1,4-phenylene group which may be substituted by 1 to 4 fluorine atoms or 1 to 2 chlorine atoms, 1 to 2 bromine atoms, 1 to 2 cyano-groups, 1 to 2 nitro-groups or 1 to 2 trifluoromethyl-groups;

$A_3$ denotes 1,4-phenylene group which may be substituted by 1 to 4 fluorine atoms or 1 to 2 chlorine atoms, 1 to 2 bromine atoms, 1 to 2 cyano-groups, 1 to 2 nitro-groups or 1 to 2 trifluoromethyl-groups; 4,4'-biphenylene-group (of which 1 or 2 CH-groups may be substituted by nitrogen atoms) or 2,6-naphthylene-group;

$A_4$ denotes 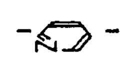, 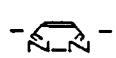 or 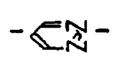.

Brief Description of the Drawings

Figs. 1, -2 and -3 show IR-, H-NMR and F-NMR spectra of the compound obtained under the Example 1 respectively.
Figs. 4 and -5 show IR- and H-NMR spectra of the compound obtained under the Example 18 respectively.
Figs. 6, -7 and -8 show IR-, H-NMR and F-NMR spectra of the compound obtained under the Example 19 respectively.
Figs. 9 and -10 show IR- and H-NMR spectra of the compound obtained under the Example 20 respectively.
Figs. 11 and -12 show IR- and H-NMR spectra of the compound obtained under the Example 21 respectively.
Figs. 13, -14 and -15 show IR-, H-NMR and F-NMR spectra of the compound obtained under the Example 22 respectively.
Figs. 16, -17 and -18 show IR-, H-NMR and F-NMR spectra of the compound obtained under the Example 23 respectively.
Figs. 19 and -20 show IR- and H-NMR spectra of the compound obtained under the Example 24 respectively.
Figs. 21 and -22 show IR- and H-NMR spectra of the compound obtained under the Example 25 respectively.
Figs. 23, -24 and -25 show IR-, H-NMR and F-NMR spectra of the compound obtained under the Example 26 respectively.
Figs. 26, -27 and -28 show IR-, H-NMR and F-NMR spectra of the compound obtained under the Example 27 respectively.
Figs. 29 and -30 show IR- and H-NMR spectra of the compound obtained under the Example 28 respectively.
Figs. 31, -32 and -33 show IR-, H-NMR and F-NMR spectra of the compound obtained under the Example 29 respectively.
Figs. 34 and -35 show IR- and H-NMR spectra of the compound obtained under the Example 30 respectively.
Figs. 36, -37 and -38 show IR-, H-NMR and F-NMR spectra of the compound obtained under the Example 31 respectively.
Figs. 39 and -40 show IR- and H-NMR spectra of the compound obtained under the Example 32 respectively.
Figs. 41, -42 and -43 show IR-, H-NMR and F-NMR spectra of the compound obtained under the Example 33 respectively.
Figs. 44 and -45 show IR- and H-NMR spectra of the compound obtained under the Example 34 respectively.
Figs. 46, -47 and -48 show IR-, H-NMR and F-NMR spectra of the compound obtained under the Example 35 respectively.
Figs. 49, -50 and -51 show IR-, H-NMR and F-NMR spectra of the compound obtained under the Example 36 respectively.
Figs. 52 and -53 show IR- and H-NMR spectra of the compound obtained under the Example 37 respectively.
Figs. 54, -55 and -56 show IR-, H-NMR and F-NMR spectra of the compound obtained under the Example 38 respectively.
Figs. 57, -58 and -59 show IR-, H-NMR and F-NMR spectra of the compound obtained under the Example 39 respectively.
Figs. 60, -61 and -62 show IR-, H-NMR and F-NMR spectra of the compound obtained under the Example 40 respectively.
Figs. 63 and -64 show IR- and H-NMR spectra of the compound obtained under the Example 41 respectively.
Figs. 65, -66 and -67 show IR-, H-NMR and F-NMR spectra of the compound obtained under the Example 42 respectively.
Figs. 68, -69 and -70 show IR-, H-NMR and F-NMR spectra of the compound obtained under the Example 43 respectively.
Figs. 71, -72 and -73 show IR-, H-NMR and F-NMR spectra of the compound obtained under the Example 44 respectively.

Figs. 74, -75 and -76 show IR-, H-NMR and F-NMR spectra of the compound obtained under the Example 45 respectively.

Figs. 77, -78 and -79 show IR-, H-NMR and F-NMR spectra of the compound obtained under the Example 46 respectively.

Figs. 80, -81 and -82 show IR-, H-NMR and F-NMR spectra of the compound obtained under the Example 47 respectively.

Figs. 83, -84 and -85 show IR-, H-NMR and F-NMR spectra of the compound obtained under the Example 48 respectively.

Figs. 86, -87 and -88 show IR-, H-NMR and F-NMR spectra of the compound obtained under the Example 49 respectively.

Figs. 89, -90 and -91 show IR-, H-NMR and F-NMR spectra of the compound obtained under the Example 50 respectively.

Figs. 92, -93 and -94 show IR-, H-NMR and F-NMR spectra of the compound obtained under the Example 51 respectively.

Figs. 95, -96 and -97 show IR-, H-NMR and F-NMR spectra of the compound obtained under the Example 52 respectively.

Figs. 98, -99 and -100 show IR-, H-NMR and F-NMR spectra of the compound obtained under the Example 53 respectively.

Figs. 101, -102 and -103 show IR-, H-NMR and F-NMR spectra of the compound obtained under the Example 54 respectively.

Figs. 104, -105 and -106 show IR-, H-NMR and F-NMR spectra of the compound obtained under the Example 55 respectively.

Figs. 107, -108 and -109 show IR-, H-NMR and F-NMR spectra of the compound obtained under the Example 56 respectively.

Figs. 110, -111 and -112 show IR-, H-NMR and F-NMR spectra of the compound obtained under the Example 57 respectively.

Figs. 113, -114 and -115 show IR-, H-NMR and F-NMR spectra of the compound obtained under the Example 58 respectively.

Figs. 116, -117 and -118 show IR-, H-NMR and F-NMR spectra of the compound obtained under the Example 59 respectively.

Figs. 119, -120 and -121 show IR-, H-NMR and F-NMR spectra of the compound obtained under the Example 60 respectively.

Figs. 122, and -123 show IR-, and H-NMR spectra of the compound obtained under the Example 63 respectively.

Figs. 124, -125 and -126 show IR-, H-NMR and F-NMR spectra of the compound obtained under the Example 64 respectively.

Figs. 127, -128 and -129 show IR-, H-NMR and F-NMR spectra of the compound obtained under the Example 65 respectively.

Figs. 130, -131 and -132 show IR-, H-NMR and F-NMR spectra of the compound obtained under the Example 66 respectively.

Figs. 133, and -134 show IR- and H-NMR spectra of the compound obtained under the Example 67 respectively.

Figs. 135, -136 and -137 show IR-, H-NMR and F-NMR spectra of the compound obtained under the Example 68 respectively.

Figs. 138, -139 and -140 show IR-, H-NMR and F-NMR spectra of the compound obtained under the Example 69 respectively.

Figs. 141, -142 and -143 show IR-, H-NMR and F-NMR spectra of the compound obtained under the Example 70 respectively.

Figs. 144, -145 and -146 show IR-, H-NMR and F-NMR spectra of the compound obtained under the Example 71 respectively.

Figs. 147, -148 and -149 show IR-, H-NMR and F-NMR spectra of the compound obtained under the Example 72 respectively.

Figs. 150, -151 and -152 show IR-, H-NMR and F-NMR spectra of the compound obtained under the Example 73 respectively.

Figs. 153, -154 and -155 show IR-, H-NMR and F-NMR spectra of the compound obtained under the Example 74 respectively.

Figs. 156, -157 and -158 show IR-, H-NMR and F-NMR spectra of the compound obtained under the Example 75 respectively.

Figs. 159, -160 and -161 show IR-, H-NMR and F-NMR spectra of the compound obtained under the Example 77 respectively.
Figs. 162, -163 and -164 show IR-, H-NMR and F-NMR spectra of the compound obtained under the Example 78 respectively.
Figs. 165, -166 and -167 show IR-, H-NMR and F-NMR spectra of the compound obtained under the Example 79 respectively.

## DETAILD DESCRIPTION OF THE INVENTION

In the general formulae (1) and (2), $R_1$ and $R_2$ are same or different alkyl groups containing usually 1 - 18 carbon atoms. Illustrative of suitable alkyl groups are straight-chain alkyl groups, such as methyl, ethyl, n-propyl, n-butyl, n-pentyl, n-hexyl, n-heptyl, n-octyl, n-nonyl, n-decyl, n-undecyl, n-dodecyl, n-tridecyl, n-tetradecyl, n-pentadecyl, n-hexadecyl and n-octadecyl groups; branched alkyl groups, such as iso-propyl, iso-butyl, 3-methylbutyl, 4-methylpentyl, 5-methylhexyl, 6-methyheptyl, 7-methyloctyl, 8-methylnonyl, 9-methyldecyl, 1-methylpropyl, 1-methylbutyl, 1-methylpentyl, 1-methylhexyl, 1-methylheptyl, 1-methyloctyl, 2-methylpropyl, 2-methylbutyl, 2-methylpentyl, 2-methylhexyl, 2-methylheptyl, 2-methyloctyl, 2-methylnonyl, 2-methyldecyl, 2-methylundecyl, 2-methyldodecyl, 2-methyltridecyl, 2-methyltetradecyl, 3-methylbutyl, 3-methylpentyl, 3-methylhexyl, 3-methylheptyl, 3-methyloctyl, 4-methylpentyl, 4-methylhexyl, 4-methylheptyl, 4-methyloctyl, 5-methylhexyl, 5-methylheptyl, 5-methyloctyl, 5-methylnonyl, 5-methyldecyl, 6-methylheptyl, 6-methyloctyl, 6-methylnonyl, 6-methyldecyl, 7-methyloctyl, 7-metyhlnonyl, 7-methyldecyl, 8-methylnonyl, 8-methyldecyl, 9-methyldecyl and 9-methylundecyl groups; substituted alkyl groups, for example including F-substituted alkyl groups, such as 2-fluoropropyl, 2-fluorobutyl, 2-fluoropentyl, 2-fluorohexyl, 2-fluoroheptyl, 2-fluorooctyl, 2-fluorononyl, 2-fluorodecyl, 2-fluoroundecyl, 2-fluorododecyl, 2-fluorotridecyl, 2-fluorotetradecyl, 3-fluoropropyl, 4-fluorobutyl, 5-fluoropentyl, 6-fluorohexyl, 7-fluoroheptyl, 8-fluorooctyl, 9-fluorononyl, 10-fluorodecyl, 11-fluoroundecyl, 12-fluorododecyl, 2-(perfluoro-n-butyl)ethyl, 2-(perfluoro-n-hexyl)ethyl, 2-(perfluoro-n-octyl)ethyl, 2-(perfluoro-n-decyl)ethyl, 2-fluoro-3-methylbutyl, 2-fluoro-3-methylpentyl and 2-fluoro-4-methylpentyl groups; Cl-substituted alkyl groups, such as 2-chloropropyl, 2-chlorobutyl, 2-chloropentyl, 2-chlorohexyl, 2-chloroheptyl, 2-chlorooctyl, 2-chlorononyl, 2-chlorodecyl, 2-chloroundecyl, 2-chlorododecyl, 2-chlorotridecyl, 2-chlorotetradecyl, 2-chloro-3-methylbutyl, 2-chloro-3-methylpentyl and 2-chloro-4-methylpentyl groups; and alkoxy-substituted alkyl groups, such as 2-methoxypropyl, 2-ethoxypropyl, 2-propoxypropyl, 2-butoxypropyl, 2-pentyloxypropyl, 2-hexyloxypropyl, 2-heptyloxypropyl, 2-octyloxypropyl, 2-nonyloxypropyl, 2-decyloxypropyl, 2-(2-methylbutyloxy)propyl, 2-iso-propoxypropyl, 3- methoxybutyl, 3-ethoxybutyl, 3-propyloxybutyl, 3-butyloxybutyl, 3-pentyloxybutyl, 3-hexyloxybutyl, 3-heptyloxybutyl, 3-octyloxybutyl, 3-nonyloxybutyl, 3-decyloxybutyl, 3-(2-methylbutyloxy)butyl, 3-iso-propyloxybutyl, 4-methoxypentyl, 4-ethoxypentyl, 4-propoxypentyl, 4-butyloxypentyl, 4-pentyloxypentyl, 4-hexyloxypentyl, 4-heptyloxypentyl, 4-octyloxypentyl, 4-nonyloxypentyl, 4-decyloxypentyl, 4-(2-methyl-butyloxy)pentyl, 4-(2-methylpentyloxy)pentyl and 4-isopropoxypentyl groups etc.

In the general formulae (3) and (4), $R_3$ and $R_4$ are same or different alkyl groups containing usually 1 - 18 carbon atoms, which may be substituted with asymmetric carbon atom(s). Illustrative of suitable alkyl groups are straight-chain alkyl groups, such as methyl, ethyl, n-propyl, n-butyl, n-pentyl, n-hexyl, n-heptyl, n-octyl, n-nonyl, n-decyl, n-undecyl, n-dodecyl, n-tridecyl, n-tetradecyl, n-pentadecyl, n-hexadecyl and n-octadecyl groups; branched alkyl groups, such as iso-propyl, iso-butyl, optically active (hereinafter referred to as oa) alkyl groups, such as oa 1-methylpropyl, oa 1-methylbutyl, oa 1-methylpentyl, oa 1-methylhexyl, oa 1-methylheptyl, oa 1-methyloctyl, oa 2-methylbutyl, oa 2-methylpentyl, oa 2-methylhexyl, oa 2-methylheptyl, oa 2-menthyloctyl, oa 2-methylnonyl, oa 2-methyldecyl, oa 2-methylundecyl, oa 2-methyldodecyl, oa 2-methyltridecyl, oa 2-methyltetradecyl, oa 3-methylpentyl, oa 3-methylhexyl, oa 3-methylheptyl, oa 3-methyloctyl, oa 4-methylhexyl, oa 4-methylheptyl, oa 4-methyloctyl, oa 5-methylheptyl, oa 5-methyloctyl, oa 5-methylnonyl, oa 5-methyldecyl, oa 6-methyloctyl, oa 6-methylnonyl, oa 6-methyldecyl, oa 7-methylnonyl, oa 7-methyldecyl, oa 8-methyldecyl and oa 9-methylundecyl groups; and substituted oa alkyl groups, for example including F-substituted oa alkyl groups, such as oa 2-fluoropropyl, oa 2-fluorobutyl, oa 2-fluoropentyl, oa 2-fluorohexyl, oa 2-fluoroheptyl, oa 2--fluorooctyl, oa 2-fluorononyl, oa 2-fluorodecyl, oa 2-fluoroundecyl, oa 2-fluorododecyl, oa 2-fluorotridecyl, oa 2-fluorotetradecyl, oa 2-fluoro-3-methylbutyl, oa 2-fluoro-3-methylpentyl and oa 2-fluoro-4-methylpentyl groups; Cl-substituted oa alkyl groups, such as oa 2-chloropropyl, oa 2-chlorobutyl, oa 2-cholropentyl, oa 2-chlorohexyl, oa 2-chloroheptyl, oa 2-chlorooctyl, oa 2-chlorononyl, oa 2-chlorodecyl, oa 2-chloroundecyl, oa 2-chlorododecyl, oa 2-chlorotridecyl, oa 2-chloro-3-methylbutyl, oa 2-chloro-3-methylpentyl and oa 2-chloro-4-methylpentyl groups; and alkoxy-substituted oa alkyl groups, such as oa 2-methoxypropyl, oa 2-ethoxypropyl, oa 2-propoxypropyl, oa 2-butoxypropyl, oa 2-pentyloxypropyl, oa 2-hexyloxypropyl, oa 2-heptyloxypropyl, oa 2-octyloxypropyl, oa 2-nonyloxypropyl, oa 2-decyloxypropyl, oa 2-(2-methylbutyloxy)propyl, oa 2-iso-propoxypropyl, oa 3-methoxybutyl, oa 3-ethoxybutyl, oa 3-propoxybutyl, oa 3-butyloxybutyl, oa 3-pentyloxybutyl, oa 3-hexyloxybutyl, oa 3-heptyloxybutyl, oa 3-octyloxybutyl, oa 3-nonyloxybutyl, oa 3-decyloxybutyl, oa 3-(2-methylbutyloxy)butyl, oa 3-iso-propyloxybutyl, oa 4-methoxypentyl, oa 4-ethoxypentyl, oa 4-propoxypentyl, oa 4-butyloxypentyl, oa 4-pentyloxypentyl, oa 4-hexyloxypentyl, oa 4-heptyloxypentyl oa 4-octyloxypentyl, oa 4-nonyloxypentyl, oa 4-decyloxypentyl, oa 4-(2-methyl-butyloxy)pentyl, oa 4-iso-propoxypentyl groups: trifluoromethyl-substituted oa alkyl groupes, such as oa 1-trifluoromethylbutyl, oa 1-trifluoromethylpentyl, oa 1-trifluoromethylhexyl, oa 1-trifluoromethylheptyl, oa 1-trifluoromethyloctyl, oa 1-trifluoromethylnonyl, oa 1-trifluoromethyldecyl, oa 1-trifluoromethyundecyl, oa 1-trifluoromethyldodecyl, oa 1-trifluoromethyl-2-methoxyethyl, oa 1-trifluoromethyl-2-ethoxyethyl, oa 1-trifluoromethyl-2-propyloxyethyl, oa 1-trifluoromethyl-2-butyloxyethyl, oa 1-trifluoromethyl-2-pentyloxyethyl, oa 1-trifluoromethyl-2-hexyloxyethyl, oa 1-trifluoromethyl-2-iso-propyloxyethyl, oa 1-trifluoromethyl-2-(1′-methylpropyloxy)ethyl, oa 1-trifluoromethyl-2-(1′-methylbutyloxy)ethyl, oa 1-trifluoromethyl-3-(1′-methylhexyloxy)propyl, oa 1-trifluoromethyl-3-(1′-methylheptyloxy)propyl, oa 1-trifluoromethyl-3-(2′-methylbutyloxy)propyl, oa 1-trifluoromethyl-4-methyloxybutyl, oa 1-trifluoromethyl-4-ethoxybutyl, oa 1-trifluoromethyl-4-propyloxybutyl, oa 1-trifluoromethyl-4-butyloxybutyl, oa 1-trifluoromethyl-4-pentyloxybutyl, oa 1-trifluoromethyl-4-hexyloxybutyl, oa 1-trifluoromethyl-4-iso-propyloxybutyl, oa 1-trifluoromethyl-4-(1′-methylpropyloxy)butyl, oa 1-trifluoromethyl-4-(1′-methylbutyloxy)butyl, oa 1-trifluoromethyl-4-(1′-methylpentyloxy)butyl, oa 1-trifluoromethyl-4-(1′-methylhexyloxy)butyl, oa 1-trifluoromethyl-2-(1′-methylpentyloxy)ethyl, oa 1-trifluoromethyl-2-(1′-methylhexyloxy)ethyl, oa 1-trifluoromethyl-2-(1′-methylheptyloxy)ethyl, oa 1-trifluoromethyl-2-(2′-methylbutyloxy)ethyl, oa 1-trifluoromethyl-3-methoxypropyl, oa 1-trifluoromethyl-3-ethoxypropyl, oa 1-trifluoromethyl-3-propyloxypropyl, oa 1-trifluoromethyl-3-butyloxypropyl, oa 1-trifluoromethyl-3-pentyloxypropyl, oa 1-trifluoromethyl-3- hexyloxypropyl, oa 1-trifluoromethyl-3-iso-propyloxypropyl, oa 1-trifluoromethyl-3-(1′-methylpropyloxy)propyl, oa 1-trifluoromethyl-3-(1′-methylbutyloxy)propyl, oa 1-trifluoromethyl-3-(1′-methylpentyloxy)propyl, oa 1-trifluoromethyl-4-(1′-methylheptyloxy)butyl, oa 1-trifluoromethyl-4-(2′-methylbutyloxy)butyl groups, cyano-substituted oa alkyl groups, such as oa 1-cyanobutyl, oa 1-cyanopentyl, oa 1-cyanohexyl, oa 1-cyanoheptyl, oa 1-cyanooctyl, oa 1-cyanononyl, oa 1-cyanodecyl, oa 1-cyanoundecyl, oa 1-cyanododecyl groups, etc.

$R_1$ and $R_2$ may preferably be alkyl group with 3 to 14 carbon atoms. $R_3$ and $R_4$ may preferably be alkyl group with 3 to 14 carbon atoms which may include asymmetric carbon atoms.

In the formulas (1) and -(2) $X_1$ and $X_2$ may preferably be direct single bond, -O-, -C≡C- and -COO-.

In the formulas (3) and (4) $X_1$ and $X_2$ may preferably be direct single bond, -O-, -C≡C-, -COO-, -OCO- and $-CH_2$ O-.

$A_1$ may preferably be 1,4-phenylene group,

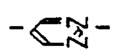 , 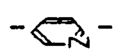 ,

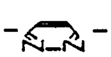 , and 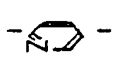

which may be substituted by 1 to 4 fluorine atoms. $A_2$ and $A_5$ may preferably be 1,4-phenylene group which may be substituted by 1 to 4 fluorine atoms. A3 may preferably be 1,4phenylene group which may be substituted by 1 to 4 fluorine atoms, 4,4′-biphenylene group (of which one or two CH-groups may be substituted by nitrogen atoms) or 2,6-naphthylene group.

Those groups, in which 1,4-phenylene group, 4,4′-biphenylene group caused their one or two CH-groups to be substituted by nitrogen atoms inolve, for example,

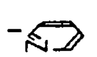, 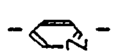 ,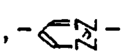, 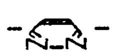,

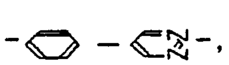, or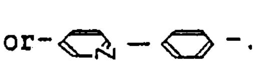. $A_4$ may preferably be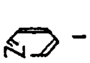 or 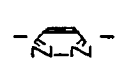.

Compounds represented by the formulas (1) to (4) are typically exemplified by those containing such groups disclosed in the Tables 1a to 4b.

Following symbols used in the tables 1 to 4 denote the respective groupes enumerated hereunder;

BUT ; $n-C_4H_9$ -

PEN ; n-$C_5H_9$ -
HEX ; n-$C_6H_{13}$-
HEP ; n-$C_7H_{15}$-
OCT ; n-$C_8H_{17}$-
NON ; n-$C_9H_{19}$-
DEC ; n-$C_{10}H_{21}$-
UND ; n-$C_{11}H_{23}$-
DOD ; n-$C_{12}H_{25}$-
TED ; n-$C_{14}H_{29}$-
NAP ; 2,6-naphthylene group
D ; direct single bond (-)
Et ; -O-
Ac ; -C ≡C-
Es> ; -COO-
<Es; -OCO-

PhF ; 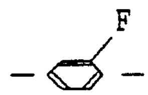

FPh ; 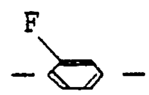

BPhF ; 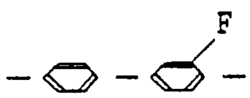

2MB ; $-CH_2\overset{CH_3}{\overset{|}{C}}{}^*HC_2H_5$

4MH ; $-CH_2CH_2CH_2\overset{CH_3}{\overset{|}{C}}{}^*HC_2H_5$

$CH_3-C_6$ : $-\overset{CH_3}{\overset{|}{C}}{}^*HC_6H_{13}$

$CF_3-C_6$ : $-\overset{CF_3}{\overset{|}{C}}{}^*HC_6H_{13}$

MO ; -$CH_2$ O-
r ; racemic form
* ; Optically active group

(1) 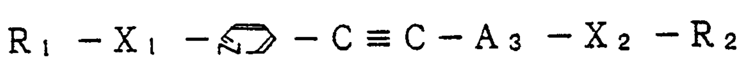 (1a)

Table 1a (1)

| No. | $R_1$ | $X_1$ | $A_3$ | $X_2$ | $R_2$ |
|---|---|---|---|---|---|
| 1a-1 | HEX | D | PhF | Et | HEX |
| 1a-2 | HEX | D | PhF | Et | HEP |
| 1a-3 | HEX | D | PhF | Et | OCT |
| 1a-4 | HEX | D | PhF | Et | NON |
| 1a-5 | HEX | D | PhF | Et | DEC |
| 1a-6 | HEP | D | PhF | Et | HEX |
| 1a-7 | HEP | D | PhF | Et | HEP |
| 1a-8 | HEP | D | PhF | Et | OCT |
| 1a-9 | HEP | D | PhF | Et | NON |
| 1a-10 | HEP | D | PhF | Et | DEC |
| 1a-11 | OCT | D | PhF | Et | HEX |
| 1a-12 | OCT | D | PhF | Et | HEP |
| 1a-13 | OCT | D | PhF | Et | OCT |
| 1a-14 | OCT | D | PhF | Et | NON |
| 1a-15 | OCT | D | PhF | Et | DEC |
| 1a-16 | NON | D | PhF | Et | HEX |
| 1a-17 | NON | D | PhF | Et | HEX |
| 1a-18 | NON | D | PhF | Et | OCT |
| 1a-19 | NON | D | PhF | Et | NON |
| 1a-20 | NON | D | PhF | Et | DEC |
| 1a-21 | DEC | D | PhF | Et | HEX |
| 1a-22 | DEC | D | PhF | Et | HEP |
| 1a-23 | DEC | D | PhF | Et | OCT |
| 1a-24 | DOD | D | PhF | Et | DEC |
| 1a-25 | TED | D | PhF | Et | DEC |
| 1a-26 | HEP | D | Ph | Et | HEX |
| 1a-27 | HEP | D | Ph | Et | HEP |
| 1a-28 | HEP | D | Ph | Et | OCT |
| 1a-29 | HEP | D | Ph | Et | NON |
| 1a-30 | HEP | D | Ph | Et | DEC |
| 1a-31 | OCT | D | Ph | Et | HEX |
| 1a-32 | OCT | D | Ph | Et | HEP |
| 1a-33 | OCT | D | Ph | Et | OCT |

Table 1 a (2)

| No. | $R_1$ | $X_1$ | $A_3$ | $X_2$ | $R_2$ |
|---|---|---|---|---|---|
| 1a-34 | OCT | D | Ph | Et | NON |
| 1a-35 | OCT | D | Ph | Et | DEC |
| 1a-36 | NON | D | Ph | Et | HEX |
| 1a-37 | NON | D | Ph | Et | HEP |
| 1a-38 | NON | D | Ph | Et | OCT |
| 1a-39 | NON | D | Ph | Et | NON |
| 1a-40 | DEC | D | Ph | Et | HEX |
| 1a-41 | DEC | D | Ph | Et | HEP |
| 1a-42 | DEC | D | Ph | Et | OCT |
| 1a-43 | OCT | Et | Ph | D | OCT |
| 1a-44 | HEP | Et | Ph | D | NON |
| 1a-45 | HEX | Et | Ph | D | DEC |
| 1a-46 | OCT | Et | Ph | Et | OCT |
| 1a-47 | NON | Et | Ph | Et | HEP |
| 1a-48 | DEC | Et | Ph | Et | HEX |
| 1a-49 | OCT | Et | PhF | Et | OCT |
| 1a-50 | NON | Et | PhF | Et | HEP |
| 1a-51 | DEC | Et | PhF | Et | HEX |
| 1a-52 | DEC | Et | PhF | Et | HEP |
| 1a-53 | PEN | Ac | Ph | Et | HEX |
| 1a-54 | HEX | Ac | Ph | Et | HEX |
| 1a-55 | HEP | Ac | Ph | Et | HEX |
| 1a-56 | OCT | Ac | Ph | Et | HEX |
| 1a-57 | PEN | Ac | Ph | Et | HEP |
| 1a-58 | HEX | Ac | Ph | Et | HEP |
| 1a-59 | HEP | Ac | Ph | Et | HEP |
| 1a-60 | OCT | Ac | Ph | Et | HEP |
| 1a-61 | PEN | Ac | Ph | Et | OCT |
| 1a-62 | HEX | Ac | Ph | Et | OCT |
| 1a-63 | HEP | Ac | Ph | Et | OCT |
| 1a-64 | OCT | Ac | Ph | Et | OCT |
| 1a-65 | PEN | Ac | Ph | Et | NON |
| 1a-66 | HEX | Ac | Ph | Et | NON |
| 1a-67 | HEP | Ac | Ph | Et | NON |

Table 1 a (3)

| No. | $R_1$ | $X_1$ | $A_3$ | $X_2$ | $R_2$ |
|---|---|---|---|---|---|
| 1a-68 | OCT | Ac | Ph | Et | NON |
| 1a-69 | PEN | Ac | PhF | Et | HEX |
| 1a-70 | HEX | Ac | PhF | Et | HEX |
| 1a-71 | HEP | Ac | PhF | Et | HEX |
| 1a-72 | OCT | Ac | PhF | Et | HEX |
| 1a-73 | PEN | Ac | PhF | Et | HEP |
| 1a-74 | HEX | Ac | PhF | Et | HEP |
| 1a-75 | HEP | Ac | PhF | Et | HEP |
| 1a-76 | OCT | Ac | PhF | Et | HEP |
| 1a-77 | PEN | Ac | PhF | Et | OCT |
| 1a-78 | HEX | Ac | PhF | Et | OCT |
| 1a-79 | HEP | Ac | PhF | Et | OCT |
| 1a-80 | OCT | Ac | PhF | Et | OCT |
| 1a-81 | PEN | Ac | PhF | Et | NON |
| 1a-82 | HEX | Ac | PhF | Et | NON |
| 1a-83 | HEP | Ac | PhF | Et | NON |
| 1a-84 | OCT | Ac | PhF | Et | NON |
| 1a-85 | PEN | Ac | PhF | Et | DEC |
| 1a-86 | HEX | Ac | PhF | Et | DEC |
| 1a-87 | HEP | Ac | PhF | Et | DEC |
| 1a-88 | OCT | Ac | PhF | Et | DEC |
| 1a-89 | PEN | Ac | Ph | Et | DEC |
| 1a-90 | HEX | Ac | Ph | Et | DEC |
| 1a-91 | HEP | Ac | Ph | Et | DEC |
| 1a-92 | OCT | Ac | Ph | Et | DEC |
| 1a-93 | BUT | D | BPhF | Et | BUT |
| 1a-94 | PEN | D | BPhF | Et | BUT |
| 1a-95 | HEX | D | BPhF | Et | BUT |
| 1a-96 | HEP | D | BPhF | Et | BUT |
| 1a-97 | BUT | D | BPhF | Et | PEN |
| 1a-98 | PEN | D | BPhF | Et | PEN |
| 1a-99 | HEX | D | BPhF | Et | PEN |
| 1a-100 | HEP | D | BPhF | Et | PEN |
| 1a-101 | BUT | D | BPhF | Et | HEX |

Table 1 a (4)

| No. | $R_1$ | $X_1$ | $A_3$ | $X_2$ | $R_2$ |
|---|---|---|---|---|---|
| 1 a-1 0 2 | PEN | D | BPhF | Et | HEX |
| 1 a-1 0 3 | HEX | D | BPhF | Et | HEX |
| 1 a-1 0 4 | HEP | D | BPhF | Et | HEX |
| 1 a-1 0 5 | BUT | D | BPhF | Et | HEP |
| 1 a-1 0 6 | PEN | D | BPhF | Et | HEP |
| 1 a-1 0 7 | HEX | D | BPhF | Et | HEP |
| 1 a-1 0 8 | HEP | D | BPhF | Et | HEP |
| 1 a-1 0 9 | HEX | Et | BPhF | Et | PEN |
| 1 a-1 1 0 | HEX | Et | BPhF | Et | HEX |
| 1 a-1 1 1 | PEN | D | BPh | Et | PEN |
| 1 a-1 1 2 | HEX | D | BPh | Et | PEN |
| 1 a-1 1 3 | HEP | D | BPh | Et | PEN |
| 1 a-1 1 4 | PEN | D | BPh | Et | HEX |
| 1 a-1 1 5 | HEX | D | BPh | Et | HEX |
| 1 a-1 1 6 | HEP | D | BPh | Et | HEX |
| 1 a-1 1 7 | HEX | Et | BPh | Et | PEN |
| 1 a-1 1 8 | HEX | Et | BPh | Et | HEX |
| 1 a-1 1 9 | HEX | D | NAP | Et | PEN |
| 1 a-1 2 0 | HEP | D | NAP | Et | PEN |
| 1 a-1 2 1 | OCT | D | NAP | Et | PEN |
| 1 a-1 2 2 | NON | D | NAP | Et | PEN |
| 1 a-1 2 3 | HEX | D | NAP | Et | HEX |
| 1 a-1 2 4 | HEP | D | NAP | Et | HEX |
| 1 a-1 2 5 | OCT | D | NAP | Et | HEX |
| 1 a-1 2 6 | NON | D | NAP | Et | HEX |
| 1 a-1 2 7 | HEX | Et | NAP | Et | PEN |
| 1 a-1 2 8 | HEP | Et | NAP | Et | PEN |
| 1 a-1 2 9 | OCT | Et | NAP | Et | PEN |
| 1 a-1 3 0 | NON | Et | NAP | Et | PEN |
| 1 a-1 3 1 | HEX | Et | NAP | Et | HEX |
| 1 a-1 3 2 | HEP | Et | NAP | Et | HEX |
| 1 a-1 3 3 | OCT | Et | NAP | Et | HEX |
| 1 a-1 3 4 | NON | Et | NAP | Et | HEX |

(2) Case of

$R_1 - X_1 -$ 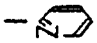 $- CH_2 CH_2 - A_3 - X_2 - R_2$ (1 b)

Table 1 b (1)

| No. | $R_1$ | $X_1$ | $A_3$ | $X_2$ | $R_2$ |
|---|---|---|---|---|---|
| 1b-1 | HEX | D | BPhF | Et | PEN |
| 1b-2 | HEP | D | BPhF | Et | HEX |
| 1b-3 | HEX | Et | BPhF | Et | PEN |
| 1b-4 | HEP | Et | BPhF | Et | HEX |
| 1b-5 | HEP | D | BPh | Et | PEN |
| 1b-6 | HEX | D | BPh | Et | HEX |
| 1b-7 | HEP | Et | BPh | Et | PEN |
| 1b-8 | HEX | Et | BPh | Et | HEX |
| 1b-9 | HEX | D | NAP | Et | HEX |
| 1b-10 | HEP | D | NAP | Et | HEX |
| 1b-11 | OCT | D | NAP | Et | HEX |
| 1b-12 | NON | D | NAP | Et | HEX |
| 1b-13 | HEX | Et | NAP | Et | HEX |
| 1b-14 | HEP | Et | NAP | Et | HEX |
| 1b-15 | OCT | Et | NAP | Et | HEX |
| 1b-16 | NON | Et | NAP | Et | HEX |

(3) Case of

$R_1 - X_1 -$ 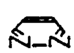 $- C \equiv C - A_3 - X_2 - R_2$ (1c)

same groups as No. 1a-1 to No. 1a-134 in the Table 1a.
No. 1c-1 to No. 1c-134

(4) Case of

$R_1 - X_1 -$ 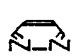 $- CH_2 CH_2 - A_3 - X_2 - R_2$ (1d)

same groups as No. 1b-1 to No. 1b-14 in the Table 1b.
No. 1d-1 to No. 1d-14

(5) Case of

$R_1 - X_1 -$ 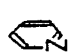 $- C \equiv C - A_3 - X_2 - R_2$ (1e)

same groups as No. 1a-1 to No. 1a-25 and No. 1a-53 to No.1a-134 in the Table 1a. And thus were added following Table.

Table 1 e (1)

| No. | $R_1$ | $X_1$ | $A_3$ | $X_2$ | $R_2$ |
|---|---|---|---|---|---|
| 1e-135 | HEP | Et | PhF | Et | HEP |
| 1e-136 | HEP | Et | PhF | Et | OCT |
| 1e-137 | HEP | Et | PhF | Et | NON |
| 1e-138 | HEP | Et | PhF | Et | DEC |
| 1e-139 | OCT | Et | PhF | Et | HEX |
| 1e-140 | OCT | Et | PhF | Et | HEP |
| 1e-141 | OCT | Et | PhF | Et | OCT |
| 1e-142 | OCT | Et | PhF | Et | NON |
| 1e-143 | OCT | Et | PhF | Et | DEC |
| 1e-144 | NON | Et | PhF | Et | HEX |
| 1e-145 | NON | Et | PhF | Et | HEP |
| 1e-146 | NON | Et | PhF | Et | OCT |
| 1e-147 | NON | Et | PhF | Et | NON |
| 1e-148 | NON | Et | PhF | Et | DEC |
| 1e-149 | DEC | Et | PhF | Et | HEX |
| 1e-150 | DEC | Et | PhF | Et | HEP |
| 1e-151 | DEC | Et | PhF | Et | OCT |
| 1e-152 | DOD | Et | PhF | Et | DEC |
| 1e-153 | TED | Et | PhF | Et | DEC |

(6) Case of

$R_1 - X_1 -$ 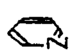 $- CH_2CH_2 - A_3 - X_2 - R_2$ (1f)

same groups as No. 1b-1 to No. 1b-14 in the Table 1b.
No. 1f-1 to No. 1f-14

(7) Case of

$R_1 - X_1 -$ 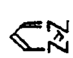 $- C \equiv C - A_3 - X_2 - R_2$ (1g)

same groups as No 1a-1 to Nola-52 and No. 1a-93 to No. 1a-134 in the Table 1a.
No. 1g-1 to No. 1g-93 to No. 1g-134

(8) Case of

$R_1 - X_1 -$ 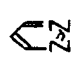 $- CH_2CH_2 - A_3 - X_2 - R_2$ (1h)

same groups as No. 1b-1 to No. 1b-14 in the Table 1b.
No. 1h-1 to No. 1h-14

(9) Case of

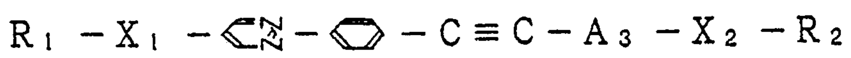 (1j)

Table 1 i (1)

| No. | $R_1$ | $X_1$ | $A_3$ | $X_2$ | $R_2$ |
|---|---|---|---|---|---|
| 1 i - 1 | BUT | D | PhF | Et | BUT |
| 1 i - 2 | BUT | D | PhF | Et | PEN |
| 1 i - 3 | BUT | D | PhF | Et | HEX |
| 1 i - 4 | PEN | D | PhF | Et | BUT |
| 1 i - 5 | PEN | D | PhF | Et | PEN |
| 1 i - 6 | PEN | D | PhF | Et | HEX |
| 1 i - 7 | HEX | D | PhF | Et | BUT |
| 1 i - 8 | HEX | D | PhF | Et | PEN |
| 1 i - 9 | HEX | D | PhF | Et | HEX |
| 1 i - 10 | HEP | D | PhF | Et | BUT |
| 1 i - 11 | HEP | D | PhF | Et | PEN |
| 1 i - 12 | HEP | D | PhF | Et | HEX |
| 1 i - 13 | OCT | D | PhF | Et | HEX |
| 1 i - 14 | OCT | D | PhF | Et | HEP |
| 1 i - 15 | OCT | D | PhF | Et | OCT |
| 1 i - 16 | NON | D | PhF | Et | HEX |
| 1 i - 17 | NON | D | PhF | Et | HEP |
| 1 i - 18 | PEN | Et | PhF | Et | PEN |
| 1 i - 19 | PEN | Et | PhF | Et | HEX |
| 1 i - 20 | HEX | Et | PhF | Et | BUT |
| 1 i - 21 | HEX | Et | PhF | Et | PEN |
| 1 i - 22 | HEX | Et | PhF | Et | HEX |
| 1 i - 23 | BUT | D | Ph | Et | BUT |
| 1 i - 24 | BUT | D | Ph | Et | PEN |
| 1 i - 25 | BUT | D | Ph | Et | HEX |
| 1 i - 26 | PEN | D | Ph | Et | BUT |
| 1 i - 27 | PEN | D | Ph | Et | PEN |
| 1 i - 28 | PEN | D | Ph | Et | HEX |
| 1 i - 29 | HEX | D | Ph | Et | BUT |
| 1 i - 30 | HEX | D | Ph | Et | PEN |
| 1 i - 31 | HEX | D | Ph | Et | HEX |
| 1 i - 32 | HEP | D | Ph | Et | HEX |
| 1 i - 33 | OCT | D | Ph | Et | HEX |
| 1 i - 34 | NON | D | Ph | Et | HEX |

Table 1 i (2)

| No. | $R_1$ | $X_1$ | $A_3$ | $X_2$ | $R_2$ |
|---|---|---|---|---|---|
| 1i-35 | PEN | Et | hF | Et | PEN |
| 1i-36 | PEN | Et | hF | Et | HEX |
| 1i-37 | HEX | Et | hF | – | HEX |
| 1i-38 | HEX | Et | hF | – | PEN |
| 1i-39 | HEX | Et | hF | – | BUT |

(10) Case of

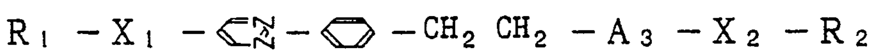

(1 j)

TABLE 1 j

| No. | $R_1$ | $X_1$ | $A_3$ | $X_2$ | $R_2$ |
|---|---|---|---|---|---|
| 1j-1 | PEN | D | PhF | Et | PEN |
| 1j-2 | PEN | D | PhF | Et | HEX |
| 1j-3 | HEX | D | PhF | Et | PEN |
| 1j-4 | HEX | D | PhF | Et | HEX |
| 1j-5 | NON | D | PhF | Et | HEX |
| 1j-6 | HEX | Et | PhF | Et | PEN |
| 1j-7 | HEX | Et | PhF | Et | HEX |
| 1j-8 | HEX | D | Ph | Et | BUT |
| 1j-9 | HEX | D | Ph | Et | PEN |
| 1j-10 | HEX | D | Ph | Et | HEX |
| 1j-11 | HEX | Et | Ph | Et | PEN |
| 1j-12 | HEX | Et | Ph | Et | HEX |
| 1j-13 | HEX | Et | Ph | Et | HEX |

(11) Case of

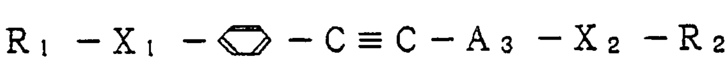

(1k)

Table 1k (1)

| No. | $R_1$ | $X_1$ | $A_3$ | $X_2$ | $R_2$ |
|---|---|---|---|---|---|
| 1k-1 | OCT | Et | PhF | Et | OCT |
| 1k-2 | OCT | Et | PhF | Et | NON |
| 1k-3 | OCT | Et | PhF | Et | DEC |
| 1k-4 | NON | Et | PhF | Et | HEP |
| 1k-5 | NON | Et | PhF | Et | OCT |
| 1k-6 | NON | Et | PhF | Et | NON |
| 1k-7 | NON | Et | PhF | Et | DEC |
| 1k-8 | DEC | Et | PhF | Et | HEX |
| 1k-9 | DEC | Et | PhF | Et | HEP |
| 1k-10 | DEC | Et | PhF | Et | OCT |
| 1k-11 | DEC | Et | PhF | Et | NON |
| 1k-12 | DEC | Et | PhF | Et | DEC |
| 1k-13 | UND | Et | PhF | Et | HEX |
| 1k-14 | UND | Et | PhF | Et | HEP |
| 1k-15 | UND | Et | PhF | Et | OCT |
| 1k-16 | UND | Et | PhF | Et | NON |
| 1k-17 | UND | Et | PhF | Et | DEC |

Table 1 k (2)

| No. | $R_1$ | $X_1$ | $A_3$ | $X_2$ | $R_2$ |
|---|---|---|---|---|---|
| 1k-18 | OCT | Et | PhF | Es> | HEX |
| 1k-19 | OCT | Et | PhF | Es> | HEP |
| 1k-20 | OCT | Et | PhF | Es> | OCT |
| 1k-21 | NON | Et | PhF | Es> | PEN |
| 1k-22 | NON | Et | PhF | Es> | HEX |
| 1k-23 | NON | Et | PhF | Es> | HEP |
| 1k-24 | NON | Et | PhF | Es> | OCT |
| 1k-25 | DEC | Et | PhF | Es> | BUT |
| 1k-26 | DEC | Et | PhF | Es> | PEN |
| 1k-27 | DEC | Et | PhF | Es> | HEX |
| 1k-28 | DEC | Et | PhF | Es> | HEP |
| 1k-29 | DEC | Et | PhF | Es> | OCT |
| 1k-30 | UND | Et | PhF | Es> | BUT |
| 1k-31 | UND | Et | PhF | Es> | PEN |
| 1k-32 | UND | Et | PhF | Es> | HEX |
| 1k-33 | UND | Et | PhF | Es> | HEP |
| 1k-34 | UND | Et | PhF | Es> | OCT |

(12) $R_1 - X_1 - A_3 - C \equiv C -$ 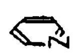 $- A_2 - X_2 - R_2$ (2a)

TABLE 2 a (1)

| No. | $R_1$ | $X_1$ | $A_3$ | $A_2$ | $X_2$ | $R_2$ |
|---|---|---|---|---|---|---|
| 2a-1 | HEX | ET | Ph | Ph | ET | BUT |
| 2a-2 | HEX | ET | Ph | Ph | ET | PEN |
| 2a-3 | HEX | ET | Ph | Ph | ET | HEX |
| 2a-4 | HEX | ET | Ph | PhF | ET | BUT |
| 2a-5 | HEX | ET | Ph | PhF | ET | PEN |
| 2a-6 | HEX | ET | Ph | PhF | ET | HEX |
| 2a-7 | HEX | ET | FPh | Ph | ET | BUT |
| 2a-8 | HEX | ET | FPh | Ph | ET | PEN |
| 2a-9 | HEX | ET | FPh | Ph | ET | HEX |
| 2a-10 | HEX | ET | FPh | PhF | ET | BUT |
| 2a-11 | HEX | ET | FPh | PhF | ET | PEN |
| 2a-12 | HEX | ET | FPh | PhF | ET | HEX |
| 2a-13 | HEX | D | Ph | PhF | ET | BUT |
| 2a-14 | HEX | D | Ph | PhF | ET | PEN |
| 2a-15 | HEX | D | Ph | PhF | ET | HEX |
| 2a-16 | HEX | D | FPh | Ph | ET | BUT |
| 2a-17 | HEX | D | FPh | Ph | ET | PEN |
| 2a-18 | HEX | D | FPh | Ph | ET | HEX |
| 2a-19 | HEX | D | FPh | PhF | ET | BUT |
| 2a-20 | HEX | D | FPh | PhF | ET | PEN |
| 2a-21 | HEX | D | FPh | PhF | ET | HEX |
| 2a-22 | HEX | DET | FPh | Ph | D | HEX |

(13) Case of

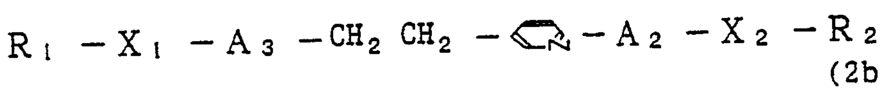

(2b)

same groups as No. 2a-1 to No. 2a-22 in the Table 2a. No. 2b-1 to 2b-22

(14) Case of

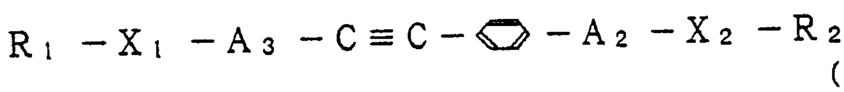

(2c)

same groups as No. 2a-1 to No. 2a-21 in the Table 2a. No. 2c-1 to 2c-21

(15) Case of

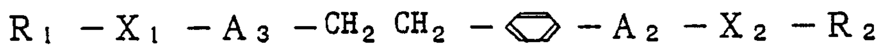

(2d)

same groups as No. 2a-1 to No. 2a-21 in the Table 2a. No. 2d-1 to 2d-21

(16) Case of

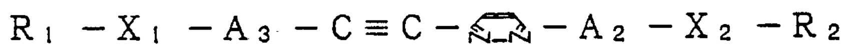

(2e)

same groups as No. 2a-1 to No. 2a-22 in the Table 2a. No. 2e-1 to 2e-22

(17) Case of

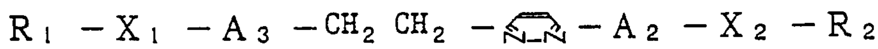

(2f)

same groups as No. 2a-1 to No. 2a-22 in the Table 2a. No. 2f-1 to 2f-22

(18) 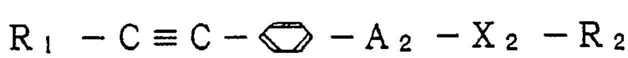 (2g)

Table 2g (1)

| No. | $R_1$ | $A_2$ | $X_2$ | $R_2$ |
|---|---|---|---|---|
| 2g-1 | HEX | PhF | Et | HEX |
| 2g-2 | HEX | PhF | Et | HEP |
| 2g-3 | HEX | PhF | Et | OCT |
| 2g-4 | HEX | PhF | Et | NON |
| 2g-5 | HEX | PhF | Et | DEC |
| 2g-6 | HEP | PhF | Et | HEX |
| 2g-7 | HEP | PhF | Et | HEP |
| 2g-8 | HEP | PhF | Et | OCT |
| 2g-9 | HEP | PhF | Et | NON |
| 2g-10 | HEP | PhF | Et | DEC |
| 2g-11 | OCT | PhF | Et | HEX |
| 2g-12 | OCT | PhF | Et | HEP |
| 2g-13 | OCT | PhF | Et | OCT |
| 2g-14 | OCT | PhF | Et | NON |
| 2g-15 | OCT | PhF | Et | DEC |
| 2g-16 | NON | PhF | Et | HEX |
| 2g-17 | NON | PhF | Et | HEP |
| 2g-18 | NON | PhF | Et | OCT |
| 2g-19 | NON | PhF | Et | NON |
| 2g-20 | NON | PhF | Et | DEC |
| 2g-21 | DEC | PhF | Et | HEX |
| 2g-22 | DEC | PhF | Et | HEP |
| 2g-23 | DEC | PhF | Et | OCT |

Table 2g (2)

| No. | $R_1$ | $A_2$ | $X_2$ | $R_2$ |
|---|---|---|---|---|
| 2g-24 | DEC | PhF | Et | NON |
| 2g-25 | DEC | PhF | Et | DEC |
| 2g-26 | UND | PhF | Et | OCT |
| 2g-27 | UND | PhF | Et | NON |
| 2g-28 | UND | PhF | Et | DEC |
| 2g-29 | DOD | PhF | Et | OCT |
| 2g-30 | DOD | PhF | Et | NON |
| 2g-31 | DOD | PhF | Et | DEC |
| 2g-32 | TED | PhF | Et | OCT |
| 2g-33 | TED | PhF | Et | NON |
| 2g-34 | TED | PhF | Et | DEC |
| 2g-35 | HEX | Ph | Et | HEX |
| 2g-36 | HEX | Ph | Et | HEP |
| 2g-37 | HEX | Ph | Et | OCT |
| 2g-38 | HEX | Ph | Et | NON |
| 2g-39 | HEX | Ph | Et | DEC |
| 2g-40 | HEP | Ph | Et | HEX |

Table 2g (3)

| No. | $R_1$ | $A_2$ | $X_2$ | $R_2$ |
|---|---|---|---|---|
| 2g-41 | HEP | Ph | Et | HEP |
| 2g-42 | HEP | Ph | Et | OCT |
| 2g-43 | HEP | Ph | Et | NON |
| 2g-44 | HEP | Ph | Et | DEC |
| 2g-45 | OCT | Ph | Et | HEX |
| 2g-46 | OCT | Ph | Et | HEP |
| 2g-47 | OCT | Ph | Et | OCT |
| 2g-48 | OCT | Ph | Et | NON |
| 2g-49 | OCT | Ph | Et | DEC |
| 2g-50 | NON | Ph | Et | HEX |
| 2g-51 | NON | Ph | Et | HEP |
| 2g-52 | NON | Ph | Et | OCT |
| 2g-53 | NON | Ph | Et | NON |
| 2g-54 | NON | Ph | Et | DEC |
| 2g-55 | DEC | Ph | Et | HEX |
| 2g-56 | DEC | Ph | Et | HEP |
| 2g-57 | DEC | Ph | Et | OCT |
| 2g-58 | DEC | Ph | Et | NON |
| 2g-59 | DEC | Ph | Et | DEC |
| 2g-60 | UND | Ph | Et | OCT |
| 2g-61 | UND | Ph | Et | NON |
| 2g-62 | UND | Ph | Et | DEC |
| 2g-63 | DOD | Ph | Et | OCT |
| 2g-64 | DOD | Ph | Et | NON |
| 2g-65 | DOD | Ph | Et | DEC |
| 2g-66 | TED | Ph | Et | OCT |
| 2g-67 | TED | Ph | Et | NON |
| 2g-68 | TED | Ph | Et | DEC |

(19) Case of

$R_1$ - 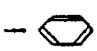 - $A_2$ - $X_2$ - $R_2$ (2h)

same groups as No. 2g-1 to No. 2g-34 in the Table 2g. No. 2h-1 to 2h-34

(20) Case of

$R_1 - C \equiv C -$ 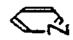 $- A_2 - X_2 - R_2$ (2i)

same groups as No. 2g-1 to No. 2g-68 in the Table 2g. No. 2i-1 to 2i-68

(21) Case of

$R_1 -$ 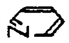 $- A_2 - X_2 - R_2$ (2j)

same groups as No. 2g-1 to No. 2g-34 in the Table 2g. No. 2j-1 to 2j-34

(22) Case of

$R_1 - C \equiv C -$ 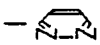 $- A_2 - X_2 - R_2$ (2k)

same groups as No. 2g-1 to No. 2g-68 in the Table 2g. No. 2k-1 to 2k-68

(23) Case of

$R_1 -$ 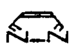 $- A_2 - X_2 - R_2$ (2l)

same groups as No. 2g-1 to No. 2g-34 in the Table 2g. No. 2l-1 to 2l-34

(24) $R_1 - X_1 -$ 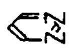 $- A_2 - X_2 - R_2$ (2m)

Table 2m (1)

| No. | $R_1$ | $X_1$ | $A_2$ | $X_2$ | $R_2$ |
|---|---|---|---|---|---|
| 2m-1 | HEX | Et | Ph | Ac | BUT |
| 2m-2 | HEX | Et | Ph | Ac | PEN |
| 2m-3 | HEX | Et | Ph | Ac | HEX |
| 2m-4 | HEX | Et | Ph | Ac | HEP |
| 2m-5 | HEX | Et | Ph | Ac | OCT |
| 2m-6 | HEP | Et | Ph | Ac | BUT |
| 2m-7 | HEP | Et | Ph | Ac | PEN |
| 2m-8 | HEP | Et | Ph | Ac | HEX |
| 2m-9 | HEP | Et | Ph | Ac | HEP |
| 2m-10 | HEP | Et | Ph | Ac | OCT |
| 2m-11 | OCT | Et | Ph | Ac | BUT |
| 2m-12 | OCT | Et | Ph | Ac | PEN |
| 2m-13 | OCT | Et | Ph | Ac | HEX |
| 2m-14 | OCT | Et | Ph | Ac | HEP |
| 2m-15 | OCT | Et | Ph | Ac | OCT |
| 2m-16 | OCT | Et | Ph | Ac | NON |
| 2m-17 | NON | Et | Ph | Ac | BUT |

Table 2m (2)

| No. | $R_1$ | $X_1$ | $A_2$ | $X_2$ | $R_2$ |
|---|---|---|---|---|---|
| 2m-18 | NON | Et | Ph | Ac | PEN |
| 2m-19 | NON | Et | Ph | Ac | HEX |
| 2m-20 | NON | Et | Ph | Ac | HEP |
| 2m-21 | NON | Et | Ph | Ac | OCT |
| 2m-22 | NON | Et | Ph | Ac | NON |
| 2m-23 | NON | Et | Ph | Ac | DEC |
| 2m-24 | DEC | Et | Ph | Ac | BUT |
| 2m-25 | DEC | Et | Ph | Ac | PEN |
| 2m-26 | DEC | Et | Ph | Ac | HEX |
| 2m-27 | DEC | Et | Ph | Ac | HEP |
| 2m-28 | DEC | Et | Ph | Ac | OCT |
| 2m-29 | DEC | Et | Ph | Ac | NON |
| 2m-30 | DEC | Et | Ph | Ac | DEC |
| 2m-31 | DOD | Et | Ph | Ac | OCT |
| 2m-32 | DOD | Et | Ph | Ac | NON |
| 2m-33 | DOD | Et | Ph | Ac | DEC |
| 2m-34 | DOD | Et | Ph | Ac | DOD |

(25) $R_3 - X_3 -$ 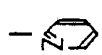 $- C \equiv C - A_3 - X_4 - R_4$ (3a)

Table 3 a (1)

| No. | $R_3$ | $X_1$ | $A_3$ | $X_2$ | $R_4$ |
|---|---|---|---|---|---|
| 3a-1 | DEC | Et | Ph | Et | $CF_3$-$C_6$ |
| 3a-2 | DEC | Et | Ph | Es > | $CF_3$-$C_6$ |
| 3a-3 | DEC | Et | Ph | MO | $CF_3$-$C_6$ |
| 3a-4 | DEC | D | Ph | Et | $CF_3$-$C_6$ |
| 3a-5 | DEC | D | Ph | Es > | $CF_3$-$C_6$ |
| 3a-6 | DEC | D | Ph | MO | $CF_3$-$C_6$ |
| 3a-7 | DEC | Et | PhF | Es > | $CF_3$-$C_6$ |
| 3a-8 | DEC | Et | PhF | MO | $CF_3$-$C_6$ |
| 3a-9 | DEC | D | PhF | Es > | $CF_3$-$C_6$ |
| 3a-10 | DEC | D | PhF | MO | $CF_3$-$C_6$ |
| 3a-11 | $CF_3$-$C_6$ | Et | Ph | D | DEC |
| 3a-12 | $CF_3$-$C_6$ | Et | Ph | Et | DEC |
| 3a-13 | $CF_3$-$C_6$ | Et | PhF | Et | DEC |
| 3a-14 | 2MB | Et | Ph | Et | $CF_3$-$C_6$ |
| 3a-15 | $CF_3$-$C_6$ | Et | Ph | Et | 2MB |
| 3a-16 | $CF_3$-$C_6$ | Et | PhF | Et | 2MB |

(25) Case of

$R_3 - X_3 -$ 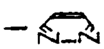 $- C \equiv C - A_3 - X_4 - R_4$ (3b)

same groups as No. 3a-1 to No. 3a-16 in the Table 3a. No. 3b-1 to 3b-16

(26) Case of

$R_3 - X_3 -$ 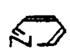 $- CH_2 CH_2 - A_3 - X_4 - R_4$ (3b)

same groups as No. 3a-1 to No. 3a-16 in the Table 3a. No. 3c-1 to 3c-16

(27) Case of

$R_3 - X_3 -$ 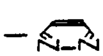 $- CH_2 CH_2 - A_3 - X_4 - R_4$ (3d)

same groups as No. 3a-1 to No. 3a-16 in the Table 3a. No. 3c-1 to 3c-16

(28) $R_3 - X_3 - A_4 - C \equiv C -$ 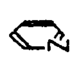 $-$ 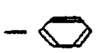 $- X_4 - R_4$ (4 a)

Table 4a (1)

| No. | $R_3$ | $X_1$ | $A_4$ | $X_2$ | $R_4$ |
|---|---|---|---|---|---|
| 4a−1 | HEX | Et | FPh | Et | 2MB |
| 4a−2 | HEX | Et | FPh | Et | r−2MB |
| 4a−3 | HEX | Et | FPh | Et | 4MH |
| 4a−4 | HEX | Et | FPh | Et | r−4MH |
| 4a−5 | 2MB | Et | FPh | Et | HEX |
| 4a−6 | r−2MB | Et | FPh | Et | HEX |
| 4a−7 | 4MH | Et | FPh | Et | HEX |
| 4a−8 | r−4MH | Et | FPh | Et | HEX |
| 4a−9 | HEX | Et | Ph | Et | 2MB |
| 4a−10 | HEX | Et | Ph | Et | r−2MB |
| 4a−11 | HEX | Et | Ph | Et | 4MH |
| 4a−12 | HEX | Et | Ph | Et | r−4MH |
| 4a−13 | 2MB | Et | Ph | Et | HEX |
| 4a−14 | r−2MB | Et | Ph | Et | HEX |
| 4a−15 | 4MH | Et | Ph | Et | HEX |
| 4a−16 | r−4MH | Et | Ph | Et | HEX |
| 4a−17 | 2MB | <Es | FPh | Et | HEX |

Table 4a (2)

| No. | $R_3$ | $X_1$ | $A_4$ | $X_2$ | $R_4$ |
|---|---|---|---|---|---|
| 4a−18 | 4MH | <Es | FPh | Et | HEX |
| 4a−19 | 2MB | <Es | Ph | Et | HEX |
| 4a−20 | 4MH | <Es | Ph | Et | HEX |
| 4a−21 | HEX | D | Ph | Et | 2MB |
| 4a−22 | HEX | D | Ph | Et | r−2MB |
| 4a−23 | HEX | D | Ph | Et | 4MH |
| 4a−24 | HEX | D | Ph | Et | r−4MH |
| 4a−25 | HEX | Et | Ph | Et | $CH_3-C_6$ |
| 4a−26 | HEX | Et | FPh | Et | $CH_3-C_6$ |
| 4a−27 | HEX | D | Ph | Et | $CH_3-C_6$ |
| 4a−28 | $CH_3-C_6$ | Et | FPh | Et | HEX |
| 4a−29 | $CH_3-C_6$ | Et | Ph | Et | HEX |
| 4a−30 | $CH_3-C_6$ | <Es | FPh | Et | HEX |
| 4a−31 | $CH_3-C_6$ | <Es | Ph | Et | HEX |
| 4a−32 | $CF_3-C_6$ | <Es | FPh | Et | HEX |
| 4a−33 | $CF_3-C_6$ | <Es | Ph | Et | HEX |
| 4a−34 | 2MB | Et | FPh | D | HEX |

Table 4 a (3)

| No. | $R_3$ | $X_1$ | $A_4$ | $X_2$ | $R_4$ |
|---|---|---|---|---|---|
| 4a-35 | r-2MB | Et | FPh | D | HEX |
| 4a-36 | 2MB | Et | Ph | D | HEX |
| 4a-37 | r-2MB | Et | Ph | D | HEX |
| 4a-38 | 2MB | <Es | FPh | D | HEX |
| 4a-39 | $CH_3-C_6$ | Et | FPh | D | HEX |
| 4a-40 | $CH_3-C_6$ | Et | Ph | D | HEX |
| 4a-41 | $CH_3-C_6$ | <Es | FPh | D | HEX |
| 4a-42 | $CH_3-C_6$ | <Es | Ph | D | HEX |
| 4a-43 | $CF_3-C_6$ | <Es | FPh | D | HEX |
| 4a-44 | $CF_3-C_6$ | <Es | Ph | D | HEX |

(29) Case of

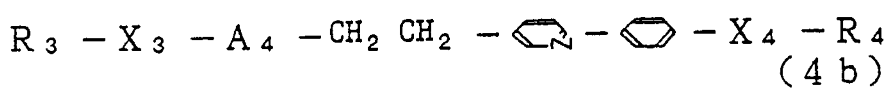

(4 b)

same groups as No. 4a-1 to No. 4a-44 in the Table 4a. No. 4b-1 to 4b-44

(30) Case of

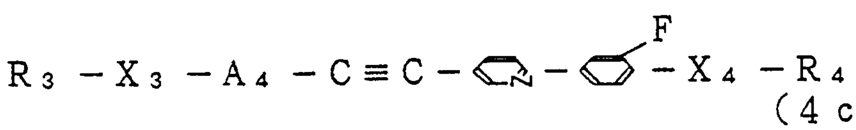

(4 c)

same groups as No. 4a-1 to No. 4a-33 in the Table 4a. No. 4c-1 to 4c-33

(31) Case of

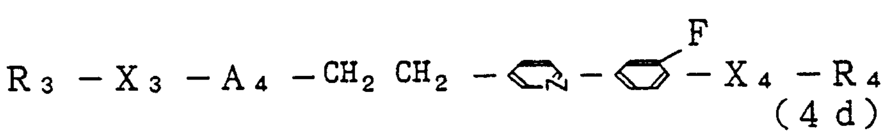

(4 d)

same groups as No. 4a-1 to No. 4a-33 in the Table 4a. No. 4d-1 to 4d-33

(32) Case of

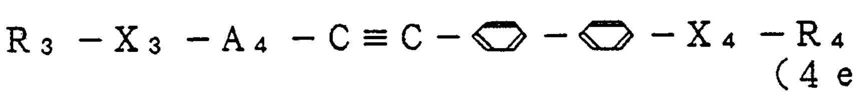

(4 e)

same groups as No. 4a-1 to No. 4a-44 in the Table 4a. No. 4e-1 to 4e-44

(33) Case of

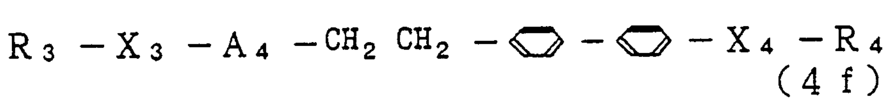

(4 f)

same groups as No. 4a-1 to No. 4a-44 in the Table 4a. No. 4f-1 to 4f-44

(34) Case of

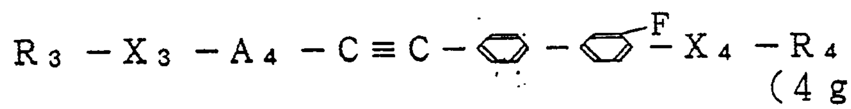

(4 g)

same groups as No. 4a-1 to No. 4a-33 in the Table 4a. No. 4g-1 to 4g-33

(35) Case of

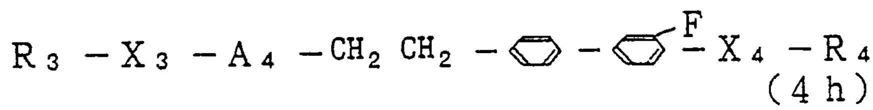

(4 h)

same groups as No. 4a-1 to No. 4a-33 in the Table 4a. No. 4h-1 to 4h-33

Those compounds which are represented by the formulas (1), for instance, may be synthesized through the process disclosed below.

($R_1$, $X_1$, $A_1$, $A_2$, n, $A_3$, $X_2$ and $R_2$ are commonly used both in the following formulas and formula (1))

[1] Where n is equal to 0 and Y equal to -C≡C-($X_1$ is direct single bond when $A_1$ is

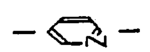

) :

$R_1-X_1-A_1-Y$ (Y is halogen group) (5)

$HC\equiv C-A_3-X_2-R_2$ (6)

[Pd] and CuI

$R_1-X_1-A_1-C\equiv C-A_3-X_2-R_2$ (1a)

As suggested by the above equations the compound (1a) may be obtained in such a manner that the compounds represented by (5) and by (6) are allowed to react within triethylamine in the presence of palladium catalyst with valency 0 or 2 under inert gas atmosphere.

Alternatively

$R_1-X_1-A_1-Y$ (5)

$HOC(CH_3)_2-C\equiv CH$

[Pd] and CuI

$R_1-X_1-A_1-C\equiv C-C(CH_3)_2-OH$ (7)

NaOH

$$R_1-X_1-A_1-C\equiv CH \qquad (8)$$

$$Y-A_3-X_2-R_2 \qquad (9)$$

[Pd] and CuI

$$R_1-X_1-A_1-C\equiv C-A_3-X_2-R_2 \qquad (1a)$$

As suggested by the above equtions, the compound proposed by the formula (8) may be obtained in such a manner that compound (5) and 3-methyl-1-butyne-3-ol are allowed to react within triethylamine in the presence of palladium catalyst with valency 0 or 2 under inert gas atmosphere so as to produce the reactant represented by the equation (7), upon which sodium hydroxide in powder form is further allowed to act within dry toluene.

Where compounds represented by the formula (8) and (9) were allowed to react within triethylamine in the presence of palladium catalyst with valency of 0 or 2 under inert gas atmosphere, the compound proposed by the formula (1a) is yielded.

Those compounds represented by the formulas (5) and (8), for instance, may be synthesized through the process disclosed below.

(a) Where $X_1$ is single bond and $A_1$ is 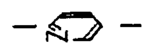 :

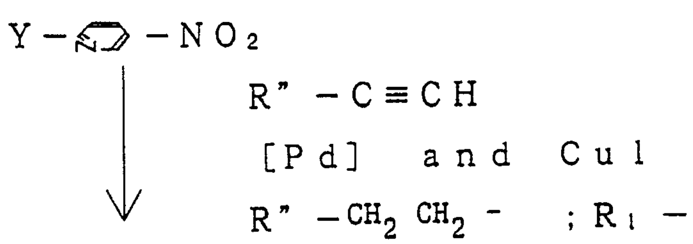


(10)

(11)

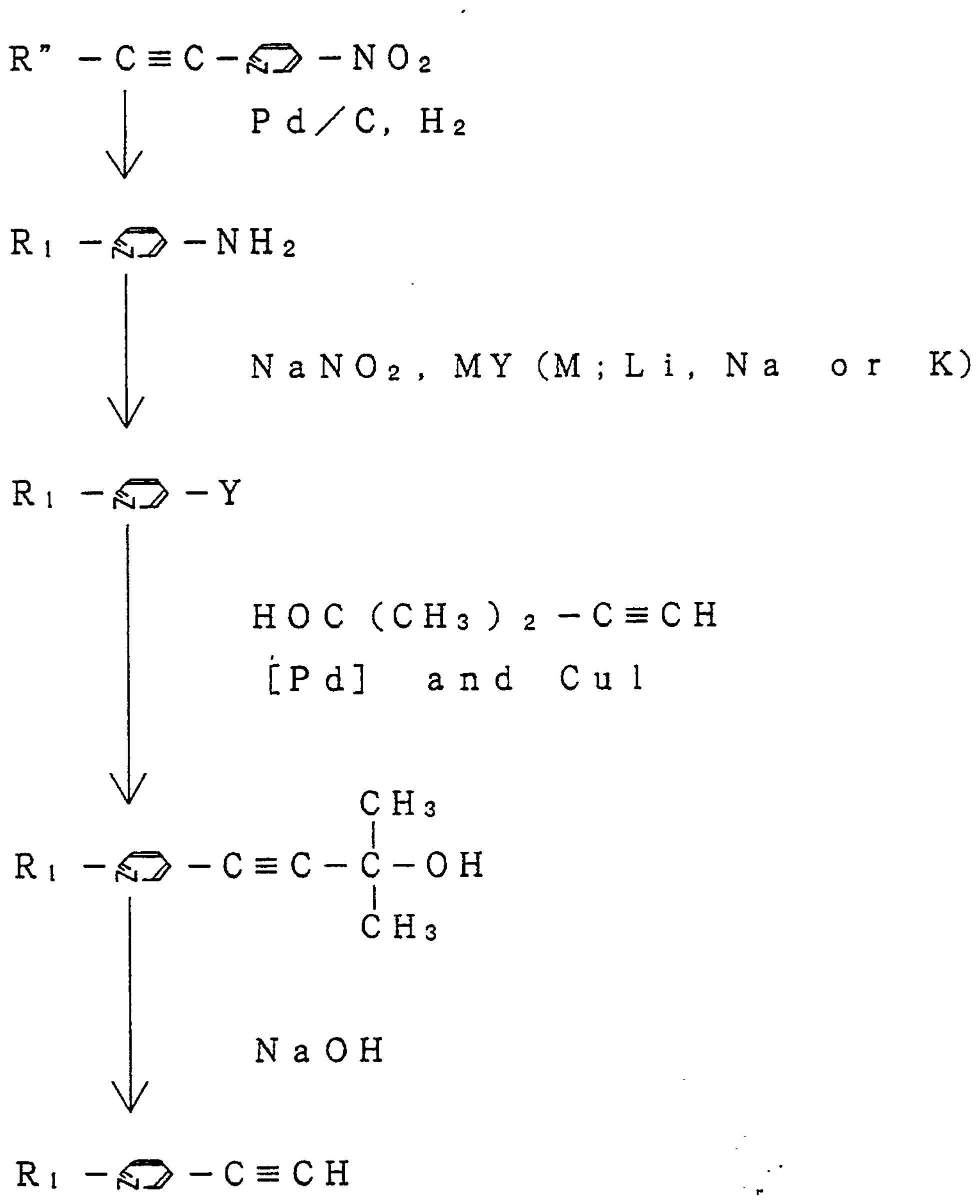


(12)

(13)

(5a)

(14)

(8a)

As suggested by the above equations, the compound proposed by the formula (12) may be obtained in such a manner that compounds (10) and (11) are allowed to react within triethylamine in the presence of palladium catalyst of valency 0 or 2 under inert gas atmosphere. Through hydrogenation of products of the formula (12) under hydrogen atmosphere using palladium carbon catalyst proposed by the formula (13) is yielded. Through diazotation of compound proposed by the formula (13) followed by allowing the product to react with alkaline wetal halide. The compound represented by the formula (5a) as the raw material useful for the compound proposed by the present invention is yielded.

Where compound represented by the formula (5a) and 3-methyl-1-butyne-3-ol are allowed to react within triethylamine in the presence of palladium catalyst of valency 0 or 2 under inert gas atmosphere, the compound represented by the formula (14) is yielded. Where sodium hydroxide in power form acts upon the last mentioned product within dry toluene. Then the compound represented by the formula (8a) as the raw material for the compound proposed by the present invention is yielded.

In addition, the compound represented by the formula (5a) may also be obtained through such process as illustrated below.

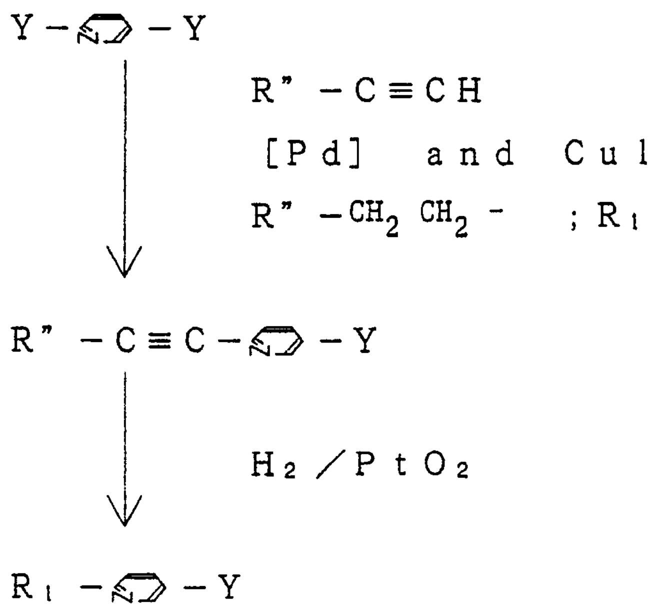


(15)

(11)

(16)

(5a)

Where as suggested by the above equations the compounds of (15) and (11) are allowed to react within triethylamine in the presence of palladium catalyst of valency 0 or 2 under inert gas atmosphere, the compound represented by the formula (16) is yielded. Through hydrogenation of last mentioned product under hydrogen atmosphere using platinum oxide, the compound represented by the formula (5a) as the raw material for the compound proposed in the present invention is yielded.

(b) Where $X_1$ is -O- and $A_1$ is 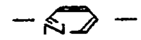 :

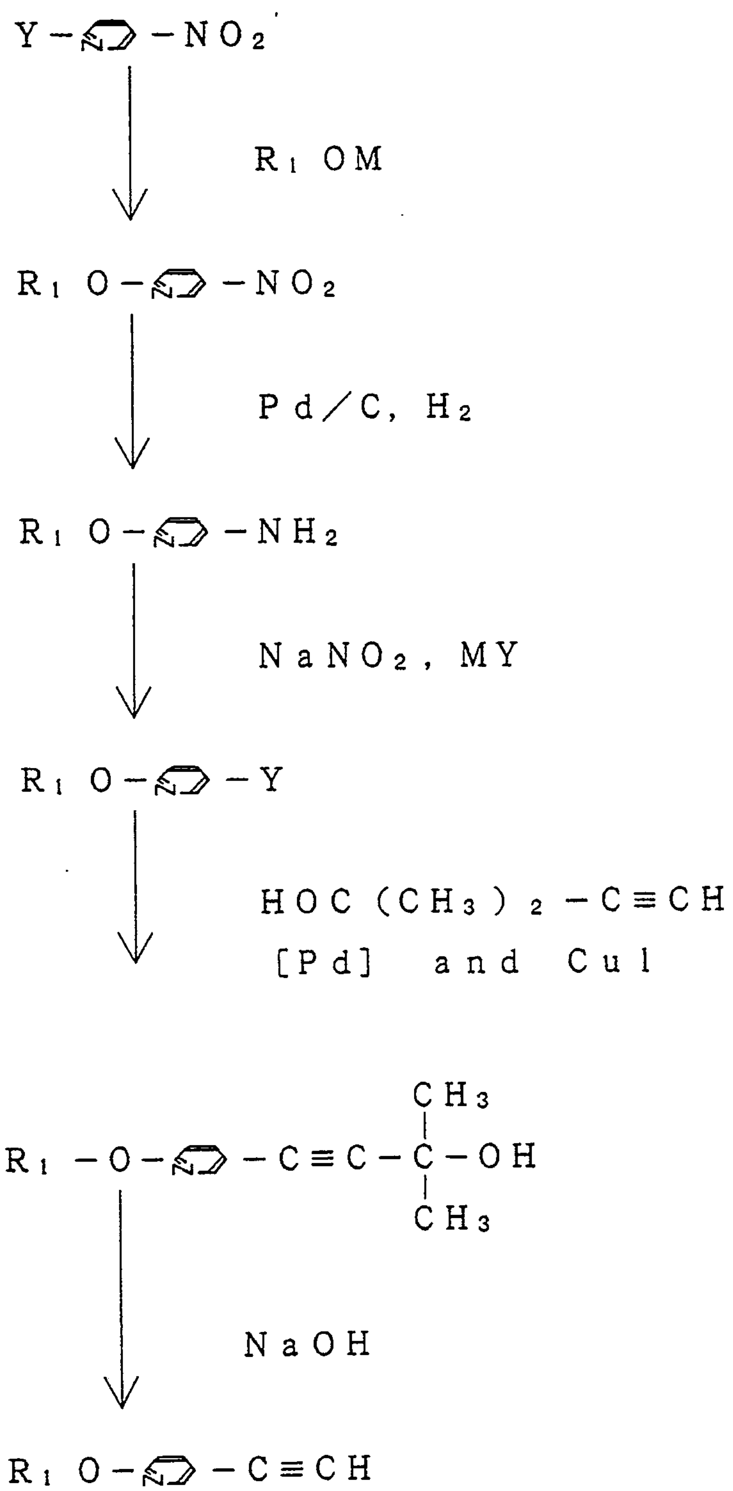


(10)

(17)

(18)

(5b)

(19)

(8b)

Where as suggested by the above equations the compound represented by the formula (10) and alkali metal salt of alcohol are allowed to react within dry dimethylformamide or dry dimethylsulfoxide, the compound represented by the formula (17) is yielded. Through hydrogenation of last mentioned product under hydrogen atmosphere using palladium-carbon, the compound represented by the formula (18) is yielded.

Through diazotating the last mentioned compound followed by allowing the resulted product to react with alkaline metal halide the compound represented by the formula (5b) as the raw material for the compound represented by the present invention is yielded. Where the compound represented by the

formula (5b) and 3-metyl-1-butyne-3-ol are allowed to react within triethylamine in the presence of palladium catalyst of valency 0 or 2 under inert gas atmosphere, the compound represented by the formula (19) is yielded.

Where sodium hydroxide in powder form is allowed to act upon the last mentioned product within dry toluene, the compound represented by the formula (8b) as the raw material for the compound proposed in the present invention is yielded.

The compound represented by the formula (5b) may be obtained also through the process illustrated below.

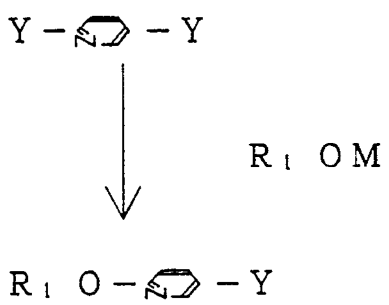


(15)

(5b)

Where as suggested by the above equation, the compound represented by the formula (15) and alkali metal salt of alcohol are allowed to react within dry dimethylformamide or dry diemethylsulfoxide, the compound represented by the formula (5b) as the raw material for the compound proposed in the present invention is yielded.

(c) Where $X_1$ is $-C\equiv C-$ and $A_1$ is 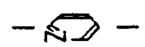 :

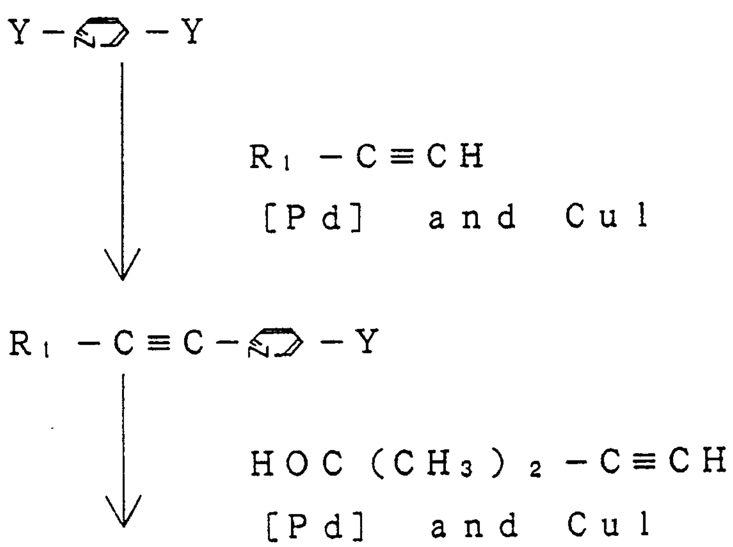


(15)

(20)

(5c)

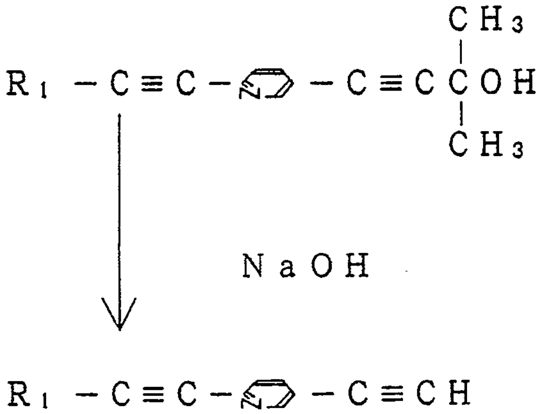


(21)

(8c)

Where the compound represented by the formula (15) and 1-alkyne represented by the formula (20) are allowed to react within triethylamine in the presence of palladium catalyst of valency 0 or 2 under inert gas atmosphere, the compound represented by the formula (5c) as the raw material for the compound proposed in the present invention is yielded. Where the last mentioned compound and 3-methyl-1-butyne-3-ol are allowed to react within triethylamine in the presence of palladium catalyst of valency 0 or 2 under inert gas atmosphere, the compound represented by the formula (21) is yielded.

Where sodium hydroxide in powder form is allowed to act upon the last mentioned compound within dry toluene, the compound represented by the formula (8c) as the raw material for the compound proposed in the present invention is yielded.

(d) Where $X_1$ is single bond and A is 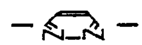 :

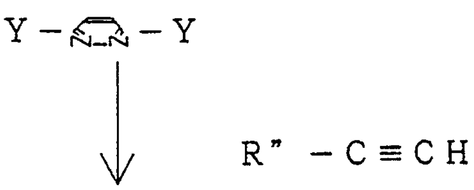


(22)

(11)

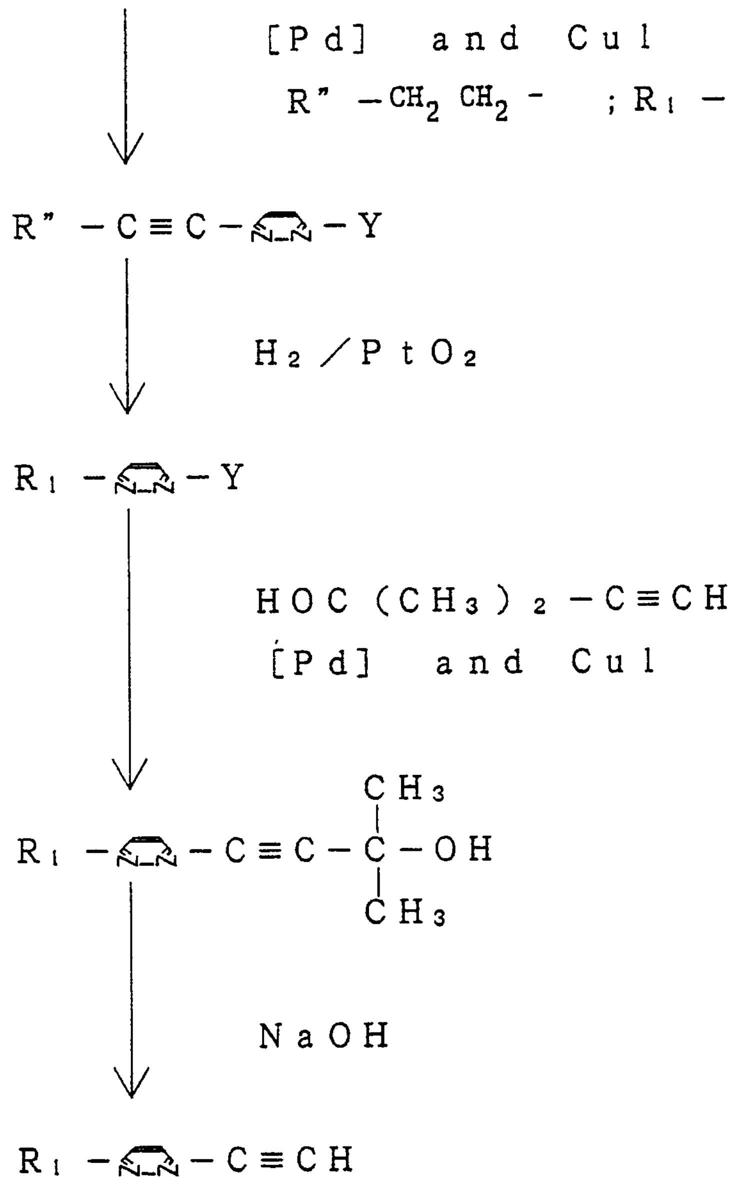


(23)

(5d)

(24)

(8d)

Where as suggested by the above equations the compounds represented by the formula (22) and (11) are allowed to react within triethylamine in the presence of palladium catalyst of valency 0 or 2 under inert gas atomosphere, the compound reprsented by the formula (23) is yielded. Through hydrogenation the last mentioned compound under hydrogen atmosphere using platinum oxide, the compound represented by the formula (5d) as the raw material for the compound proposed in the present invention is yielded. Where the last mentioned compound and 3-methyl-1-butyne-3-ol are allowed to react within triethylamine in the presence of palladium catalyst of valency 0 or 2 under inert gas atmosphere, the compound represented by the formula (24) is yielded. Where sodium hydroxide in powder form is allowed to act upon the last mentioned compound within dry toluene, the compound represented by the formula (8d) as the raw material for the compound proposed in the present invention is yeilded.

(e) Where $X_1$ is -O- and A is 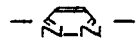 :

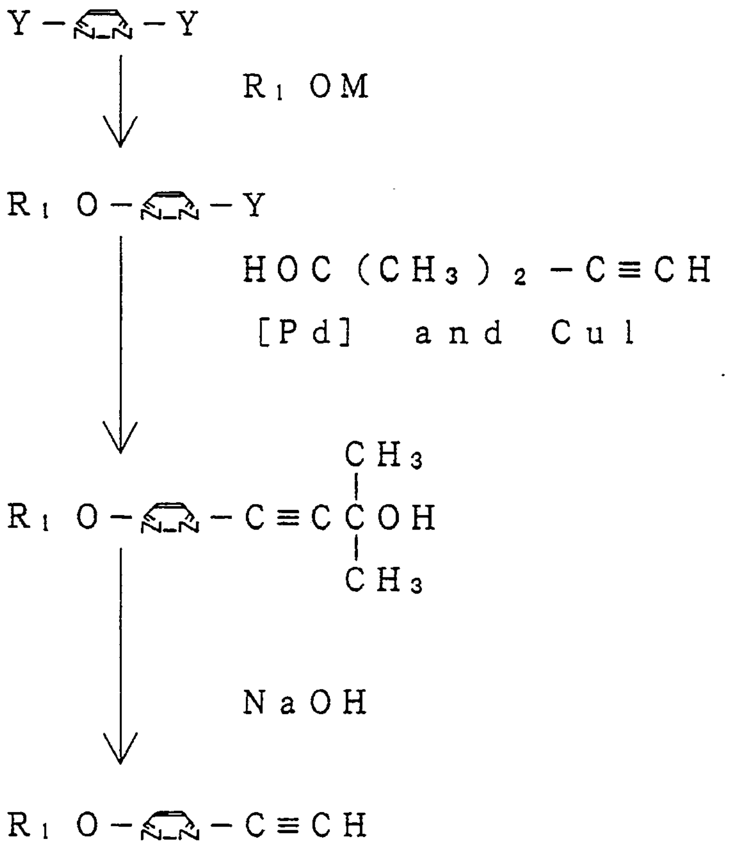


(22)

(5e)

(25)

(8e)

Where as suggested by the above equations, the compound represented by the formula (22) and alkali metal salt of alcohol are allowed to react within dry dimethylformamide or dry dimethylsulfoxide, the compound represented by the formula (5e) as the raw material for the compound proposed in the present invention is yielded. Where the last mentioned compound and 3-methyl-1-butyne-3-ol are allwoed to react within triethylamine in the presence of palladium catalyst of valency 0 or 2 under inert gas atmosphere, the compound represented by the formula (25) is yielded. Where sodium hydroxide in powder form is allowed to act upon the last mentioned compound within dry toluene, the compound represented by the formula (8e) as the raw material for the compound represented in the present invention is yielde.

(f) Where $X_1$ is $-C \equiv C-$ and A is 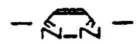 :

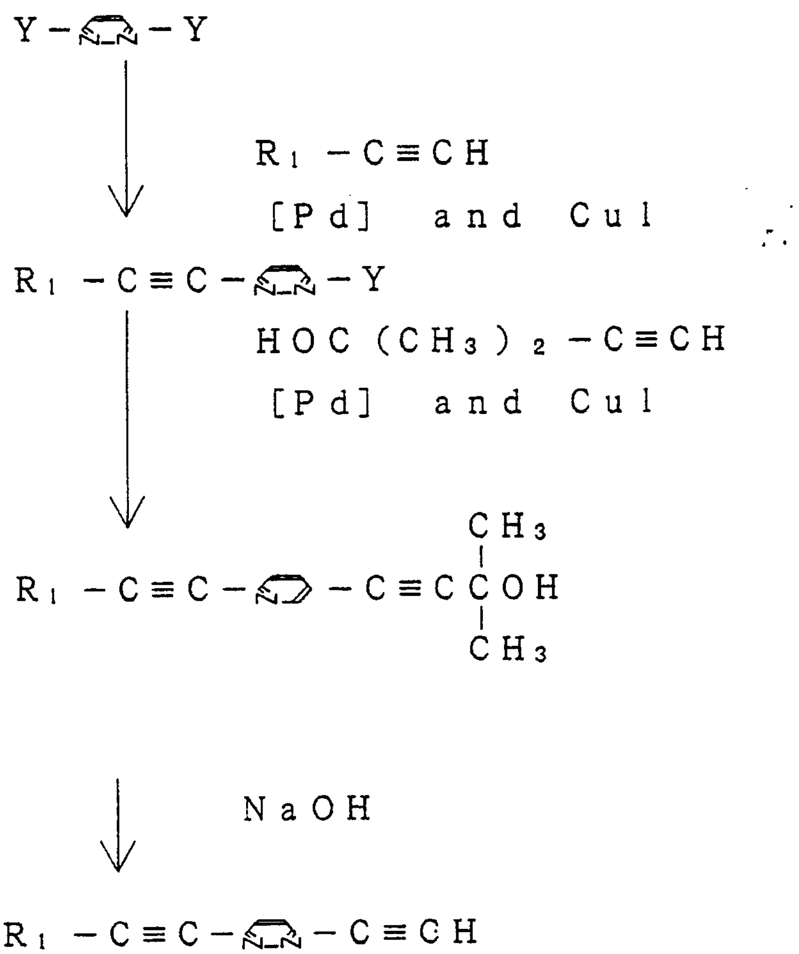


(22)

(20)

(5f)

(26)

(8f)

Where as suggested by the above equations, the compound represented by the formula (22) and 1-alkyne represented by the formula (20) are allowed to react within triethylamine in the presence of palladium catalyst of valency 0 or 2 under inert gas atmosphere, the compound represented by the formula (5f) as the raw material for the compound proposed in the present invention is yielded. Where the last mentioned compound and 3-methyl-1-butyne-3-ol are allowed to react within triethylamine in the presence of palladium catalyst of valency 0 or 2 under inert gas atmosphere, the compound represented by the formula (26) is yielded. Where sodium hydroxide in powder form is allowed to act upon the last mentioned compound within dry toluene, the compound represented by the formula (8f) as the raw material for the compound proposed in the present invention is yielded.

(g) Where $X_1$ is direct single bond and A is 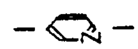 :

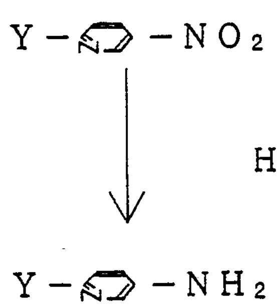


(10)

(27)

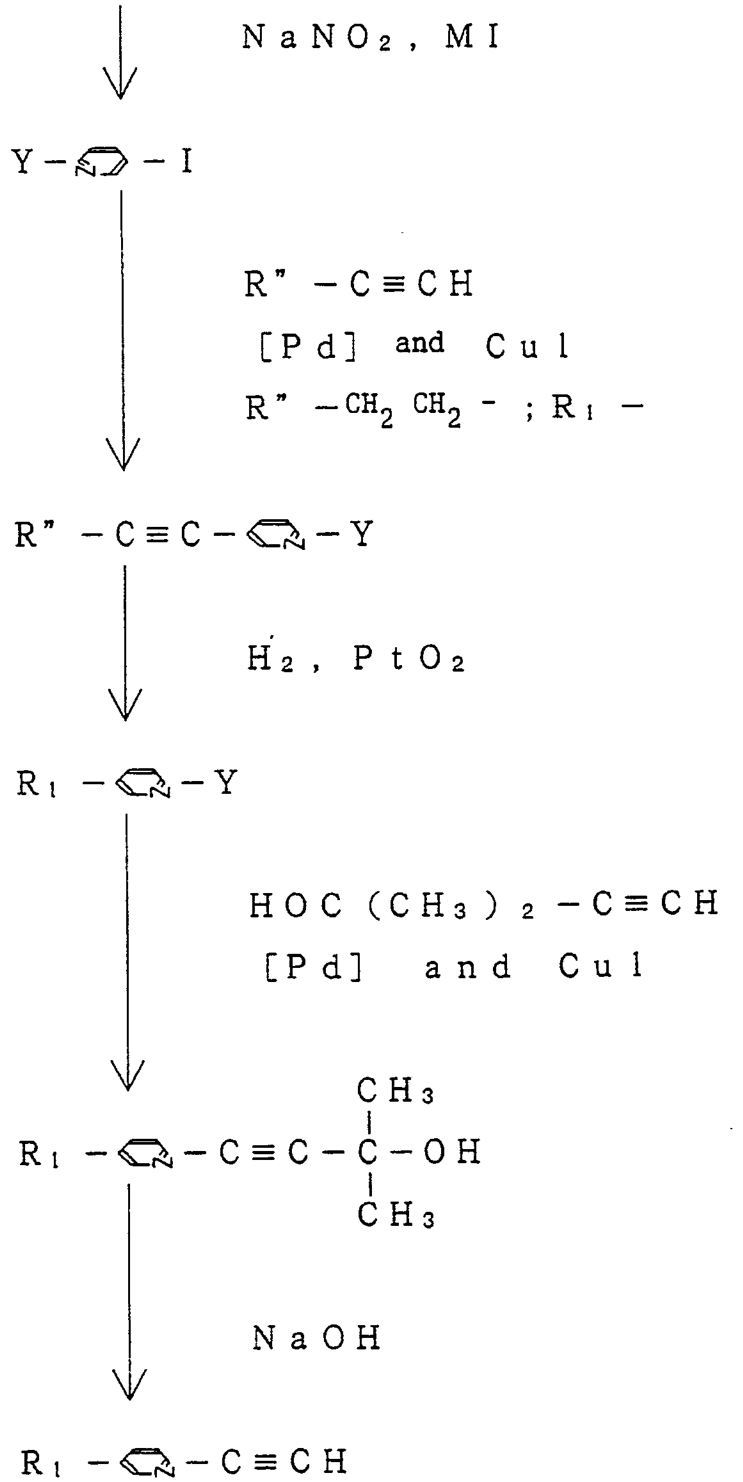


(28)

(11)

(29)

(5g)

(30)

(8g)

As suggested by the above equations, the compound represented by the formula (27) is yielded through hydrogenation the compound represented by the formula (10) under hydrogen atmosphere using platinum oxide. Where the compound represented by the formula (27) is diazotated and then allowed to react with alkaline metal iodide, the compound represented by the formula (28) is yielded. Where the last mentioned compound (28) and the compound represented by the formula (11) are allowed to react within triethylamine in the presence of palladium catalyst of valency 0 or 2 under inert gas atmosphere, the compound represented by the formula (29) is yielded. Through hydrogenation the last mentioned compound under hydrogen atmosphere using platinum oxide, the compound represented by the formula (5g) is yielded. Where last mentioned compound and 3-methyl-1-butyne-3-ol are allowed to react within

triethylamine in the presence of palladium catalyst of valency 0 or 2 under inert gas atmosphere, the compound represented by the formula (30) is yielded. Through allowing sodium hydroxide in powder form to act upon the last mentioned compoud within dry toluene, the compound represented by formula (8g) as the raw material for the compound proposed in the present invention is yielded.

(h) Where $X_1$ is direct single bond or -O- and A is 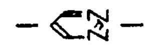 :

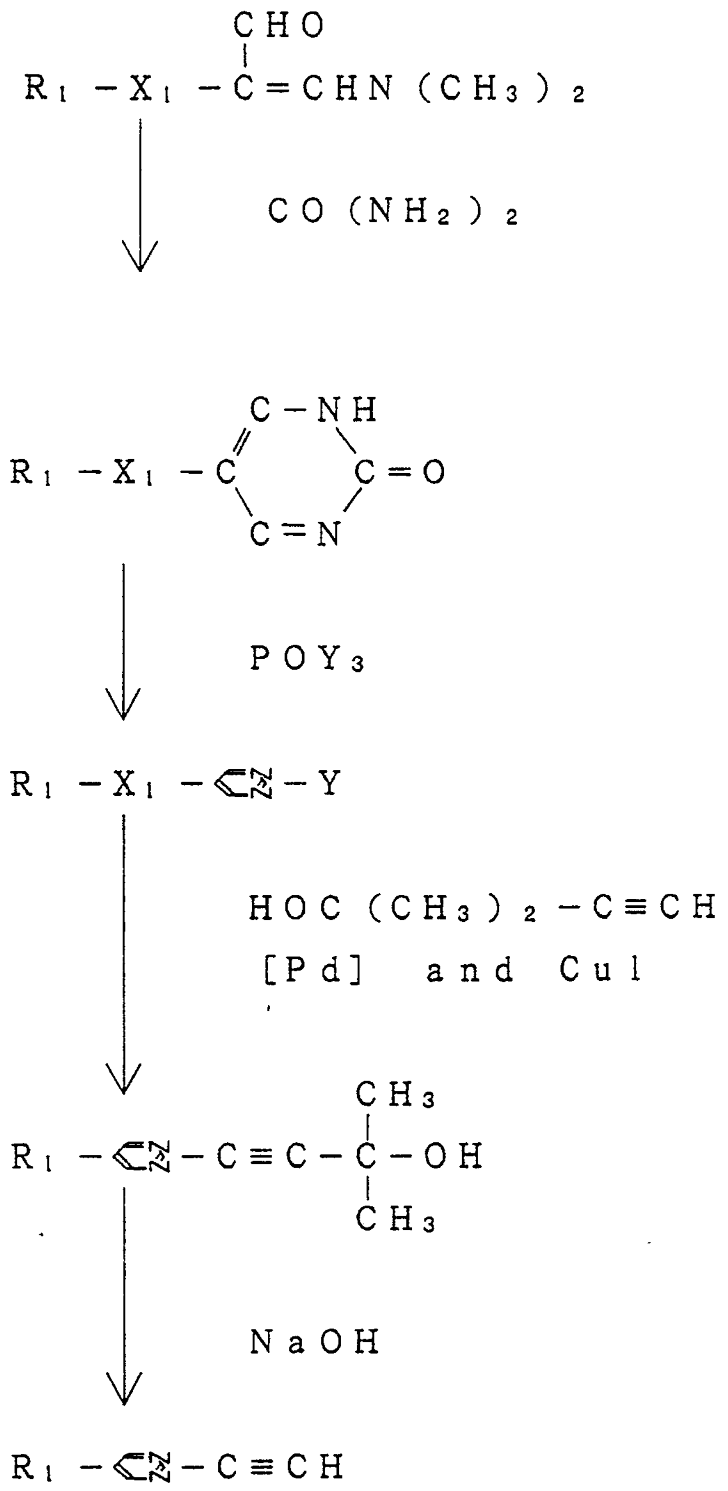


(31)

(32)

(5h)

(33)

(8h)

Through allowing the compound represented by the formula (31) and urea to react in the presence of acid catalyst, the compound represented by the formula (32) is yielded. Through allowing halogenating reagent (e.g. phosphorus oxychloride) to aot upon the last mentioned compound, the compound represented by the formula (5h) as the raw material for the compound proposed in the present invention is yielded. Where the compound represented by the formula (5h) and 3-methyl-1-butyne-3-ol are allowed to react within triethylamine in the presence of palladium catalyst of valency 0 or 2 under inert gas atmosphere, the compound represented by the formula (33) is yielded. Through allowing sodium hydroxide in powder form to act upon the last mentioned compound within dry toluene, the compound represented by the formula (8h) as the raw material for the compound proposed in the present invention is yielded.

The compound represented by the formula (31) may also be obtained through the process illustrated e.g. as follows.

(a) Where $X_1$ is direct single bond:

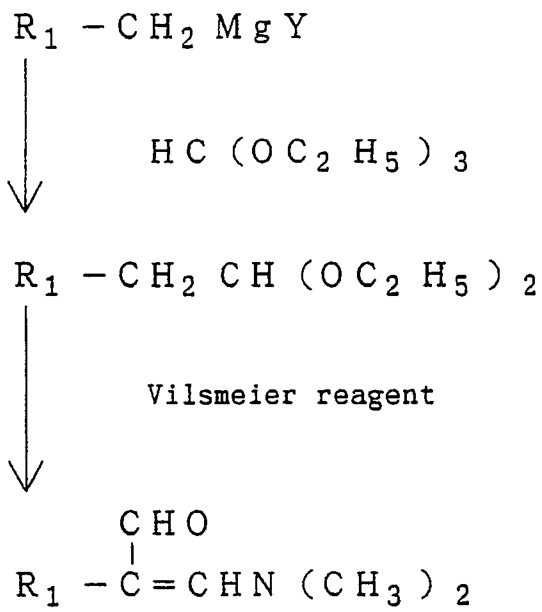


(33a)

(33b)

(31a)

Where as suggested by the above equations, the compound represented by (33a) and triethylorthoformate are allowed to react, the compound represented by (33b) is yielded. Through causing Vilsmeier reagent (e.g. reaction product from phosphorus oxychloride and dimethylformamide) to act upon the last mentioned compound, the compound represented by (31a) may be yielded.

(b) Where $X_1$ is -O-:

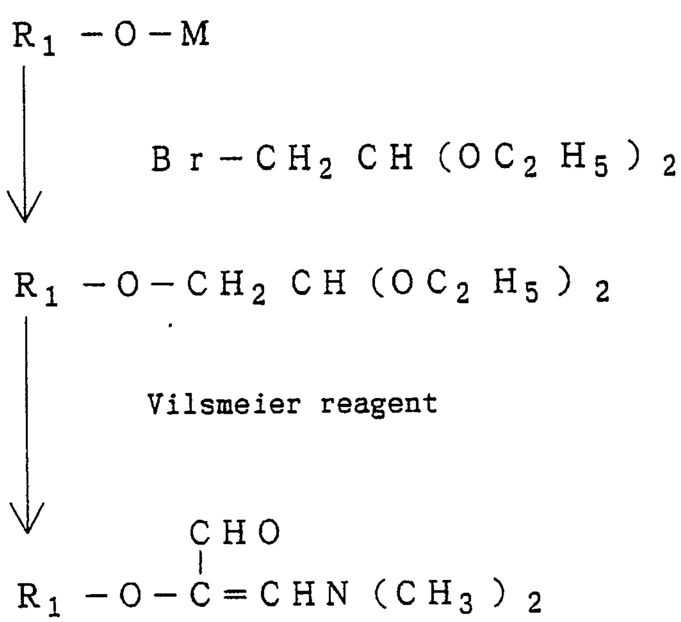


(34)

(35)

(31b)

Where the compound represented by (34) and bromoacetoaldehydediethylacetal are allowed to react within dry dimethylformamide, dry dimethysulfoxide or dry tetrahydrofuran under heated condition, the compound represented by (35) is yielded. Through causing Vilsmier reagent (e.g. reaction product from trichlomethylchloroformate and dimethylformamide) to act upon the last mentioned compound, the compound represented by (31b) may be yielded.

In addition, those compounds from (6) and (9) are made synthesized through the process e.g, as introduced below.

(a) Where $X_2$ is -O- and $A_3$ is 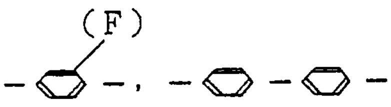 or 2,6-naphtylene group:

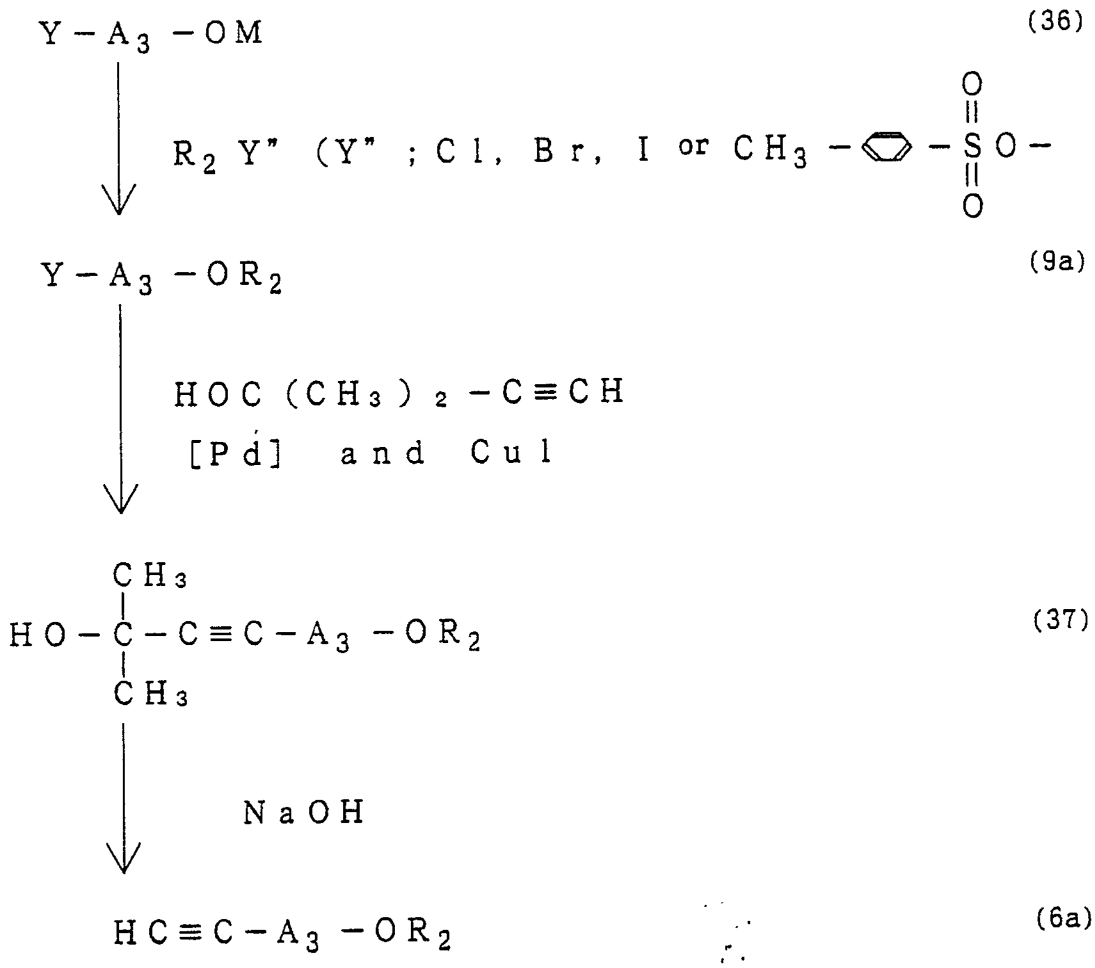


(36)

(9a)

(37)

(6a)

Where as suggested by the above equations, the compound represented by (36) is subjected to alkylation through alkylating reagent (e.g. alkyl halide), the compound represented by (9a) as the raw material for the compound proposed in the present invention is yielded. Where the compound represented by (9a) and 3-methyl-1-butyne-3-ol are allowed to react within triethylamine in the presence of palladium catalyst of valency 0 or 2, under inert gas atmosphere, the compound represented by the fomula (37) is yielded.

Through allowing sodium hydroxide in powder form to act upon the last mentioned compound within dry toluene, the compound represented by the formula (6a) as the raw material for the compound proposed in the present invention is yielded.

(b) Where $X_2$ is -O-, and $A_3$ is 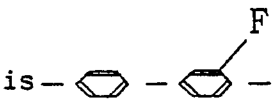 :

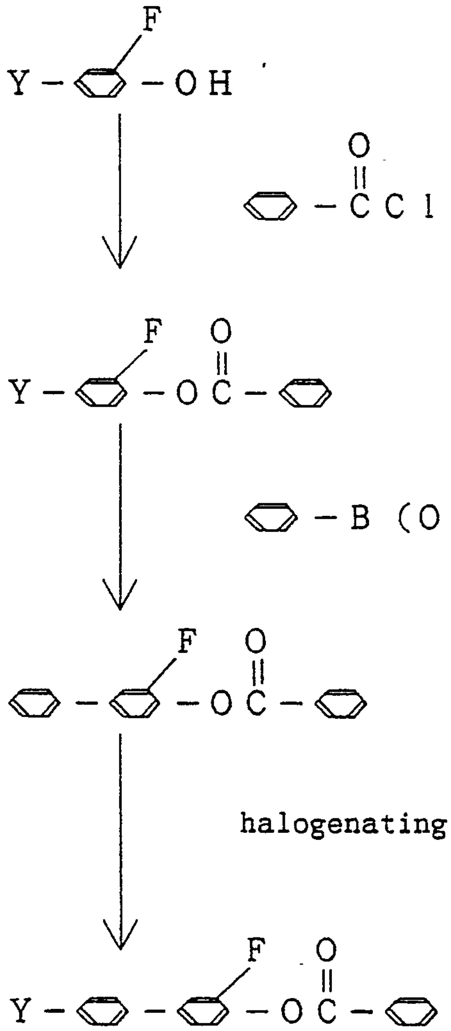


(38)

(39)

(40)

(41)

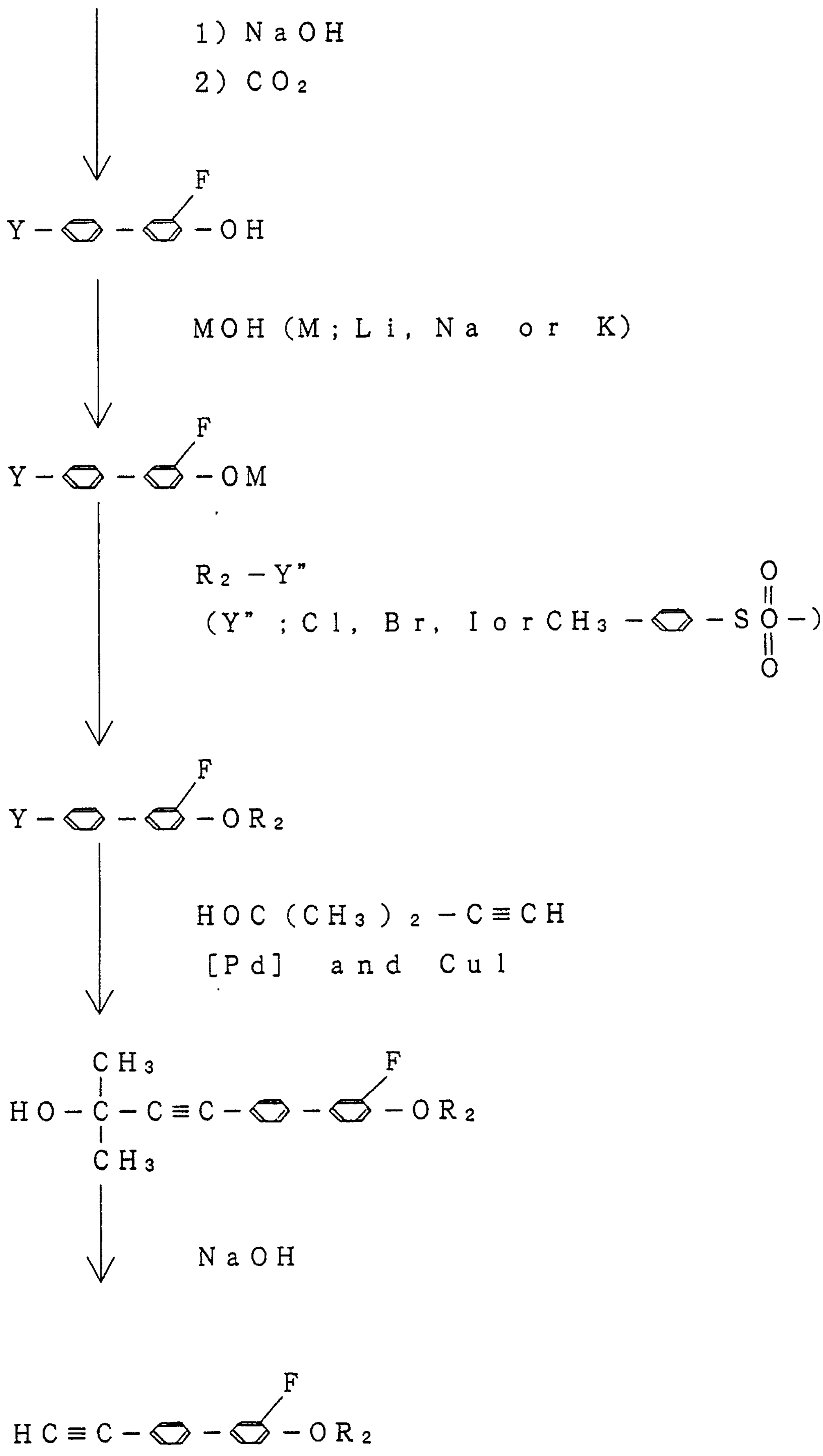
1) NaOH
2) $CO_2$
MOH (M; Li, Na or K)
$R_2$ − Y"
(Y"; Cl, Br, I or $CH_3$
HOC($CH_3$)$_2$ − C≡CH
[Pd] and CuI
NaOH

(42)

(43)

(9b)

(44)

(6b)

Where as suggested in the above the compound represented by (39) derived from the compound of (38) through esterification using benzoylchloride and phenyl boric acid are allowed to react in the presence of palladium catalyst, the compound represented by (40) is yielded. Through halogenation the last mentioned compound using halogenating reagent (e.g. periodic acid and iodine) followed by isolation and purification through hydrolysis using alkali (sodium hydroxide), the compound represented by (42) is yielded. Where the last mentioned compound and alkali metal hydroxide are allowed to react for subsequent alkylation using alkylating reagent (e.g. alkyl halide), the compound represented by (9b) as the raw material for the compound proposed in the present invention is yielded. Where the compound represented by (9b) and 3-methyl-1-butyne-3-ol are allowed to react within triethyamine in the presence of palladium catalyst of valency 0 or 2 under inert gas atmosphere to yield the compound represented by (44) and then sodium hydroxide in powder form is allowed to act upon the last mentioned compound within dry toluene, the compound represented by (6b) as the raw material for the compound proposed in the present invention is yielded.

(c) Where $X_2$ is direct single bond, and $A_3$ is :

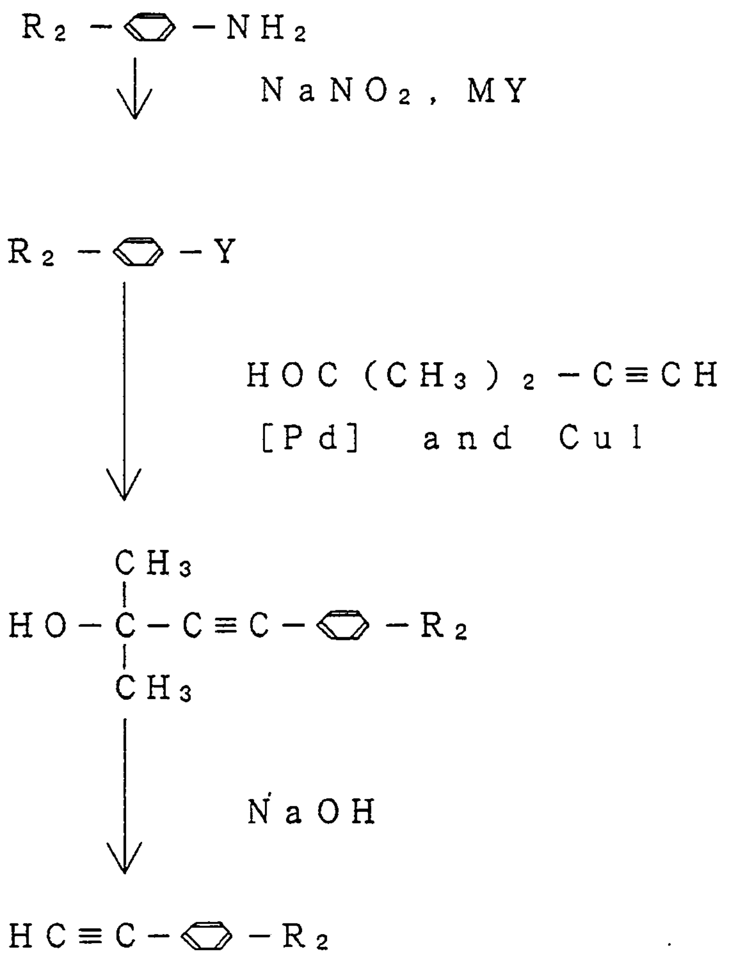


(45)

(9c)

(46)

(6c)

Where as suggested in the above the compound represented by (45) upon completion of diazotation is allowed to react with alkaline metal halide, the compound represented by (9c) as the raw material for the compound proposed in the present invention is yielded. Where the compound represented by (9c) and 3-methyl-1-butyne-3-ol are allowed to react within triethylamine in the presence of palladium catalyst of valency 0 or 2 under inert gas atmosphere, the compound represented by (46) is yielded. Where sodium hydroxide in powder form is allowed to act upon the last mentioned compound within dry toluene, the compound represented by (6c) as the raw material for the compound proposed in the present invention is yielded.

(d) Where $X_2$ is -COO-, and $A_3$ is — 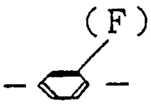 — :

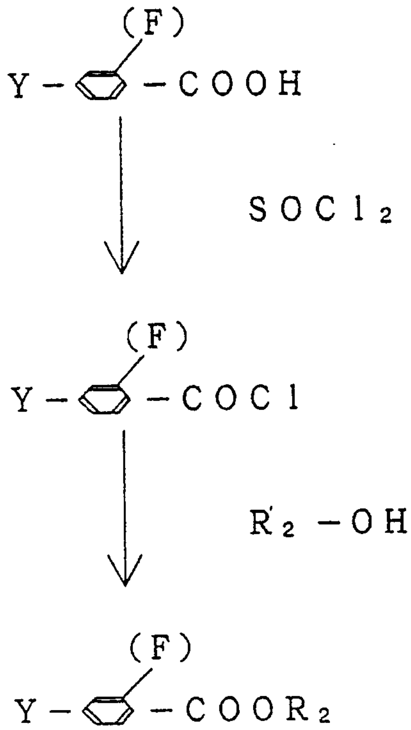


(47)

(48)

(9d)

Where as suggested in the above the compound of a acid chloride represented by (48) modified by allowing thionyl chloride to act upon the compound represented by (47) is allowed to react with alcohol in the presence of base, the compound represented by (9d) as the raw material proposed in the present invention is yielded.

[2] Where n is 0; Y is -C≡C-;

$A_1$ is — 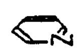 — :

and $X_1$ is -O-or -C≡C :

(1) Where $X_1$ is -O-, Y is — C ≡ C — :

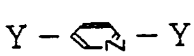 (15)

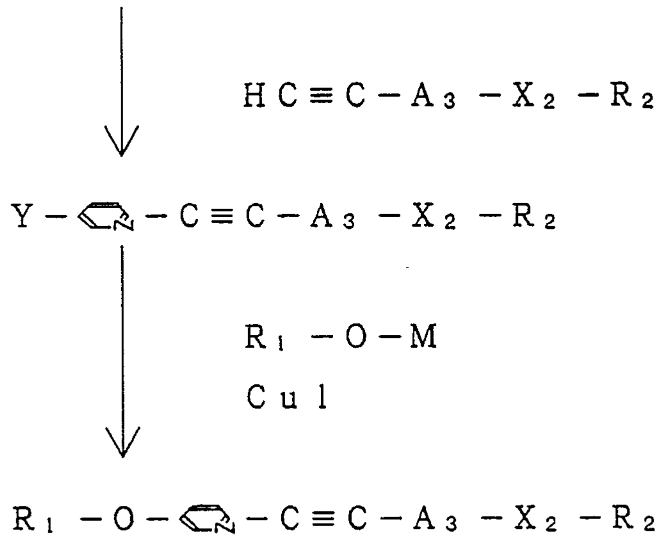


(6)

(49)

(1b)

Where as suggested in the above the compounds represented by (15) and (6) are allowed to react within triethylamine in the presence of palladium catalyst of valency 0 or 2 under inert gas atmosphere, the compound represented by (49) is yielded.

Where the last mentioned compound and alkali metal salt of alcohol are allowed to react within dry dimethylformamide or dry dimethylsulfoxide in the presence of cuprous iodide, the compound represented by the formula (1b) as the compound proposed in the present invention is yielded.

(2) Where both $X_1$ and Y are -C≡C-:

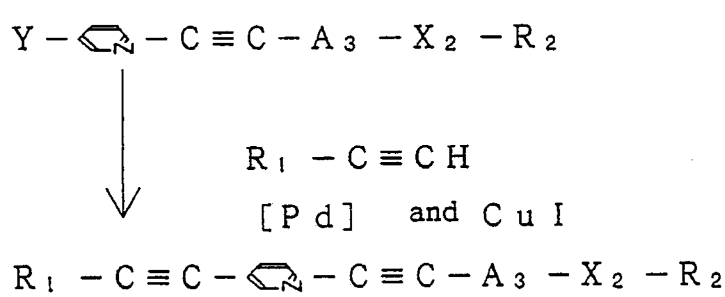


(49)

(20)

(1c)

Where as suggested in the above the compound represented by (49) and 1-alkyne represented by (20) are allowed to react within triethylamine in the presence of palladium catalyst of valency 0 or 2 under inert gas atmosphere, the compound represented by the formula (1c) as the compound proposed in the present invention is yielded.

[3] Where n is 1; Y is $-C\equiv C-$; $A_1$ is $-$ 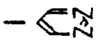 $-$ ; and $A_2$ is $-$ 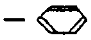 $-$ :

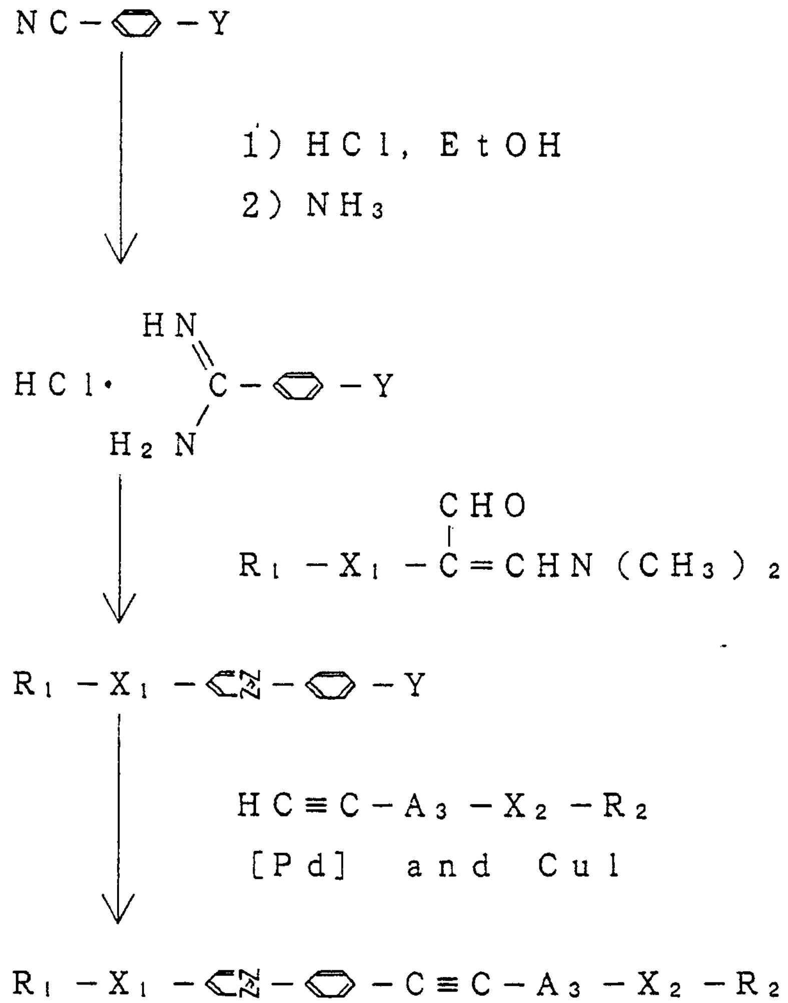


(50) (51) (31) (52) (6) (1d)

[1] Where n is 1 and Y is -C≡C-:

$$R_1-X_1-A_3-Y \quad (Y ;\ \text{halogen}) \qquad (53)$$

$$HC\equiv C-A_1-A_2-X_2-R_2 \qquad (54)$$

[Pd] and CuI

$$R_1-X_1-A_3-C\equiv C-A_1-A_2-X_2-R_2 \qquad (2a)$$

Where as suggested in the above the compounds (53) and (54) are allowed to react within triethylamine in the presence of palladium catalyst of valency 0 or 2 under inert gas atmosphere, the compound represented by (2a) as the compound proposed in the present invention is thus yielded.

Alternatively:

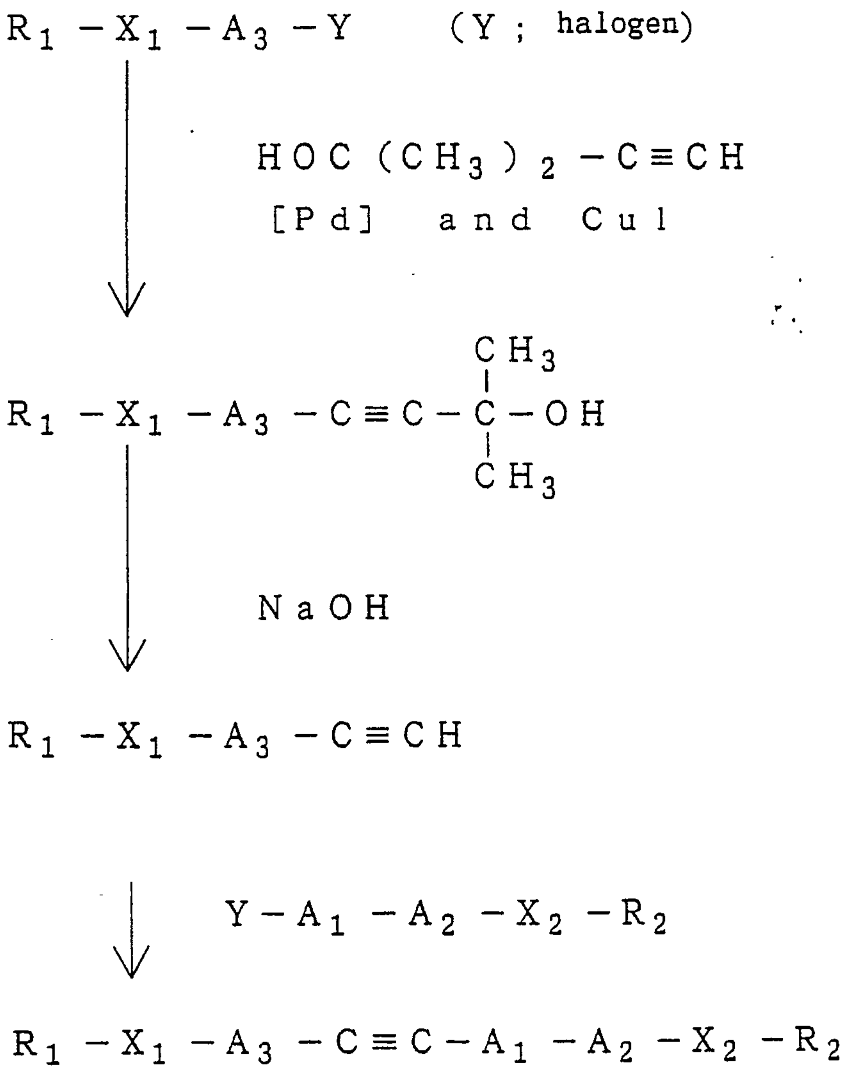


(53)

(55)

(56)

(57)

(2a)

Where as suggested in the above the compound (55) which was derived from the reaction of the compound (53) with 3-methyl-1-butyne-3-ol within triethylamine in the presence of palladium catalyst of valency 0 or 2 under inert gas atmosphere is allowed to react with sodium hydroxide in powder form within dry toluene, the compound represented by (56) is yielded.

Where the compounds (56) and (57) are allowed to react within triethylamine in the presence of palladium catalyst of valency 0 or 2 under inert gas atmosphere, the compound represented by (2a) as the compound proposed in the present invention is yielded. Also compounds (53) and (56) are obtained in the same manner as are for the compounds (6a) and (9a).

Those compounds (54) and (57) are synthesized through such process e.g. as introduced below.

(a) Where both $A_1$ and $A_2$ are

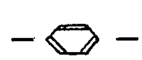

and $X_2$ is -O-:

Same process as is useful for those compounds (6a) and (9a) and for the case where $A_3$ is

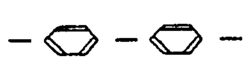

is made in use.

(b)Where $A_1$ is

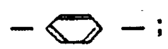 ; $A_2$ is 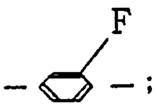

and $X_2$ is -O-:

Same process as is useful for those compounds (6b) and (9b) is made in use.

(c) Where $A_1$ is 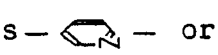 or 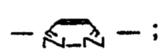 ; $A_2$ is 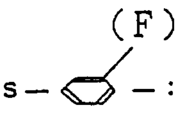

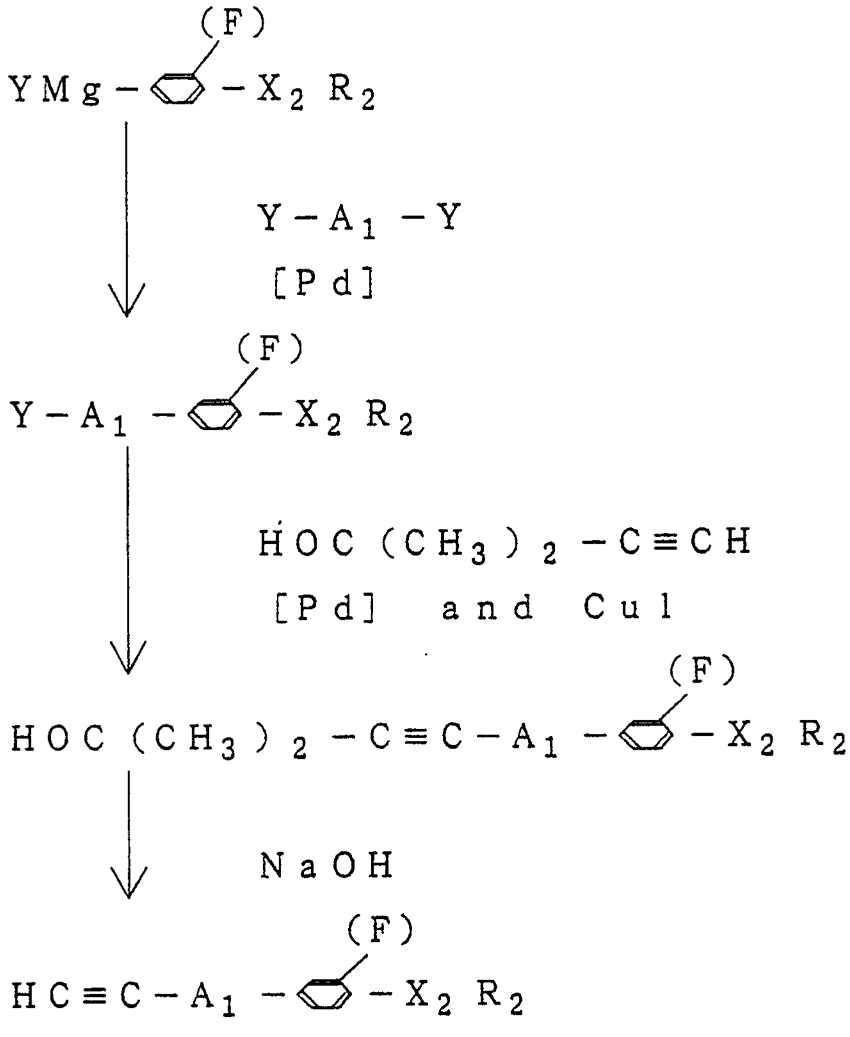


(58)

(59)

(57c)

(60)

(54c)

Where as suggested in the above the compounds (58) and (59) are allowed to react in the presence of palladium catalyst of valency 0 or 2 under inert gas atmosphere, the compound represented by (57c) as the raw material for the compound proposed in the present invention is yielded. Where the compound (60) which is derived through the reaction of the compound (57c) with 3-methyl-1-butyne-3-ol within triethylamine in the presence of palladium catalyst of valency 0 or 2 under inert gas atmosphere is allowed to react with sodium hydroxide in powder form within dry toluene, the compound represented by (54c) as the raw material for the compound proposed in the present invention is yielded.

[2] Where n is 1 and Y is $-CH_2CH_2-$:

$$R_1-X_1-A_3-C\equiv C-A_1-A_2-X_2-R_2 \qquad (2a)$$

$H_2$, Pd/C

$$R_1-X_1-A_3-CH_2CH_2-A_1-A_2-X_2-R_2 \qquad (2b)$$

Where as directed by the equations the compound (2a) is subjected to hydrogenation under hydrogen atmosphere using palladium carbon, the compound represented by the formula (2b) as the compound proposed in the present invention is yielded.

[3] Where n is 0; $A_1$ is—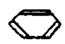—, —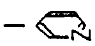— or —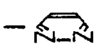—, and $X_1$ is $-C\equiv C-$ :

$$Y-A_1-A_2-X_2-R_2 \qquad (57)$$

$$R_1-C\equiv CH \qquad (20)$$

[Pd] and CuI

$$R_1-C\equiv C-A_1-A_2-X_2-R_2 \qquad (2c)$$

Where as directed by the equations the compounds (57) and (20) are allowed to react within triethylamine in the presence of palladium catalyst of valency 0 or 2 under inert gas atmosphere, the compound represented by (2c) as the compound proposed in the present invention is yielded.

[4] Where n is 0; $A_1$ is 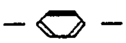, 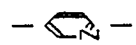 or 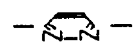 and $X_1$ is direct single bond:

$$Y-A_1-A_2-X_2-R_2$$ (57)

$R''-C\equiv CH$ (11)

[Pd] and CuI

$R''-CH_2CH_2-;\ R_1-$

$$R''-C\equiv C-A_1-A_2-X_2-R_2$$ (2'c)

$H_2$, Pd/C

$$R_1-A_1-A_2-X_2-R_2$$ (2d)

Where as directed by the above equations, the compound represented by (2'c) which was derived through reaction between those compounds (57) and (11) within triethylamine in the presence of palladium catalyst of valency 0 or 2 under inert gas atmosphere is subjected to hydrogenation under hydrogen atmosphere using palladium carbon, the compound represented by (2d) as the compound proposed in the present invention is yielded.

[5] Where n is 0; $X_1$ is direct single bond; $A_1$ is 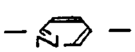, and $A_2$ is 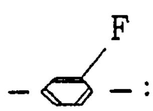:

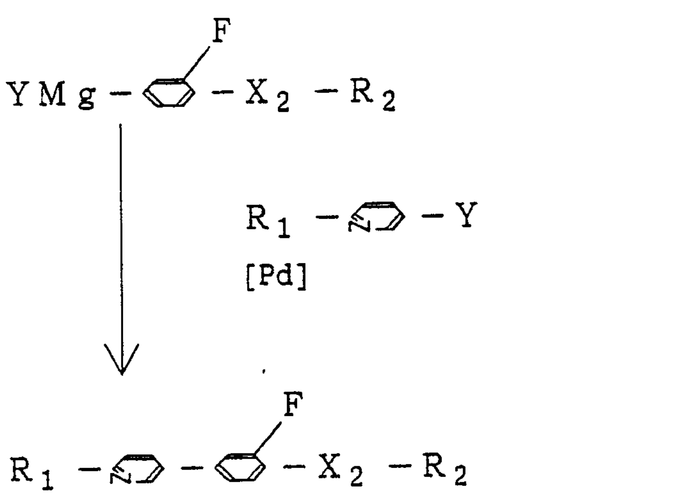


(58') (5a) (2e)

Where as suggested in the above those compounds (58') and (5a) are allowed to react in the presence of palladium catalyst under inert gas atmosphere, the compound represented by (2e) as the compound

proposed in the present invention is yielded.

[6] Where n is 0; $X_1$ is -O-; $A_1$ is -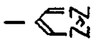-, $A_2$ is -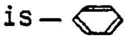-, and $X_2$ is $-C\equiv C-$:

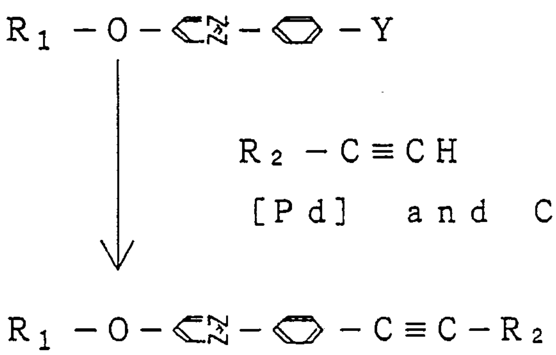


(52')

(20')

(25)

Where as directed by the above equations, the compounds (52') and (20') are allowed to react within triethylamine in the presence of palladium catalyst of valency 0 or 2 under inert gas atmosphere, the compound represented by (2f) as the compound proposed in the present invention is yielded.

The compound which is represented by the formula (3), may be obtained through a process e.g. as introduced below (Symbols $R_3$ ,$X_1$ ,$A_4$ ,$A_3$ ,$X_2$ , and $R_4$ are referred to the formula (3)).

[1] Where Y is -C≡C-:

$R_3-X_1-A_4-Y$ (Y ; halogen ) (61)

$HC\equiv C-A_3-X_2-R_4$ (62)

[Pd] and CuI

$R_3-X_1-A_4-C\equiv C-A_3-X_2-R_4$ (3a)

Where as directed by the above equations, the compounds (61) and (62) are allowed to react within triethylamine in the presence of palladium catalyst of valency 0 or 2 under inert gas atmosphere, the compound represented by (3a) as the compound proposed in the present invention is yielded.

Alternatively:

$R_3-X_1-A_4-Y$ (61)

$HOC(CH_3)_2-C\equiv CH$

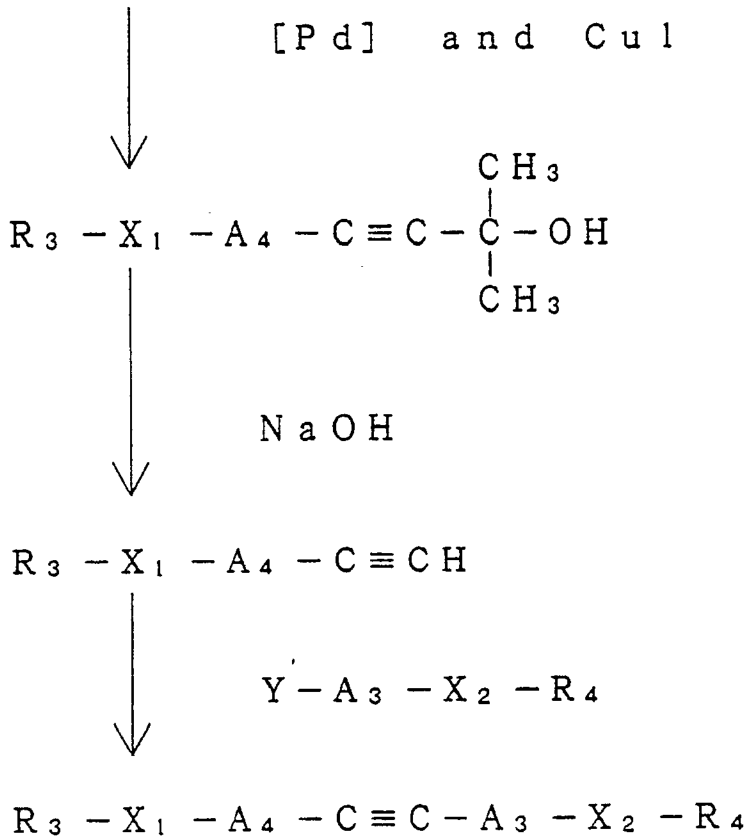


(63)

(64)

(65)

(3a)

Where as directed by the above equation, the compound represented by (63) which was derived through reaction between the compound derived via (61) and 3-methyl-1-butyne-3-ol within triethylamine in the presence of palladium catalyst of valency 0 or 2 under inert gas atmosphere is allowed to react with sodium hydroxide in powder form within dry toluene, the compound represented by the formula (64) is yielded. Where those compounds (64) and (65) are allowed to react within triethylamine in the presence of palladium catalyst of valency 0 or 2 under inert gas atmospher, the compound represented by (3a) as the compound proposed in the present invention is yielded. Also those compounds represented by (61) and (64) is obtained through a process as introduced below.

(a) Where $A_4$ is

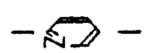 :

Same process as was used for the compounds represented by (5a), (5b), (8a) and (8b) is made in use here.

(b) Where $A_4$ is

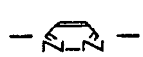 :

Same process as was used for the compounds represented by (5d), (5e), (8d) and (8e) may be made in use here.

Also those compounds represented by (62) and (65) is synthesized through a process as introduced below.

(a) Where $X_2$ is -O- and $A_3$ is

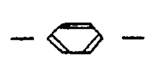 :

Same process as was used for the compounds represented by (6a) and (9a) is made in use here.
(b) Where $X_2$ is -COO- and $A_3$ is

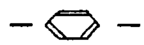 :

Same process as was used for the compound represented by (9d) is made in use here.
[2] Where Y is $-CH_2CH_2-$:

$$R_3-X_1-A_4-C\equiv C-A_3-X_2-R_4 \quad (3a)$$

$H_2$, Pd/C

$$R_3-X_1-A_4-CH_2CH_2-A_3-X_2-R_4 \quad (3b)$$

As directed by the above equations the compound represented by (3b) as the compound proposed in the present invention is yielded through hydrognation under hydrogen atmosphere using palladium carbon.

The compound represented by the formula (4) is synthesized through a process e.g. as introduced below (symbols $R_3$, $X_1$, $A_5$, $A_4$, $A_1$, $A_2$, $X_2$, and $R_4$ are referred to the formula(4)).

[1] Where Y is -C≡C-:

$$R_3-X_1-A_5-Y \quad (Y\ ;\ \text{halogen}) \quad (66)$$

$$HC\equiv C-A_1-A_2-X_2-R_4 \quad (67)$$

[Pd] and CuI

$$R_3-X_1-A_5-C\equiv C-A_1-A_2-X_2-R_4 \quad (4a)$$

Where as directed by the above equations the compounds (66) and (67) are allowed to react within triethylamine in the presence of palladium catalyst of valency 0 or 2 under inert gas atmosphere, the compound represented by (4a) as the compound proposed in the present invention is yielded.

Alternatively:

$$R_3-X_1-A_5-Y \quad (66)$$

$$HOC(CH_3)_2-C\equiv CH$$

[Pd] and CuI

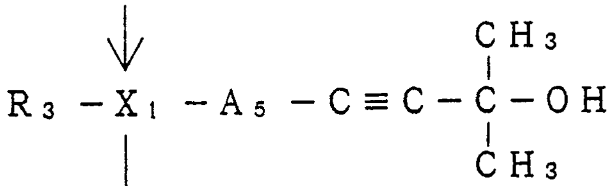

(68)

NaOH

$$R_3-X_1-A_5-C\equiv CH \quad (69)$$

$$Y-A_1-A_2-X_2-R_4 \quad (70)$$

$$R_3-X_1-A_5'-C\equiv C-A_1-A_2-X_2-R_4 \quad (4a)$$

Where as directed by the above equations, the compound represented by (68) which was derived through reaction of the compound of (66) and 3-methyl-1-butyne-3-ol within triethylamine in the presence of palladium catalyst of valency 0 or 2 under inert gas atomosphere is allowed to react with sodium hydroxide in powder form within dry toluene, the compound represented by (69) is yielded. Where the compounds represented by (69) and (70) are allowed to react within triethylamine in the presence of palladium catalyst of valency 0 or 2 under inert gas atmosphere, the compound represented by (4a) as the compound proposed in the present invention is yielded.

The compounds (66) and (69) is obtainable also through the same process as was used for the compouds represented by (6a), (9a) and (9d).

The compounds (67) and (70) is obtainable also through the same process as was used to obtain the compounds represented by (6b), (9b), (54c) and (57c).

[2] Where Y is $-CH_2CH_2-$

$$R_3-X_1-A_5-C\equiv C-A_1-A_2-X_2-R_4 \quad (4a)$$

$H_2$, Pd/C

$$R_3-X_1-A_5-CH_2CH_2-A_1-A_2-X_2-R_4 \quad (4b)$$

As is suggested in the above the compound represented by (4b) is obtainable through hydrogenation of compound (4a) under hydrogen atmosphere using palladium carbon.

Those optically active parts represented by $R_3$ and $R_4$ under formulas (3) and (4) may be derived from the respective optically active alcohol or optically active carboxylic acid.

Some of the last mentioned compounds available in the market; those may be obtainable through asymmetric reduction of the corresponding ketone with the aid of enzyme, microorganism or asymmetric metal catalyst; or may be derived from optically active aminoacid, optically active hydroxy acid and optically active hydroxy acid ester existing as natural products. In addition, optically active 2-fluoroalkylalcohol and optically active 2-chloroalkylalcohol are obtainable utilizing optically active epoxy compounds as initial material. Optically active 1- trifluoromethylalcohol are obtainable through asymmetric hydrolysis of the

corresponding ester with the aid of enzyme.

In general liquid crystall is provided as liquid crystalline composition comprising more than 2 components and the liquid crystal compound proposed in the present invention are made in use as a component of liquid crystal composition. The liquid crystal composition may comprise smectic liquid crystals, i.e. smectic liquid crystals which do not contain the optically active part [for examples, 2-p-alkyloxyphenyl-5-alkylpyrimidine, 2-p-alkylphenyl-5-alkyloxypyrimidine, 2-p-alkanoyloxyphenyl-5-alkylpyrimidine, 2-p-alkyloxycarbonylphenyl-5-alkylpyrimidine, 2-p-alkylphenyl-5-p-alkyloxyphenylpyrimidine, 2-p-aklyloxy-m-fluorophenyl-5-alkylpyrimidine, 2-p-alkyloxyphenyi-5-alkylpyridine, 2-p-alkyloxyphenyl-5-(trans-4-alkylcyclohexyl) pyrimidine, 2-p-alkyloxy-m-fluorophenyl-5-alkylpyridine, 2-p-(p′-alkylphenyl) phenyl-5-alkylpyrimidine, 2-p-alkylphenyl-5-p-alkylphenylpyrimidine, p-alkyloxyphenyl-5-alkylpicolinate, 2-p-alkyloxyphenyl-5-alkyloxypyrazine, 2-p-alkylphenyl-5-alkylpyrimidine, 2-p-alkyloxyphenyl-5-alkyloxypyrimidine, 2-p-alkylphenyl-5-alkyloxypyridine, 4-alkyloxy-4′-biphenylcarboxylic acid-p′-(alkyloxycarbonyl) phenylester, 4-alkyloxy-4′-biphenylcarboxylic acid-alkylester or otherwise] and / or ferroelectric liquid crystal [for examples, optically active 4-alkyloxy-4′-biphenylcarboxylic acid-p′-(2-methylbutyloxycarbonyl) phenylester, optically active 4-n-alkyloxy-4′-biphenylcarboxylic acid-2-methylbutylester, optically active p-alkyloxybenzyriden-p′-amino-2-chrolopropylcinnamate, optically active p-alkyloxybenzyriden-p′-amino-2-methylbutylcinnamate or otherwise] and / or ordinary chiral smectic liquid crystal [for examples, optically active 4-(p-alkyloxybiphenyl-p′-oxycabonyl)-4′- (2-methylbutyloxycarbonyl) cyclohexane, optically active p-n- alkyloxybenzyriden-p′-(2-methylbutyloxycarbonyl) aniline or otherwise].

Liquid crystal compositions may contain other components, for example, chiral compounds showing no liquid crystaline properties, pleochroic dyes (such as anthraqiunone dyes, azo dyes and the like) so on.

By applying voltage, the ferroelectric liquid crystal composition is featured with photo-switching phenomena.

This feature provides a display device with a faster response time. [refer to Japanese Patent Laid Open Publications Nos. 56-107216 and 59-118744; N.A.Clark, S.T.Lagerwall; Applied Physics Letter, 36, 899 (1980) and otherwise].

The liquid crystal composition proposed in the present invention may be utilized as photo-switching device (display device), where it is sealed in a evacuated ,liquid cryatal cell with a gap of 0.5 to 10$\mu$m, more preferably 0.5 to 3$\mu$m and provided with both end polarizer.

For the purpose of producing said liquid crystal cell, transparent electrodes and 2 sheets of glass substrates which are purposely made oriented and assembled with spacers therebetween are advantageously made in use. As spacers, for example, alumina beads, glass fibers and polyimide films are used. Referring to the orientation process, ordinary processes, e.g. rubbing processing, of polymer film such as polyimide film etc. and oblique vaper-deposition of SiO.

The present invention makes it possible to provide a novel smectic C liquid crystal, smectic liquid crystal useful as components of smectic C liquid crystal composition and optically active compound useful as components which are made of such components are specially featured with the following advantages.

(1) Negative anisotropy of dielectric constant is generally considered desirous with ferroelectric liquid crystal composition. Those smectic liquid crystal components under the present invention, in which have halogen atoms tending to attract electrons in the direction ofshorter axis of molecule and/or contain nitrogen atoms, show negative anisotropy of dielectric constant. For this reason such component is significantly useful as the component which is capable to make the anisotropy of dielectric constant negative for smectic C liquid crystal composition.

(2) Those components with phase series Cr-Sc-$S_A$ -N-I of smectic liquid crystal under present invention are identified as the ones which are considered desirous particularly for chiral smectic C liquid crystal composition used in practice. For this reason the components with such phase series are significantly useful as the possible main components of base liquid crystal composition.

(3) Those components particularly with phase series Cr-Sc of (chiral) smectic liquid crystal in the present invention are capable to lower the bottom temperature threshold of smectic C through admixing. For this reason they are significantly useful as the components capable to magnify the temperature range of (chiral) smectic Cphase for liquid crystal composition used in practice.

4) Those components particularlyl with phase sevies Sc-N-I or Sc-I of (chiral) smectic liquid crystal in the present invention have a larger tilt-angle. For this reason they are significantly useful as the components capable to adjust the tilt-angle of liquid crystal composition used in practice.

(5) Those components particularly with higher top temperature threshold of smectic C of (chiral) smectic liquid crystal in the present invention are significantly useful as the components capable to elevate the top temperature threshold of smectic C for liquid crystal composition used in practice.

(6) Those, components particularly classified into bicyclic group and with higher top temperature

threshold of smectic C are significantly useful since they are capable to elevate the top temperature threshold without causing viscosity of liquid crystal composition to be enhanced.

(7) Those components particularly with acetylene bond within skeleton of (chiral) smectic liquid crystal in the present invention are significantly useful because of their good orientation on account of rigid skeleton.

(8) Those components particularly with naphthalene ring have a rigid skeleton, For this reason they are significantly useful as the components capable to improve the orientation of liquid crystal composition used in practice by admixing, even though does not show smectic C.

(9) Those component of the present invention are featured with a lower viscosity than is in (chiral) smectic C liquid crystal of phenylbenzoate series.

(10) Those components particularly with acetylene bond within skeleton of smectic liquid crystal in the present invention are found featured with a higher birefringence. For this reason they are useful not only in smectic C but also nematic liquid crystal composition, so that display cell may be reduced in thickness and have a fast response time.

(11) Optically active compound in the present invention is featured with a larger dipole moment. Accordingly those liquid crystalline compounds or compositions of smectic C or H phase which contain such optically active compound are capable to provide a phase with lorger spontaneous polarization value, i.e. a fast accelerated optical response.

(12) Optically active compound in the present invention is featured with a linear structure of molecule. Accordingly those liquid crystalline compounds or compositions which contain such optically active compound are capable to have an adverse effect on their phase series and phase transition temperature reduced to the minimum level.

(13) In applying optically active compounds in liquid crystalline compounds or compositions it is generally known that configuration of these optically active compounds shall be limited to either R- or S-form depending on positive or negative sign of spontaneous polarization of the base ferroelectric liquid crystal compounds or ferroelectric liquid crystal compositions, those optically active compounds proposed in the present invention may be commonly available regardless of R- or S- form and are capable to significantly enhance the spontaneous polarization value.

(14) In addition to the applicability in the smectic compositions with a ferroelectric or non-chiral smectic ones as aforesaid the optically active compounds proposed in the present invention, when applied in nematic compositions, are capable to suppress possible occurence of reverse domain in liquid crystall cells of TN-type.

(15) Those composition of the present invention have a good stability against possible effect of light, heat and moisture.

The liquid crystal compounds and optically active compounds proposed in the present invention are found significantly useful for developing a practical ferroelectric smectic liduid crystal compositions.

## Embodiments

The invention is described in detail with reference to the different examples discussed hereunder but the following claims are not to be considered as solely limited thereto.

In the following examples, % represents by weight %, and the structure of the compunds was confirmed with analysis of NMR (nuclear magnetic resonance), MS (mass spectrum), IR (infrared ray adsorption) and elemental analysis.

Following symbols used in the tables under the following examples denote the respective phase enumerated hereunder;

Cry: Crystal phase

$S_1$ : Unidentified smectic phase

$S_A$ : Smectic A phase

$S_C$ : Smectic C phase

N : Nematic phase

I : Isotropic phase

## Example 1

Preparation of compound No. 1a-21 in the Table 1a

1) 5.0 g of 2-bromo-5-nitropyridine and 3.5g of 1-decyne were allowed to react overnight within 50 ml of triethylamine in the presence of 204 mg of bis-triphenylphosphinepalladiumdichloride and 51 mg of cuprous iodide as catalysts under nitrogen atmosphere at room temperature. Upon completion of reaction, the solution caused triethylamine to be removed for subsequent hexane extraction. The hexane layer produced thereby was washed with hydrochloric acid solution in water, then with water for subsequent treatment through a silicagel-column. Through a recrystallization using methanol, 3.0 g of compound (a) with a structure represented by the following formula was finally yielded.

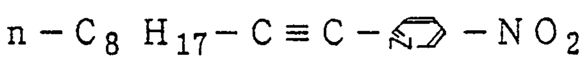

(a)

2) 30 ml of ethanol containing 3.0 g of compound yielded under 1) were added with 0.5 g of 5% Pd-C-(5% palladium-carbon) as catalyst and subjected to hydrogenation and reduction under hydrogen atmosphere of atmospheric pressure at room temperature with the solution kept stirred. Upon confirmation that hydrogen became found no longer absorbed, the solution caused catalyst to be removed using filtration and then ethanol to be removed by evaporation, whereupon 2.6 g of oily 2-n-decyl-5-aminopyridine were finally yielded.

3) 100 ml of water containing 11 g of 36% hydrochloric acid were added with 2.6 g of 2-n-decyl-5-aminopyridine and then cooled down to below 5 °C Sodium nitrite solution in water separately prepared (sodium nitrite 0.72 g, water 5 ml) was allowed to drop thereon under the temperature below 5°C. Upon completion of dropping process, the solution was kept stirred further for 1 hr., then added with potassium iodide solution in water (potassium iodide 8.6 g, water 10 ml) with the tempereture restored slowly to room temperature, the solution being kept stirred until the evolution of nitrogen was completed. Upon completion of reaction, the solution was made to turn alkaline through adding sodium hydroxide for subsequent hexane extraction. The hexane layer produced thereby was washed with water, then with sodium hydrogen sulfite solution in water and again with water. It was then subjected to a treatment using a silicagel-column and 2.4 g of oily 2-n-decyl-5-iodopyridine was thus yielded.

4) 1.4 l of water containing 210 g of sodium bicarbonate therein were added with 187 g of o-fluorophenol and cooled down to below 10°C. 423 g of iodine of fine grain were added thereto in five aliquot parts. Upon confirmation that iodine color has faded away, the solution was subjected to toluene extraction. The toluene layer produced thereby was washed with water, then with sodium hydrogen sulfite solution in water and again with water; and caused toluene to be removed. The reddish purpule colored solution yielded from distillation under reduced pressure (b.p, 124 to 145 °C/18 mmHg) was made recrystallized using hexane, whereupon 120 g of o-fluoro-p-iodophenol in white crystal were finally yielded.

5) 120 ml of dimethylsulfoxide containing 15.0 g of o-fluoro-p-iodophenol were added with sodium hydroxide solution in water (sodium hydroxide 3.0 g and water 10 ml) and stirred into homogeneous state. It was added with 10.4 g of n-hexylbromide and kept stirred for 3 days. The reactant was poured into 1ℓ of ice water and it was extracted thrice using hexane. The hexane layer produced thereby was washed with water and caused hexane to be removed. 16.7 g of oily p-n-hexyloxy-m-fluoroiodobenzene was thus yielded.

6) 14.0 g of p-n-hexyloxy-m-fluoroiodobenzene and 3-methyl-1-butyne-3-ol were allowed to react overnight within 80 ml of triethylamine in the presence of 272 mg of bis- triphenylphosphinepalladiumdichloride and 68 mg of cuprous iodide as catalysts under nitrogen atmosphere at room temperature. Upon completion of reaction, the solution caused triethylamine to be removed for subsequent hexane extraction. The hexane layer produced thereby was washed with 1 N hydrochloric acid solution in water, then with water. Through hexane removal, 12.5 g of compound (b) with the structure represented by the following formula.

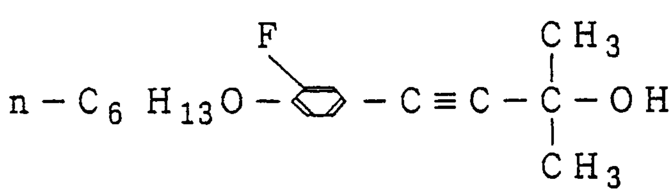

(b)

7) 400 ml of dry toluene containing 12.5 g of compound (b) yielded under 6) were added with 5.4 g of sodium hydroxide in powder form and subjected to reflux for 1 hr. Upon completion of cooling and water washing, the solution caused toluene to be removed. The black colored oil yielded was subjected to a methanol extraction and 7.55 g of oily p-n-hexyloxy-m-fluorophenylacetylene was thus obtained.

8) 1.30 g of 2-n-decyl-5-aminopyridine and 0.83 g of p-n-hexyloxy-m-fluorophenylacetylene yielded under 7) were allowed to react overnight within 30 ml of triethylamine in the presence of 32 mg of bistriphenylhosphinepalladiumdichloride and 8 mg of cuprous iodide as catalysts under nitrogen atmosphere at room temperature. Upon completion of reaction, the solution caused triethylamine to be removed for subsequent hexane extraction. The hexane layer produced thereby was washed with hydrochloric acid solution in water, then with water and subjected to a treatment through a silicagel-column. Through recrystallization repeated twice using ethanol, 0.35 g of white crystalline compound No. 1a-21 in the Table 1a proposed in the present invention was thus yielded. IR, H-NMR, and F-NMR spectra of said compound are illustrated in Figs. 1, -2, and -3 espectively.

Results from elemental analysis:

| Calculated values of components(%) | Observed values of components(%) |
|---|---|
| C: 79.64<br>H: 9.15<br>N: 3.20<br>F: 4.35 | C: 79.43<br>H: 9.27<br>N: 3.35<br>F: 4.22 |

Example 2 ~ 17

Same reaction as described of the Example 1 took place, and the structure represented by following formura were thus yielded.

Example No. — compound — compound No. in the Table 1a

2 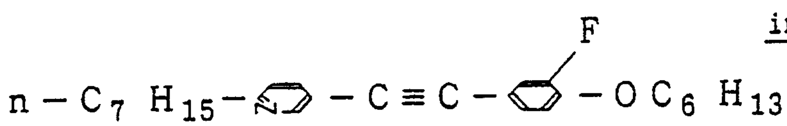 1a-6

3 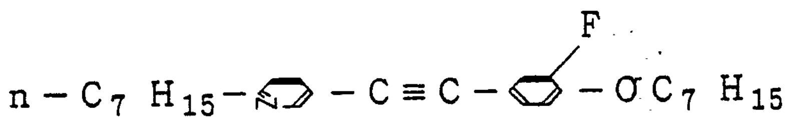 1a-7

4 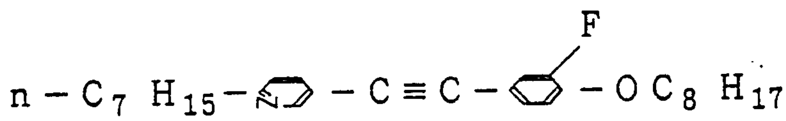 1a-8

5 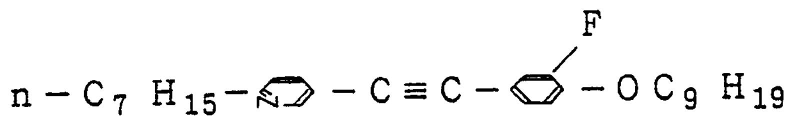 1a-9

6 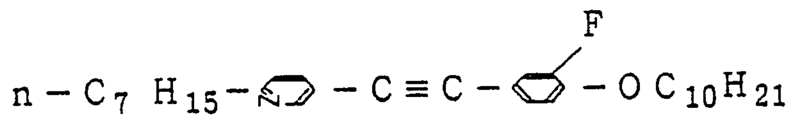 1a-10

Example 18

Preparation of compound No. 1a-40 in the Table 1a

1) 250 ml of dimethylsulfoxide containg p-iodophenol 25.0 g therein were added with sodium hydroxide solution in water (sodium hydroxide 5.0 g, water 20 ml) and stirred into homogeneous state. It was then added with 17.8 g of n-hexylbromide and kept stirred for 3 days at room temperature. The reactant was poured into ice water for subsequent hexane extraction to be repeated thrice. The hexane layer produced thereby was washed with water and caused hexane to be removed, 29.5 g of oily p-n-hexyloxyiodobenzene was thus yielded.

2) 29.5 g of p-n-hexyloxyiodobenzene and 12.4 g of 3-methyl-1-butyne-3-ol were allowed to react overnight within 150 ml of triethylamine in the presence of 300 mg of bis-triphenylphosphinepalladiumdichloride and 75 mg of cuprous iodide as catalysts under nitrogen atmosphere at room temperature. Upon completion of reaction, the solution caused triethylamine to be removed for subsequent hexane extraction. The hexane layer produced thereby was washed with 1 N hydrochloric acid solution in water, then with water and caused hexane to be removed, whereupon 23.2 g of solid compound (c) with structure represented by the follwing formula.

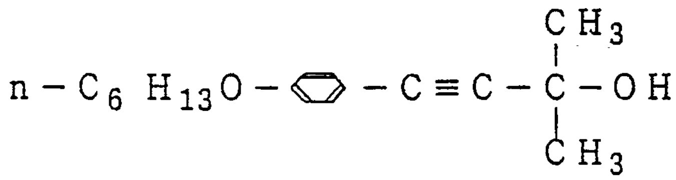

(c)

3) 800 ml of dry toluene containing 23.2 g of compound (c) yielded under 2) were added with 10.7 g of sodium hydroxide in powder form and subjected to reflux for 1 hr. The solution was upon cooled and washed with water. The toluene was removed by evaporation and subjected to methanol extraction. It caused methanol to be removed to finally yield 12.8 g of oily p-n-hexyloxyphenylacetylene.

4) 0.65 g of p-n-hexyloxyphenylacetylene yielded under 3) and 1.10 g of 2-n-decyl-5-iodopyridine yielded under 3) of Example 1 were allowed to react overnight within 30 ml of triethylamine in the presence of 28 mg of bis-triphenylphosphinepalladiumdichloride and 7 mg of cuprous iodide as catalysts under nitrogen atmosphere at room temperature. Upon completion of reactive process the solution caused triethylamine to be removed for subsequent hexane extraction. The hexane layer produced thereby was washed with hydrochloric acid solution in water, then with water for subsequent treatment through a silicagel-column. Recrystallization repeated twice using ethanol yielded 0.23 g of white crystalline compound No. 1a-40 under the Table 1a proposed in the present invention. IR and H-NMR spectra of said compound are illustrated in Figs. 4 and 5 respectively.

Result from elemental analysis:

| Calculated values of components(%) | Observed values of components(%) |
|---|---|
| C: 83.05<br>H: 9.79<br>N: 3.34 | C: 82.93<br>H: 9.88<br>N: 3.51 |

Example 19

Preparation of compound NO. 1a-51 in the Table 1a

1) Onto 250 ml of dry dimethylformamide containing 3.8 g of sodium hydride (60% content) therein 15.5 g of n-decylalcohol were allowed to drop under 70 to 80 °C at such a rate that hyrogen evolved not so vigorously. Upon confirmation that the evolution of hydrogen was completed, the solution caused its temperature to be restored down to room temperature and was added with 15.5 g of 2-chloro-5-nitropyridine and subjected to reflux for 2 hrs. Upon completion of reaction, the solution was poured into

ice water and the precipitate produced thereby was subjected to filtration followed by drying. Of the solid matter resulted such part being soluble to hexane was subjected to recrystallization using methanol to yield finally 14.4 g of 2-n-decyloxy-5-nitropyridine.

2) 150 ml of ethanol containing 14.4 g of 2-n-decyloxy-5-nitropyridine yielded under 1) were added with 1.0 g of 5% palladium-carbon for subsequent reduction under hydrogen atmosphere of atmospheric pressure. Upon completion of reaction, the solution caused catalyst to be removed. 12.1 g of 2-n-decyloxy-5-aminopyridine was thus yielded in fluid form.

3) 450 ml of water containing 50 g of 36% hydrochloric acid therein were added with 12.1 g of 2-n-decyloxy-5-aminopyridine so as to be cooled to a temperature below 5 °C. Sodium nitrite solution in water separately prepared (sodium nitrite 3.3 g, water 20 ml) was allowed to drop thereon under a temperature below 5 °C . Upon completion of dropping process, the solution was kept stirred for 1 hr. and added with potassium iodide solution in water (potassium iodide 40 g, water 40 ml) with the temperature restored slowly to room temperature, the solution being kept stirred until the evolution of nitrogen was completed. Upon completion of reaction, 9.4 g of oily 2-n- decyloxy-5-iodopyridine was yielded through hexane extraction, washing the hexane layer with water, then with sodium hydrogen sulfite solution in water and again with water followed by treatment through a silicagel-column.

4) 1.03 g of 2-n-decyloxy-5-iodopyridine yielded under 3) and 0.63 g of p-n-hexyloxy-m-fluorophenylacetylene yielded under 7) of Example 1 were allowed to react overnight within 20 ml of triethylamine in the presence of 24 mg of bis-triphenylphosphinepalladiumdichloride and 6 mg of cuprous iodide as catalysts under nitrogen atmosphere at room temperature. Upon completion of reaction, the solution caused triethylamine to be removed for subsequent extraction by hexane. The hexane layer produced thereby was washed with hydrochloric acid solution in water, then with water for subsequent treatment through a silicagelcolumn. Recrystallization was repeated twice using ethanol. 0.32 g of white crystalline compound No. 1a-51 under the Table 1a proposed the present invention was thus yielded. IR, H-NMR, and F-NMR spectra of said compound are illustrated in Figs. 6, -7 and -8 respectively.

Result from elemental analysis:

| Calculated values of components(%) | Observed values of components(%) |
|---|---|
| C: 76.83 | C: 76.99 |
| H: 8.83 | H: 8.76 |
| N: 3.09 | N: 3.25 |
| F: 4.19 | F: 4.08 |

Example 20

Preparation of compound NO. 1a-48 in the Table 1a

1) 1.0 g of 2-n-decyloxy-5-iodopyridine yielded under 3) of Example 19 and 0.56 g of p-n-hexyloxyphenylacetylene yielded under 3) of Example 18 were allowed to react overnight within 20 ml of triethylamine in the presence of 24 mg of bis-triphenylphosphinepalladiumdichloride and 6 mg of cuprous iodide as catalysts under nitrogen atmosphere at room temperature. The reactant so resulted caused triethylamine to be removed for subsequent hexane extraction. The hexane layer produced thereby was washed with hydrochloric acid solution in water, then with water for subsequent treatment through a silicagel-column. Through recrystallization repeated twice using ethanol, 0.41 g of white crystalline compound No. 1a-48 under the Table 1a proposed in the present invention was thus yielded. IR and H-NMR spectra of said compound are illustrated in Figs. 9 and -10 respectively.

Result from elemental analysis:

| Calculated values of components(%) | Observed values of components(%) |
|---|---|
| C: 80.00<br>H: 9.43<br>N: 3.21 | C: 80.17<br>H: 9.31<br>N: 3.31 |

Example 21

Preparation of compound No. 1a-45 in the Table 1a

1) 11.7 g of p-iodonitrobenzene and 6.5 g of 1-decyne were allowed to react overnight within 150 ml of triethylamine in the presence of 408 mg of bis-triphenylphosphinepalladiumdichloride and 102 mg of cuprous iodide as catalysts under nitrogen atmosphere at room temperature. The reactant so resulted caused triethylamine to be removed for subsequent hexane extraction. The hexane layer produced thereby was washed with 1 N hydrochloric acid solution in water, then with water for subsequent treatment through a silicagel-column. 11.2 g of oily compound (d) with structure represented by the following formula.

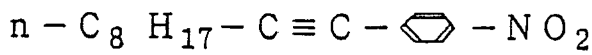

(d)

2) 150 ml of ethanol containing 11.2 g of compound yielded under 1) were added with 1.0 g of 5% Pd-C-(5% palladium carbon) as catalyst and subjected to hydrogenation and reduction under hydrogen atmosphere of atmospheric pressure at room temperature with the solution kept stirred. Upon confirmation that hydrogen was found no longer absorbed the solution caused catalyst to be removed through filtration and then ethanol to be removed by evaporation. 9.7 g of oily p-n-decylaniline was thus yielded.

3) 500 ml of water containing therein 43 g of 36% hydrochloric acid were added with 9.7 g of p-n-decylaniline and cooled down to below 5 °C. sodium nitrite solution in water (sodium nitrite 2.87 g, water 20 ml) separatery prepared was allowed to drop thereon under a temperature below 5 °C. Upon completion of dropping process, the solution was stirred for 1 hr., added with potassium iodide solution in water (potassium iodide 35 g, water 50 ml) with the temperature slowly restored to room temperature, being kept stirred until the evolution of nitrogen was completed. The reactant was subjected to hexane extraction. The hexane layer was washed with water, then with sodium hydrogen sulfite solution in water and again with water for subsequent treatment through a silicagel-column. Oily p-n-decyliodobenzene of 11.3 g was thus yielded:

4) 9.3 g of p-n-decyliodobenzene and 2.5 g of 3-methyl-1-butyne-3-ol were allowed to react overnight within 60 ml of triethylamine in the presence of 200 mg of bis-triphenylphosphinepalladiumdichloride and 50 mg of cuprous iodide as catalysts under nitrogen atmosphere at room temperature. The reactant caused triethylamine to be removed for subsequent hexane extraction. The hexane layer produced thereby was washed with 1 N hydrochloric acid solution in water, then with water. Through evaporation of hexane, 6.2 g of compound (e) in fluid form represented by the following formula was thus yielded.

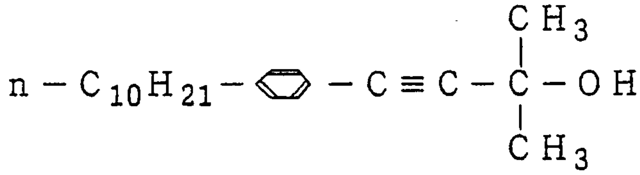

(e)

5) 150 ml of dry toluene containing therein 6.2 g of compound (e) yielded under 4) were added with 3.1 g of sodium hydroxide in powder form for subsequent reflux for 1 hr. The solution was upon cooled and washed with water. The toluene was removed by evaporation. Black colored oil as reactant was

subjected to methanol extraction. Through evaporation of methanol, 4.2 g of oily p-n-decylphenylacetylen was finally yielded.

6) On to 250 ml of dry diemethylformamide containing therein 2.0 g of sodium hydride (60% content), 5.3 g of n-hexylalcohol were allowed to drop at room temperature at such a rate that hydrogen evolution was not so vigorously. Upon confirmation that the evolution of hydrogen was completed, the solution was added with 7.9 g of 2-chloro-5-nitropyridine and then subjected to reflux for 2 hrs. The reactant was poured into ice water, precipitate produced thereby being subjected to filtration followed by drying. Of the resultant solid matter such part being soluble to hexane was subjected to a treatment through a silicagel-column. 2-n-hexyloxy-5-nitropyridine of 6.9 g was thus yielded.

7) 30 ml of ethanol containing therein 2.3 g of 2-n-hexyloxy-5-nitropyridine yielded under 6) were added with 0.5 g of 5% palladium-carbon for the subsequent reduction under hydrogen atmosphere of atmospheric pressure. The reactant caused catalyst to be removed through filtration and further ethanol to be removed, whereupon 1.9 g of 2-n-hexyloxy-5-aminopyridine in fluid form was thus yielded.

8) 80 ml of water containing therein 10 g of 36% hydrochloric acid were added with 1.9 g of 2-n-hexyloxy-5-aminopyridine so as to be cooled down to below 5°C. Sodium nitrite solution in water (sodium nitrite 0.67 g, water 5ml) separately prepared was allowed to drop thereon. Upon completion of dropping process the solution was stirred further for 1 hr., and added with potassium iodide solution in water (potassium iodide 8 g, water 10 ml) with the temperature slowly restored to room temperature, the solution being kept stirred until the evolution of nitrogen was completed. The reactant was subjected to hexane extraction. The hexane layer produced thereby was washed with water, then with sodium hydrogen sulfite solution in water and again with water for subsequent treatment through a silicagel-column. Oily 2-n-hexyloxy-5-iodopyridine of 1.3 g was thus yielded.

9) 1.4 g of p-n-decylphenylacetyrene yielded under 5) and 1.3 g 2-n-hexyloxy-5-iodopyridine yielded under 8) were allowed to react overnight within 40 ml of triethylamine in the presence of 40 mg of bistriphenylphosphinepalladiumdichloride and 10 mg of cuprous iodide as catalysts under nitrogen atmosphere at room temperature. The reactant caused triethylamine to be removed for the subsequent hexane extraction. The hexane layer produced thereby was washed with hydrochloric acid solution in water, then with water for the subsequent treatment through a silicagel-column. Through recrystallization repeated twice using ethanol, 0.67 g of white crystalline compound No. 1a-45 under the Table 1a proposed in the present invention were thus yielded. IR-and H-NMR spectra of said compound are illustrated in Figs. 11 and 12 respectively.

Result from elemental analysis:

| Calculated values of components(%) | Observed values of components(%) |
|---|---|
| C: 83.05<br>H: 9.79<br>N: 3.34 | C: 80.30<br>H: 9.61<br>N: 3.35 |

Example 22

Preparation of compound No. 1a-72 in the Table 1a

1) 25.0 g of 2,5-dibromopyridine and 17.4 g of 1-decyne were allowed to react for 6 hrs. within 250 ml of triethylamine in the presence of 400 mg of bistriphenylphosphinepalladiumdichloride and 100 mg of cuprous iodide as catalysts under nitrogen atmosphere at room temperature. Upon completion of reaction, the reactant caused triethylamine to be removed for subsequent hexane extract. Hexane layer produced thereby was washed with hydrochloric acid solution in water, and then with water for subsequent treatment through a silicagel-column. 26.6 g of liquid compound (f) represented by the following formula was thus yielded.

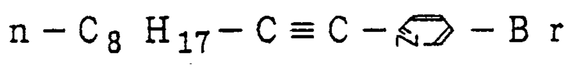 (f)

2) 2.94 g of compound (f) obtained under 1) and 2.2 g of p-n-hexyloxy-m-fluorophenylacetylene obtained under 7) of the Example 1 were allowed to react within 40 ml of triethylamine in the presence of 100 mg of bistriphenylphosphinepalladiumdichloride, 25 mg of cuprous iodide and 150 mg of triphenylphosphine as catalysts under nitrogen atmosphere of reflux temperature. Upon completion of reaction, the reactant caused triethylamine to be removed for subsequent toluene extraction. Toluene layer produced thereby was washed with hydrochloric acid solution in water, then with water for subsequent treatment through a silicagel-column. Through removal of toluene followed by recrystallization repeated thrice using ethanol, 2.33 g of white crystalline compound No. 1a-72 under Table 1a as the compund proposed in the present invention were thus yielded. IR, H-NMR and F-NMR spectra of the compound are illustrated in Figs. 13, 14 and 15 respectively.

Results from elemental analysis:

| Calculated values of components(%) | Observed values of components(%) |
|---|---|
| C: 80.37<br>H: 8.31<br>N: 3.23<br>F: 4.39 | C: 80.12<br>H: 8.46<br>N: 3.25<br>F: 4.18 |

Example 23

Preparation of compound No. 1a-99 in the Table 1a

1) 30.0 g of o-fluoro-p-iodophenol was dissolved in 300 ml of dry toluene. The solutiotn was then added with 16 ml of pyridine and stirred at room temperature, 17.6 ml of benzoylchloride were allowd to drop into the solution during 15 min. The solution was kept stirred for subsequent 24 hrs at room temperature. The reactant mixture was washed successively with water, with 1 N hydrochloric acid, with saturated solution of sodium bicarbonate in water and again with water and caused toluene to be removed. Through recrystallization of solid substance obtained using methanol, 38.2 g of compound (g) with the structure represented by the following formula was yielded.

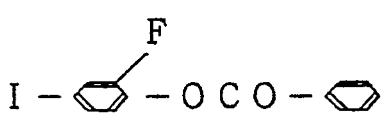 (g)

2) 12.8 g of compound (g) yielded under 1) was dissolved in 50 ml of toluene. 25 ml of 2 M sodium carbonate solution in water were added thereto for subsequent stirring at room temperature. 860 mg of tetrakistriphenylphsophinepalladium and 30 ml of ethanol solution with 5.0 g of phenylboric acid dissolved therein were further added to the solution for subsequent heating reflux for 5 hrs. The on-going reaction was, upon cooled, quenched using 3 ml of 30% hydrogen peroxide solution in water for subsequent toluene extraction. The extract, upon washed with water, caused toluene to be removed. Through recrystallization of solid matter obtained using hexane 7.0 g of the compound (h) with the structure represented by the following formula was yielded.

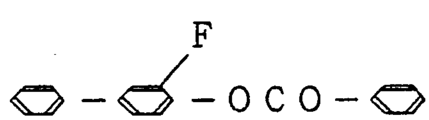 (h)

3) 5.6 g of compound (h) yielded under 2) was dissolved in 70 ml of acetic acid, 2 ml of sulfuric acid, 1.1 g of metaperiodic acid and 2.4 g of iodine were further added thereto and kept stirred for 10 hrs. at 90 °C. The reactant mexture, upon cooled, was poured into 200 ml of ice water, excess iodine to be reduced using sodium hydrogen sulfite and also precipitate developed thereby to be removed. Through subsequent recrystallization using toluene, 3.5 g of compound (i) with the structure represented by the following formula was yielded.

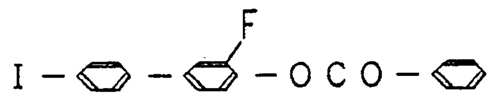 (i)

4) 3.5 g of the compound (i) yielded under 3) were made suspended in 60 ml of ethanol. The solution was then added with 1.0 g of sodium hydroxide and subjected to heating reflux for 1 hr. The reactant, upon cooled, caused ethanol to be removed. The solid matter yielded thereby was dissolved in 80 ml of water. Carbon dioxide gas was allowed to be blown therein. Through subsequent filtration of solid precipitate, 2.4 g of compound (j) with the structure represented by the following formula was yielded.

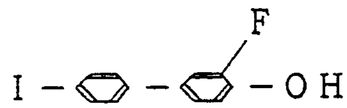 (j)

5) 2.4 g of the compound (j) yielded under 4) was dissolved in 20 ml of dimethylsulfoxide. Sodium hyroxide solution in water(1.0 g/10 ml) and 1.2 g of 1-bromopentane were added thereto and kept stirred for 5 hrs. at 65 °C. The reactant mixture, upon cooled, subjected to hexane extraction followed by water washing repeated twice. Through subsequent removal of hexane, 2.6 g of compound (k) with the structure represented by the following formula was yielded.

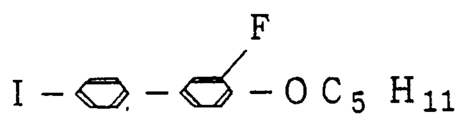 (k)

6) 7.0 g of 2-n-hexyl-5-iodopyridine obtained in the same way as in 1) through 3) of the Example 1 using 1-hexyne instead of 1-decyne and 2.5 g of 3-methyl-1-butyne-3-ol were allowed to react overnight within 70 ml of triethylamine in the presence of 60 mg of bis-triphenylphosphinepalladiumdichloride and 15 mg of cuprous iodide as catalysts under nitrogen atmosphere at room temperature. Upon completion of reaction, solution caused triethylamine to be removed for subsequent hexane extracition.
Hexane layer produced thereby was washed with 1 N hydrochloric acid solution in water, and then with water for subsequent removal of hexane. 5.7 g of compound (1) represented by the following formula was thus yielded.

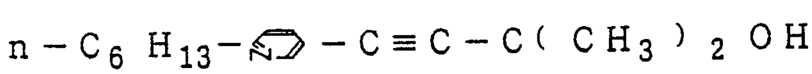 (1)

7) 300 ml of dry toluene with 5.7 g of compound (1) obtained under 6) were added with 1.9 g of sodium hydroxide in powder form and subjected to heating reflux for 1 hr. The solution, upon cooled, was washed with water for subsequent removal of toluene. Through extraction of black colored oil obtained using methanol followed by removal of methanol, 3.1 g of oily compound (m) represented by the following formula was obtained.

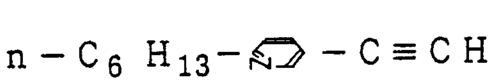 (m)

8) 2.0 g of compound (k) obtained under 5) and 1.0 g of compound (m) obtained under 7) were allowed to react overnight within 60 ml of triethylamine in the presence of 32 mg of bis-triphenylphosphinepalladiumdichloride and 8 mg of cuprous iodide as catalysts under nitrogen atmosphere at room temperature. Upon completion of recation, the solution caused triethylamine to be removed for subsequent toluene extraction. Toluene layer produced thereby was washed with hydrochloric acid solution in water, and then with water for subsequent treatment through a silicagel-column. Through subsequent recrystallization repeated twice using ethanol, 0.95 g of white crystalline compound No. 1a-99 under Table 1a as the compound proposed in the present invention were finally yielded. IR-, H-HMR- and F-NMR spectra of the compound are illustrated in Figs. 16, 17 and 18 respectively.

Results from elemental analysis:

| Calculated values of components(%) | Observed values of components(%) |
|---|---|
| C: 81.27<br>H: 7.67<br>N: 3.16<br>F: 4.29 | C: 81.35<br>H: 7.55<br>N: 3.36<br>F: 4.21 |

Example 24

Preparation of compound No. 1a-123 in the Table 1a

1) 300 ml of dimethylsulfoxide with 6-bromo-2-naphthol dissolved therein were added with sodium hydroxide solution in water (sodium hydroxide 9.0g, water 20 ml) and stirred to a homogeneous state. The solution was then added with 37.0 g of n-hexylbromide and kept stirred for 3 days at room temperature.

The solution was then poured into 1.5 ℓ of ice water for subsequent hexane extraction repeated thrice. The solution resulted had hexane layer produced thereby washed with water, caused hexane to be removed for subsequent recrystallization using methanol. Thus 54.4 g of 2-n-hexyloxy-6-bromonaphthalene was yielded.

2) 13.0 g of 3-n-hexyloxy-6-bromonaphthalene obtained under 1) and 4.3 g of 3-methyl-1-butyne-3-ol were allowed to react for 6 hrs. within 150 ml of triethylamine in the presence of 120 mg of bis-triphenylphosphinepalladiumdichloride, 30 mg of cuprous iodide and 180 mg of triphenylphosphine as catalysts under nitrogen atmosphere at reflux temperature. Upon completion of reaction, the solution caused triethylamine to be removed for subsequent toluene layer produced thereby washed with 1 N hydrochloric acid solution in water, then with water, caused moisture of toluene layer to be removed and was added with 3.5 g of sodium hydroxide in powder form for subsequent heating reflux for 1 hr. The resulted solution, upon cooled, was subjected to water washing followed by removal of toluene. Through subsequent recrystallization of black colored oil obtained using methanol, 5.6 g of 6-n-hexyloxy-2-ethynylnaphthalene was yielded.

3) 1.7 g of 6-n-hexyloxy-2-ethynylnaphthalene obtained under 2) and 2.0 g of 2-n-hexyl-5-iodopyridine obtained under 6) of the Example 23 were allowed to react for 6 hrs. within 30 ml of triethylamine in the presence of 40 mg of bis-triphenylphosphinepalladiumdichloride and 10 mg of cuprous iodide as catalysts under nitrogen atmosphere at room temperature. Upon completion of reaction, the solution caused triethylamine to be removed for subsequent toluene extraction. The solution had then toluene layer produced thereby washed with 1 N hydrochloric acid solution in water, then with water for subsequent treatment through a silicagel-column. Through subsequent removal of toluene followed by recrystallization repeated thrice using ethanol, 1.20 g of white crystalline compound No. 1a-123 under Table 1a as the compound proposed in the present invention were finally yielded. IR and H-NMR spectra of the compund are illustrated in Figs. 19 and 20 respectively.

Results from elemental analysis:

| Calculated values of components(%) | Observed values of components(%) |
|---|---|
| C: 84.27<br>H: 8.47<br>N: 3.39 | C: 83.99<br>H: 8.55<br>N: 3.46 |

Example 25

Preparation of compound No. 1a-131 in the Table 1a

1) 5.3 g of n-hexylalcohol was allowed to drop into 250 ml of dry dimethylformamide containing therein 2.0 g of sodium hydride (60% content) at such a rate that hydrogen evolved not so vigorously. Upon confirmation that evolution of hydrogen was completed, the solution was added with 11.8 g of 2,5-dibromopyridine for subsequent reflux for 2 hrs. Upon completion of reaction, the solution was poured into ice water for subsequent hexane extraction. The solution had then hexane layer produced thereby washed with water and subjected to treatment through a silicagel-column. Thus 8.9 g of 2-n-hexyloxy-5-bromopyridine was yielded.

2) 2.1 g of 2-n-hexyloxy-5-bromopyridine obtained under 1) and 2.0 g of 6-n-hexyloxy-2-ethynylnaphthalene obtained under 2) of the Example 24 were allowed to react for 6 hrs. within 50 ml of triethylamine in the presence of 80 mg of bis-triphenylphosphinepalladiumdichloride, 20mg of cuprous iodide and 120 mg of triphenylphosphine as catalysts under nitrogen atmosphere at room temperature. Upon completion of reaction, the solution caused triethylamine to be removed for subsequent toluene extraction. The solution had then toluene layer washed with 1 N hydrocholric acid solution in water, then with water for subsequent treatment through a silicagel-column.

Through subsequent removal of toluene followed by recrystallization vepeated thrice using ethanol, 1.75 g of white crystalline compound No. 1a-131 under Table 1a as the compound proposed in the present invention was finally yielded. IR and H-NMR spectra of the compound are illustrated in Figs. 21 and 22 respectively.

Results from elemental analysis:

| Calculated values of components(%) | Observed values of components(%) |
|---|---|
| C: 81.12<br>H: 8.16<br>N: 3.26 | C: 81.29<br>H: 8.05<br>N: 3.46 |

Example 26

Preparetion of compound No. 1b-1 in the Table 1b

30 ml of ethanol was added with 0.5 g of compound No. 1a-99 under Table 1a obtained in the Example 23 and 10 mg of 5% palladium-carbon and subjected to hydrogenation under reflux temperature and atmospheric pressure. Upon comfirmation that hydrogen absorption became found no longer taking place, the solution caused catalyst to be removed through filtration with the temperature brought to room temperature. Through subsequent recrystallization of crystal obtained using ethanol, 0,32 g of white crystalline compound No. 1b-1 under Table 1b as the compound proposed in the present invention was finally yielded. IR, H-NMR and F-NMR spectra of the compound are illustrated in Figs. 23, 24 and 25

respectively.

Results from elemental analysis:

| Calculated values of components(%) | Observed values of components(%) |
|---|---|
| C: 80.54<br>H: 8.50<br>N: 3.13<br>F: 4.25 | C: 80.29<br>H: 8.67<br>N: 3.36<br>F: 4.21 |

Example 27

Preparation of compond No. 1C-72 in the Table 1C

1) 7.0 g of 3.6-dichloropyridazine and 3.2 g of 1-decyne were subjected to reflux for 1 hr. within 70 ml of triethylamine in the presence of 180 mg of bistriphenylphosphinepalladiumdichloride and 45 mg of cuprous iodide as catalysts under nitrogen atmosphere. Upon completion of reaction, the reactant caused triethylamine to be removed for subsequent hexane extraction. The hexane layer was washed hydrochloric acid solution in water, then with water. Through treatment using a silicagel-column, 3.0 g of oily compound (m) were yielded with the structure represented by the following formula.

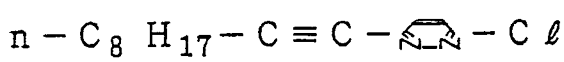

(m)

2) 1.4 ℓ of water containing therein 210 g of sodium bicarbonate was added with 187 g of o-fluorophenol for subsequent cooling down to below 10 °C. 423 g of iodine in fine grain was added thereto in 5 aliquot parts. Upon confirmation that iodine color had disappeared, the solution was subjected to toluene extraction. The toluene layer produced thereby was washed with water, then with sodium hydrogen sulfite solution in water and again with water and caused toluene to be removed. Through distillation under reduced pressure (b.p. 124 to 145 °C/18 mm Hg) the solution turned to show reddish purple color and was then subjected to recrystallization using hexane. 120g of o-fluoro-p-iodophenol were thus yielded in white crystal. 120 ml of dimethylsulfoxide containing therein 15.0 g of said o-fluoro-p-iodophenol were added with sodium hydroxide solution in water (sodium hydroxide 3.0 g, water 10 ml) and stirred to homogenous state. The solution was then added with 10.4 g of n-hexylbromide and kept stirred for subsequent 3 days at room temperature. The reactant was poured into 1l of ice water for subsequent hexane extraction repeated thrice. Through washing of hexane layer produced thereby with water followed by removal of hexane, 16.7 g of oily p-n-hexyloxy-m-fluoroiodobenzen was yielded.

3) 14.0 g of p-n-hexyloxy-m-fluoroiodobenzene and 3-methyl-1-butyne-3-ol were allowed to react overnight within 80 ml of triethylamine in the presence of 272 mg of bis-triphenylphosphinepalladiumdichloride and 68 mg of cuprous iodide as catalysts under nitrogen atmosphere at room temperature. Upon completion of reaction, the reactant caused triethylamine to be removed for subsequent hexane extraction. The hexane layer produced thereby was washed with 1 N hydrochloric acid solution in water and then with water. Through subsequent removal of hexane, 12.5 g of compound (n) was yielded with the structure represented by the following formula.

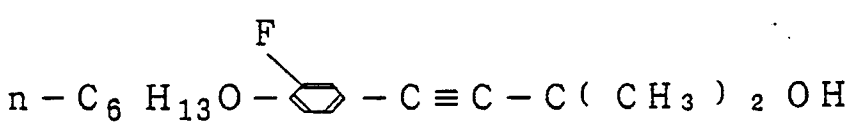

(n)

4) 400 ml of dry toluene containing therein 12.5 g of compound (n) yielded under 3) was added with 5.4

g of sodium hydroxide in powder form and subjected to reflux for 1 hr. The reactant, upon cooled and water washed, caused toluene to be removed. The black oil produced thereby was subjected to methanol extraction. Through removal of methanol, 7.55 g of oily p-n-hexyloxy-m-fluorophenylacetylene was thus yielded.

5) 1.6 g of compound (m) yielded in 1) and 1.4 g of p-n-hexyloxy-m-fluorophenylacetylene were subjected to reflux for 5 hrs. within 30 ml of triethylamine in the presence of 40 mg of bis-triphenylphosphinepalladiumdichloride, 10 mg of cuprous iodide and 60 mg of triphenylphosphine as catalysts under nitrogen atmosphere. Upon completion of reaction, the reactant caused triethylamine to be removed for subsequent toluene extraction. The toluene layer produced thereby was washed with hydrochloric acid solution in water, then with water for subsequent treatment through a silicagel-column. Through recrystallization repeated twice using methanol 0.95 g of compound No. 1c-72 under Tabel 1c proposed in the present invention was yielded.

IR, H-NMR and F-NMR spectra of the compound are illustrated in Figs. 26, 27 and 27 respectively.

Result from elemental analysis:

| Calculated values of components(%) | Observed values of components(%) |
|---|---|
| C: 77.42<br>H: 8.06<br>N: 6.45<br>F: 4.38 | C: 77.25<br>H: 8.36<br>N: 6.21<br>F: 4.45 |

Example 28

Preparation of compound No. 1c-56 in the Table 1c

1) 250 ml of dimethylsulfoxide containing therein 25.0 g of p- iodophenol was added with sodium hydroxide solution in water (sodium hydroxide 5.0 g, water 20 ml) and stirred to homogeneous state. The solution was added with 17.8 g of n-hexylbromide and kept stirred for the following 3 days at room temperature. The reactant was then poured into ice water for subsequent hexane extraction to be repeated thrice. The hexane layer produced thereby was washed with water. Through removal of hexane, 29.5 g oily p-n-hexyloxyiodobenzene was yielded.

2) 29.5 g of p-n-hexyloxyiodobenzene and 12.4 g of 3-methyl-1-butyn-3-ol were allowed to react overnight within 150 ml of triethylamine in the presence of 300 mg of bistriphenylphoshinepalladiumdichloride and 75 mg of cuprous iodide as catalysts under nitrogen atmosphere at room temperature. Upon completion of reaction, the reactant caused triethylamine to be removed for subsequent hexane extraction. The hexane layer produced thereby was washed with 1 N hydrochloric acid solution in water and then with water. Through removal of hexane, 23.2 g of compound (o) were yielded with structure represented by the following formula.

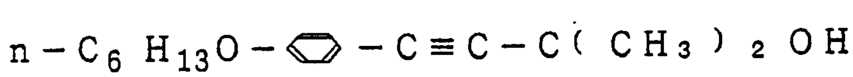 (o)

3) 800 ml of dry toluene containing therein 23.2 g of compound (o) yielded in 2) were added with 10.7 g of sodium hydroxide in powder form and subjected to reflux for 1 hr. The solution ,upon cooled and washed with water, caused toluene to be removed. Black oil produced thereby was then subjected to methanol extraction. Through removal of methanol 12.8 g of oily p-n-hexyloxyphenylacetylen were yielded.

4) 1.0 g of p-n-hexyloxyphenylacetylene yielded under 3) and 1.2g of compound (m) yielded under 1) in the Example 27 were subjected to reflux for 5 hrs. within 30 ml of triethylamine in the presence of 40 mg of bistriphenylphosphinepalladiumdichloride, 10 mg of cuprous iodide and 60 mg of triphenylphosphine as catalysts under nitrogen atmosphere. Upon completion of reaction, the reactant caused triethylamine

to be removed for subsequent toluene extraction. The toluene layer poduced thereby was washed with hydrochloric acid solution in water, then with water and subjected to treatment through a silicagel-column. Through recrystallization repeated twice using methanol, 0.68 g of compound No.1c-56 under Table 1c proposed in the present invention were yielded. IR and H-NMR spectra of the compound are illustrated in Figs. 29 and -30 respectively.

Result from elemental analysis:

| Calculated values of components(%) | Observed values of components(%) |
|---|---|
| C: 80.77<br>H: 8.65<br>N: 3.85 | C: 80.53<br>H: 8.79<br>N: 4.09 |

Example 29

Preparation of compound No. 1c-51 in the Table 1c

1) 17.6 g of n-decylalcohol was allowed to drop onto 400 ml of dry dimethylformamide containing therein 2.67 g of sodium hydride at such a rate that hydrogen evolved not so vigorously at 70 to 80 °C Upon confirmation that the evolution of hydrogen was completed, the temperature of the solution was restored to room temperature, the solution was added with 25.0 g of 3,6-dichloropyridazine and subjected to reflux for 2 hrs. Upon completion of reaction, the reactant was poured into ice water, whereupon precipitate produced thereby was subjected to filtration followed by drying. Such part of the solid matter produced thereby, which is soluble to hexane, was subjected to treatment through a silicagel-column. Through recrystallization using methanol, 19.4 g of 3-n-decyloxy-6-chloropyridazine were yielded.

2) 1.0 g of 3-n-decyloxy-6-chloropyridazine and 0.8 g of p-n-hexyloxy-m-fluorophenylacetylene yielded under 4) of the Example 27 were subjected to reflux for 5 hrs. within 30 ml of triethylamine in the presence of 40 mg of bistriphenylphosphinepalladiumdichloride, 10 mg of cuprous iodide and 60 mg of triphenylphosphine as calatysts under nitrogen atmosphere. Upon completion of reaction, the reactant caused triethylamine to be removed for subsequent toluene extraction. The toluene layer produced thereby was washed with hydrochloric acid solution in water, then with water and subjected to treatment through a siiicagel-column. Through recrystallization repeated twice using methanol, 0.65 g of compound No. 1c-51 under Table 1c proposed in the present invention were yielded IR, H-NMR- and F-NMR spectra of the compound are illustrated in Figs. 31, 32 and 33 respectively.

Result from elemental analysis:

| Calculated values of components(%) | Observed values of components(%) |
|---|---|
| C: 74.00<br>H: 8.59<br>N: 6.17<br>F: 4.19 | C: 74.23<br>H: 8.43<br>N: 6.06<br>F: 4.31 |

Example 30

Preparation of compound No. 1c-48 in the Table 1c

1) 1.2 g of 3-n-decyloxy-6-chloropyridazine yielded under 1) of the Example 29 and 0.9 g of p-n-hexyloxyphenylacetylene yielded under 3) of the Example 28 ware subjected to reflux for 5 hrs. within 30 ml of triethylamine in the presence of 40 mg of bis-triphenylphosphinepalladiumdichloride, 10 mg of cuprous iodide and 60 mg of triphenylphosphine as catalysts under nitrogen atmosphere. Upon completion of reaction, the reactant caused triethylamine to be removed for subsequent toluene extraction. The toluene layer produced thereby was washed with hydrochloric acid solution in water, then with water and subjected to treatment through a silicagel-column. Througr recrystallization repeated twice using methanol 0.74 g of compound No, 1c-48 under Table 1c proposed in the present invention were yielded. IR and H-NMR spectra of the compound are illustrated in Figs. 34 and 35 respectively.

Result from elemental analysis:

| Calculated values of components(%) | Observed values of components(%) |
|---|---|
| C: 77.07<br>H: 9.17<br>N: 6.42 | C: 76.83<br>H: 9.31<br>N: 6.28 |

Example 31

Preparation of compound No. 1c-21 in the Table 1c

1) 100 ml of ethanol containing therein 6.8 g of compound(m) obtained through the same process as in 1) of the Example 27 was subjected to hydrogenation under hydrogen atomosphere of atmospheric pressure using 210 mg of platinium oxide. Upon confirmation that hydrogen became found no longer absorbed, the reactant caused the catalyst to be removed through filtration for subsequent removal of ethanol. Through recrystallization repeated twice using hexane, 3.1 g of white solid 3-n-decyl-6-chloropyridadine were thus yielded.

2) 1.9 g of p-n-hexyloxy-m-fluorophenylacetylene obtained through the same process as in 2) to 4) of the Example 27 and 2.2 g of 3-n-decyl-6-chloropyridazine obtained through 1) were subjected to reflux for 5 hrs. within 50 ml of triethylamine in the presence of 100 mg of bistriphenylphosphinepalladiumdichloride, 25 mg of cuprous iodide and 150 mg of triphenylphosphine as catalysts under nitogen atmosphere. Upon completion of reaction, the reactant caused triethylamine to be removed for subsequent toluene extraction. The toluene layer produced thereby was washed with hydrochloric acid solution in water, then with water for subsequent treatment through a silicagel-column. Through subsequent recrystallization repeated twice using ethanol, 1.75 g of compound No. 1c-21 under Table 1c as the compound proposed in the present invention was thus yielded. IR, H-NMR- and F-NMR spectra of the compound are illustrated in Figs. 36, -37 and -38 respectively.

Result from elemental analysis

| Calculated values of components(%) | Observed values of components(%) |
|---|---|
| C: 76.72<br>H: 8.90<br>N: 6.39<br>F: 4.34 | C: 76.56<br>H: 9.12<br>N: 6.24<br>F: 4.29 |

Example 32

Preparation of compound No. 1c-40 in the Table 1c

1) 1.3 g of 3-n-decyl-6-chloropyridazine obtained through the same process as in 1) of the Example 31 and 1.0 g of p-n-hexyloxyphenylacetylene obtained through the same process as in 1) to 3) of the Example 28 were subjected to reflux for 5 hrs. within 30 ml of triethylamine in the presence of 56 mg of bis-triphenylphosphinepalladiumdichloride, 14 mg of cuprous iodide and 94 mg of triphosphine as catalysts under nitrogen atmosphere. Upon completion of reaction, the reactant caused triethylamine to be removed for subsequent toluene extraction. The toluene layer produced thereby was washed with hydrochloric acid solution in water, and then with water for subsequent treatment through a silicagel-column followed by recrystallization repeated twice using ethanol. 0.83 g of compound No. 1c-40 under Table 1c as the compound proposed in the present invention was thus yielded. IR and H-NMR spectra of the compound are illustrated in Figs. 39 and 40 respectively.

Result from elemental analysis

| Calculated values of components(%) | Observed values of components(%) |
|---|---|
| C: 80.00<br>H: 9.52<br>N: 6.67 | C: 79.83<br>H: 9.63<br>N: 6.81 |

Example 33

Preparation of compound No. 1c-109 in the Table 1c

1) 11.4 g of n-hexylalcohol was allowed to drop onto 400 ml of dry dimethyformamide containing therein 2.67 g of sodium hydride at such a rate that hydrogen evoluved not so vigorously at 70 to 80 °C

Upon confirmation that the evolution of hydrogen was completed, the solution was added with 25.0g of 3.6-dichloropyridazine and subjected to reflux for 2 hrs. with the temperature restored to room temperature. Upon completion of reaction, the solution was poured into ice water, precipitate developed being soluble to hexane of the solid obtained thereby was subjected to treatment through a silicagel-column followed by recrystallization using methanol. 13.5 g of 3-n-hexyloxy-6-chloropyridazine was thus yielded.

2) 6.0 g of 3-n-hexyloxy-6-chloropyridazine obtained under 1) and 3.5 g of 3-methyl-1-butyne-3-ol were subjected to reflux for 8 hrs. within 80 ml of triethylamine in the presence of 200 mg of bis-triphenylphosphinepalladiumdichloride, 50 mg of cuprous iodide and 400 mg of triphenylphosphine as catalysts under nitrogen atmosphere. Upon completion of reaction, the reactant caused triethylamine to be removed for subsequent toluene extraction. Toluene layer produced thereby was washed with hydrochloric acid solution in water and then with water. Upon removal of toluene, 6.6 g of the compound (p) represented by the following formula were thus yielded.

$n-C_6H_{13}O-$ 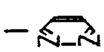 $-C\equiv CC(CH_3)_2OH$ (p)

3) 200 ml of dry toluene containing therein 6.6 g of the compound (p) obtained under 2) was added with 1.5 g of sodium hydroxide in powder form and subjected to heating reflux for 1 hr. The solution, upon cooled, was washed with water for subsequent removal of toluene. Through recrystallization of black colored substance using hexane, 2.3 g of 3-n-hexyloxy-6-ethynylpyridazine were thus yielded.

4) 0.8 g of 3-n-hexyloxy-6-ethynylpyridazine obtained under 3) and 1.5 g of the compound (k) obtained through operation under 1) through 5) of the Example 23 were allowed to react for 3 hrs. at room temperature within 30 ml of triethylamine in the presence of 24 mg of bis-triphenylphosphinepalladiumdichloride and 6 mg of cuprous iodide as catalysts under nitrogen atmosphere. Upon completion of reaction, the solution caused triethylamine to be removed for subsequent toluene extraction. Toluene layer produced thereby was washed with hydrochloric acid solution in water, and then with water for

subsequent treatment through a silicagel-column. Through subsequent recrystallization repeated twice using ethanol, 0.83 g of compound No. 1c-109 under Table 1c as the compound proposed in the present invention was thus yielded. IR-, H-NMR and F-NMR spectra of the compound are illustrated in Figs. 41, -42 and -43 respectively.

Result from elemental analysis

| Calculated values of components(%) | Observed values of components(%) |
|---|---|
| C: 75.65<br>H: 7.17<br>N: 6.09<br>F: 4.13 | C: 75.79<br>H: 7.03<br>N: 5.95<br>F: 4.32 |

Example 34

Preparation of compound No. 1c-131 in the Table 1c

2.0 g of 2-n-hexyloxy-6-ethynylnaphthalene obtained under 2) of the Example 24 and 1.7 g of 3-n-hexyloxy-6-chloropyridazine obtained under 1) of the Example 33 were subjected to reflux for 5 hrs. within 60 ml of triethylamine in the presence of 100 mg of bis-triphenylphosphinepalladiumdichloride, 25 mg of cuprous iodide and 150 mg of triphenylphosphine as catalysts under nitrogen atmosphere. Upon completion of reaction, the reactant caused triethylamine to be removed for subsequent toluene extraction. The toluene layer produced thereby was washed with hydrochloric acid solution in water, and then with water for subsequent treatment through a silicagel-column. Through recrystallization repeated thrice using ethanol, 1.38 g of compound No. 1c-131 under Table 1c as the compound proposed in the present invention was thus yielded. IR and H-NMR spectra of the compound are illustrated in Figs. 42 and 43 respectively.

Result from elemental analysis

| Calculated values of components(%) | Observed values of components(%) |
|---|---|
| C: 78.14<br>H: 7.91<br>N: 6.51 | C: 78.28<br>H: 7.73<br>N: 6.32 |

Example 35

Preparation of compound No. 1d-3 in the Table 1d

30 ml of ethanol containing therein 0.5 g of compound No. 1c-109 under Table 1c obtained in the Example 33 and 10 mg of 5% palladium-carbon were subjected to hydrogenation under atmospheric pressure at reflux temperature. Upon confirmation that hydrogen became found no longer absorbed, the solution caused catalyst to be removed through filtration with the temperature cooled down to room temperature. Through recrystallization of the crystal produced thereby using ethanol, 0.38 g of white crystalline compound No. 1d-3 under Table 1d as the compound proposed in the present invention was thus yielded. IR-, H-NMR- and F-NMR spectra of the compound are illustrated in Fig. 46, -47 and -48 respectively.

Result from elemental analysis

| Calculated values of ccmponents(%) | Observed values of components(%) |
|---|---|
| C: 75.00<br>H: 7.97<br>N: 6.03<br>F: 4.10 | C: 75.21<br>H: 7.76<br>N: 5.94<br>F: 4.05 |

Example 36

Preparation of compound No. 1e-13 in the Table 1e

1) 100 ml of ethanol containing therein 10.0 g of 2-chloro-5-nitropyridine were subjected to reduction of nitro-group using platinum oxide under hydrogen atomosphere of atmospheric pressure. Upon confirmation that hydrogen became found no longer absorbed, the solution caused catalyst to be removed through filtration for subsequent removal of ethanol. Through recrystallization of solid matter produced thereby using toluene, 5.8 g of 2-chloro-5-aminopyridine was thus yielded.

2) 400 ml of water containing therein 45 g of 36 hydrochloric acid solution were added with 5.8 g of 2-chloro-5-aminopyridine obtained under 1) for subsequent cooling down to below 5 °C. Sodium nitrite solution in water (sodium nitrite 3.1 g, water 30 ml) prepared separately was allowed to drop thereon under temperature below 5 °C. Upon completion of dropping process, the solution was kept stirred for additional 1 hr. and then added with potassium iodide solution in water (potassium iodide 15 g, water 30 ml) with the temperature slowly restored to room temperature the solution being kept stirred until the evolution of nitrogen was completed. Upon completion of reaction, the solution was made neutralized using sodium hydroxide, whereupon it was added with sodium hydrogen sulfite for subsequent stirring for 1 hr. Through filtration of precipitate produced thereby, followed by water washing and subsequent recrystallization using hexane, 7.3 g of 2-chloro-5-iodopyridine were thus yielded.

3) 7.0 g of 2-chloro-5-iodopyridine obtained under 2) and 3.2 g of 1-octyne were allowed to react for 5 hrs. at room temperature within 100 mg of triethylamine in the presence of 120 mg of bistriphenylphosphinpalladiumdichloride and 30 mg of cuprous iodide as catalysts under nitrogen atmosphere. Upon completion of reaction, the reactant caused triethylamine to be removed for subsequent hexane extraction. Hexane layer produced thereby was washed with hydrochloric acid solution in water, then with water for subsequent treatment through a silicagel-column.

5.9 g of oily compound (q) represented by the following formula were thus yielded.

$n-C_6H_{13}-C\equiv C-$ 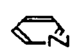 $-C\ell$ (q)

4) 80 ml of ethanol containing therein 5.9 g of the compound (q) obtained under 3) were subjected to hydrogenation using platinum oxide under hydrogen atmosphere of atmospheric pressure. Upon confirmation that hydrogen absorption became found no longer taking place, the solution caused catalyst to be removed through filtration for subsequent removal of ethanol. Through dissolving the solution produced thereby in hexane followed by purification through a silicagel-column, 5.4 g of 2-chloro-5-octylpyridine was thus yielded.

5) 1.6 g of p-n-octyloxy-m-fluorophenylacetylene obtained using n-octylbromide instead of n-hexylbromide in the same manner as in 4) to 7) of the Example 1 and 1.5g of 2-chloro-5-octylpyridine obtained under 4) were subjected to reflux for 8 hrs. within 60 ml of triethylamine in the presence of 48 mg of bis-triphenylphosphinepalladiumdichloride, 12 mg of cuprous iodide and 96 mg of triphenylphosphine as catalysts under nitrogen atmosphere. Upon completion of reaction the reactant caused triethylamine to be removed for subsequent toluene extraction. Toluene layer produced thereby was washed with hydrochloric acid solution in water and then with water for subsequent treatment through a silicagel-column. Through recrystallization repeated thrice using ethanol, 0.54 g of compound No. 1e-131 under Table 1e as the compound proposed in the present invention was thus yielded. IR, H-NMR- and F-NMR spectra of the compound are illustrated in Figs. 49, -50 and -51 respectively.
Result from elemental analysis

| Calculated values of components(%) | Observed values of components(%) |
|---|---|
| C: 79.64<br>H: 9.15<br>N: 3.20<br>F: 4.35 | C: 79.53<br>H: 9.21<br>N: 3.34<br>F: 4.29 |

Example 37

Preparation of compound No. 1e-56 in the Table 1e

1) 250 ml of dimethylsulfoxide containing 25.0 g of p-iodophenol dissolved therein were added with sodium hydroxide solution in water (sodium hydroxide 5.0 g, water 20 ml) and stirred to homogenous state. The solution was added with 17.8 g of n-hexylbromide and kept stirred for 3 days at room temperature. The reactant was poured into 1 ℓ of ice water so as to be extracted thrice using hexane. The hexane layer was washed with water, caused hexane to be removed, whereupon 29.5 g of oily p-n-hexyloxyiodobenzene were yielded.

2) 29.5 g of p-n-hexyloxyiodobenzene and 12.4 g of 3-methyl-1-butyne-3-ol allowed to react overnight within 150 ml of triethylamine in the presence of 300 mg of bis-triphenylphosphinepalladiumdichloride and 75 mg of cuprous iodide as catalysts under nitrogen atmosphere at room temperature. Upon completion of reaction, the reactant caused triethylamine to be removed for subsequent hexane extraction. Through washing of hexane layer with 1 N hydrochloric acid solution in water, then with water followed by removal of hexane 23.2 g of solid compound (r) with the structure represented by the following formula was yielded.

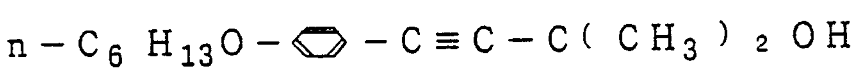 (r)

3) 800 ml of dry toluene containing 23.2 g of compound (r) yielded under 2) dissolved therein was added with 10.7 g of sodium hydroxide in powder form and subsequent reflux for 1 hr. The reactant, upon cooled and washed with water, caused toluene to be removed. The black oil produced thereby was subjected to methanol extraction. Through removal of methanol, 12.8 g of oily p-n-hexyloxyphenylacetylene were yielded.

4) 4.2 g of p-n-hexyloxyphenylacetylene yielded under 3) and 5.0 g of 2.5-dibromopyridine were allowed to react overnight within 80 ml of triethylamine in the presence of 160 mg of bis-triphenylphosphinepalladiumdichloride and 40 mg of cuprous iodide as catalysts under nitrogen atmosphere at room temperature. Upon completion of reaction, the reactant caused triethylamine to be removed for subsequent toluene extraction. The toluene layer produced thereby was washed with hydrochloric acid solution in water, then with water and caused toluene to be removed. Through recrystallization using methanol, 6.5 g of compound (s) with the structure represented by the following formula were yielded.

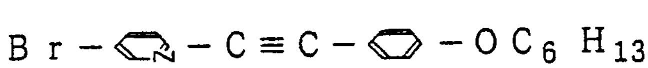 (s)

5) 1.0 g of 1-decyne and 1.3 g of the compound (s) yielded under 4) were allowed to react for 5 hrs. within 40 ml of triethylamine in the presence of 80 mg of bis-triphenylphosphinepalladiumdichloride and 20 mg of cuprous iodide under nitrogen atmosphere at reflux temperature. Upon completion of reaction, the reactant caused triethylamine to be removed for subsequent hexane extraction. The hexane layer produced thereby was washed with hydrochloric acid solution in water, then with water and subjected to

treatment through a silicagel-column. Through recrystallization repeated twice using ethanol 0.54 g of white crystalline compound No. 1e-56 under Table 1e proposed in the present invention were yielded. IR and H-NMR spectra of the said compound are illustrated in Figs. 52 and -53 respectively.

Result from elemental analysis

| Calculated values of components(%) | Observed values of components(%) |
|---|---|
| C: 83.85<br>H: 8.92<br>N: 3.37 | C: 83.62<br>H: 9.03<br>N: 3.60 |

Example 38

Preparation of compound No. 1e-72 in the Table 1e

1) 1.4 l of water containing 210 g of sodium bicarbonate were added with 187 g of orthofluorophenol and allowed to be cooled down to temperature below 10 °C. The mixture was added with 423 g of grain iodine in five aliquot parts. Upon confirmation that iodine color had disappeared, extraction of mixture took place using toluene. The resulted toluene layer was washed with water, then with sodium hydrogen sulfite solution in water, and again with water. And then toluene was evaporated therefrom. The fluid in reddish purpul color obtained through distillation under reduced pressure (b.p. 124 to 145 °C/18mmHg) was subjected to a recrystallization using hexane, whereupon 120 g of o-fluoro-p-iodophenol were finally yielded in white crystal.

2) 120 ml of dimethylsulfoxide, into which 15.0 g of said o-fluoro-p-iodophenol had been dissolved, was added with sodium hydroxide solution in water (sodium hydroxide 3.0 g and water 10 ml ) and stirred into a homogenous state. The mixture was then added with 10.4 g of n-hexylbromide and stirred for 3 days at room temperature. The reactant was transferred into 1 ℓ of ice water so as to be extracted thrice using hexane. The resulted hexane layer was washed with water and the hexan was removed therefrom, 16.7 g of oily p-n-hexyloxy-m-fluoroiodobenzene was finally obtained.

3) 9.5 g of 2.5-dibromopyridine and 4.0 g of 3-methy-1-butyne 3-ol were allowed to react overnight within 80 ml of triethylamine in the presence of 320 mg of bistriphenylphosphinepalladiumdichloride and 80mg of cupurous iodide as catalysts under nitrogen atmosphere at room temperature. Upon completion of reaction process, the reactant caused triethylamine to be removed for subsequent toluene extraction. The toluene layer produced thereby was washed with hydrochloric acid solution in water, then with water followed by removal of toluene, 7.7 g of compound (t) with the structure represented by the following formula was thus yielded.

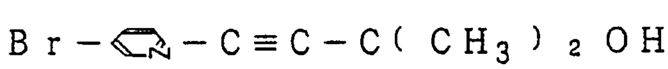

(t)

4) 3.2 g of compound (t) yielded under 3) and 2.7 g of 1-decyne were allowed to react for 5 hrs. within 50 ml of triethylamine in the presence of 240 mg of bis-triphenylphosphinepalladiumdichloride and 60 mg of cuprous iodide as catalysts under nitrogen atmosphere at reflux temperature. Upon completion of reaction, the solution caused triethylamine to be removed for subsequent hexane extraction. The hexane layer produced thereby was washed with hydrochloric acid solution in water, then with water, caused hexane to be removed for subsequent methanol extraction. Through removal of methanol, 3.6 g of compound (u) with the structure represented by the following formula was yielded.

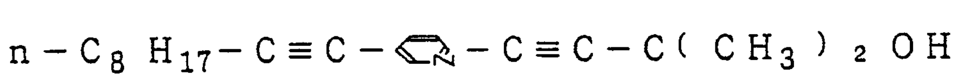

(u)

5) 100 ml of dry toluene containing 3.6 g of compound (u) yielded under 4) dissolved therein was added

with 1.5g of sodium hydroxide in powder form and subjected to reflux for 1 hr. The solution was allowed to be cooled, washed with water for subsequent removal of toluene. The black oil produced thereby was dissolved in hexane and subjected to purification through a silicagel-column, whereupon 1.0 g of compound (v) with the structure represented by the following formula was yielded.

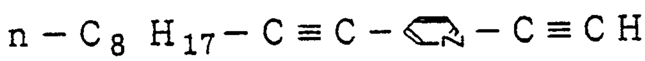

(v)

6) 1.4 g of p-n-hexyloxy-m-fluoroiodobenzene yielded under 2) and 1.0 g of compound (v) yielded under 5) were allowed to react overnight within 30 ml of triethylamine in the presence of 40 mg of bis-triphenylphosphinepalladiumdichloride and 10 mg of cuprous iodide as catalysts under nitrogen atmosphere at room temperature. Upon completion of reaction, the reactant caused triethylamine to be removed for subsequent hexane extraction. The hexane layer produced thereby was washed with hydrochloric acid solution in water, then with water and subjected to treatment through a silicagel-column. Through subsequent recrystallization repeated twice using methanol, 0.47 g of white crystalline compound No. 1e-72 under the Table 1e proposed in the present invention was yielded. IR H-NMR and F-NMR spectra of the compound are illustrated in Figs. 54, 55 and 56 respectively.

Result from elemental analysis:

| Calculated values of components(%) | Observed values of components(%) |
|---|---|
| C: 80.37<br>H: 8.31<br>N: 3.23<br>F: 4.39 | C: 80.12<br>H: 8.53<br>N: 2.99<br>F: 4.61 |

Example 39

Preparation of compound No. 1g-8 in the Table-1g

1) 200 ml of ethanol containing therein 15.0 g of β-n-heptyl-α-dimethylaminoacrolein (obtainable through allowing Vilsmeier reagent to react with n-heptylacetoaldehydediethylacetal which was derived from the reaction between n-octylmagnesiumbromide and triethylorthoformate) and 6.8 g of urea were added with 3.0 g of concentrated hydrochloric acid for subsequent reflux for 15 hrs. Upon completion of reaction, the reactant caused ethanol to be removed and then added with water. The solution was made neutralized using sodium hydroxide, whereupon precipitate produced thereby was filtrated and dried. Through recrystallization using toluene, 11.4 g of 5-n-heptyl-2-hydroxypyrimidine was thus yielded.

2) A mixture containing therein 7.2 g of 5-n-heptyl-2-hydroxypyrimidine obtained under 1) and 1.8 g of N,N-dimethylaniline was added with 28.4 g of phosphorus oxychloride for subsequent reflux for 5 hrs. Upon completion of reaction, the reactant with its temperature restored to room temperature was poured into ice water. The solution was then made neutralized using sodium hydroxide for subsequent ether extraction. Ether layer produced thereby was washed with 1 N hydrochloric acid solution in water, and then with water for subsequent removal of ether followed by dissolving in hexane and following purification through a silicagel-column. 6.5 g of oily 2-chloro-5-heptylpyrimidine was thus yielded.

3) 1.0 g of 2-chloro-5-heptylpyrimidine and 1.0 g of p-n-octyloxy-m-fluorophenylacetylene was allowed, to react for 10 hrs. within 30 ml of triethylamine in the presence of 60 mg of bis-triphenylphosphinepalladiumdichloride, 15 mg of cuprous iodide and 90 mg of triphenylphosphine as catalysts under nitrogen atmosphere at reflux temperature. Upon completion of reaction, the reactant caused triethylamine to be removed for subsequent toluene extraction. Toluene layer produced thereby was washed with hydrochloric acid solution in water, then with water for subsequent treatment through a silicagel-column followed by recrystallization repeated twice using ethanol. 0.38 g of white crystalline compound No. 1g-8 under Table 1g as the compound proposed in the present invention was thus yielded. IR, H-NMR- and F-NMR

spectra of the compound are illustrated in Figs. 57, -58 and -59 respectively.

Result from elemental analysis

| Calculated values of components(%) | Observed values of components(%) |
|---|---|
| C: 76.42<br>H: 8.73<br>N: 6.60<br>F: 4.48 | C: 76.30<br>H: 8.91<br>N: 6.45<br>F: 4.56 |

Example 40

Preparation of compound No. 1g-104 in the Table 1g

1) 3.4 g of 2-chloro-5-heptylpyrimidine obtained under 1) to 2) of the Example 39 and 1.6 g of 3-methyl-1-butyne-3-ol were allowed to react for 13 hrs. within 40 ml of triethylamine in the presence of 100 mg of bistriphenylphosphinepalladiumdichloride, 25 mg of cuprous iodide and 200 mg of triphenylphosphine as catalysts under nitrogen atmosphere at reflux temperature. Upon completion of reaction, the reactant caused triethylamine to be removed for subsequent toluene extraction. Toluene layer produced thereby was washed with 1 N hydrochloric acid solution in water, and then with water for subsequent removal of toluene followed by recrystallization using hexane. 2.8 g of solid compound (w) with the structure represented by the following formula.

$n-C_7H_{15}-$ 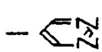 $-C\equiv CC(CH_3)_2OH$ (w)

2) 100 ml of dry toluene containing 2.8 g of compound (w) was added with 1.7 g of sodium hydroxide in powder form for subsequent reflux for 1 hr. The solution was subjected to cooling followed by water washing and following removal of toluene. The black colored oil produced thereby was subjected to dissolving in hexane followed by purification through a silicagel-column. 1.7 g of 2-ethynyl-5-n-heptyl-pyrimidine was thus yielded. 3) 1.5g of 3-fluoro-4-n-hexyloxy-4′-iodobiphenyl synthesized using n-hexylbromide instead of n-pentylbromide in the same manner as in 1) to 5) of the Example 23 and 0.8 g of the compound obtained under 2) were allowed to react for 5 hrs. within 50 ml of triethylamine in the presence of 20 mg of bis-triphenylphosphinepalladiumdichloride and 5 mg of cuprous iodide as catalysts under nitrogen atmosphere at room temperature. Upon completion of reaction, the reactant caused triethylamine to be removed for toluene extraction. Toluene layer produced thereby was washed with 1 N hydrochloric acid solution in water, and then with water for subsequent treatment through a silicagel-column. Through subsequent recrystallization repeated twice using ethanol, 0.97 g of white crystalline compound No. 1g-104 under Table 1g as the compound proposed in the present invention was finally yielded. IR, H-NMR and F-NMR spectra of the compound are illustrated in Figs. 60, -61 and -62 respectively.

Result from elemental analysis

| Calculated values of components(%) | Observed values of components(%) |
|---|---|
| C: 78.81<br>H: 7.84<br>N: 5.93<br>F: 4.03 | C: 78.65<br>H: 7.65<br>N: 6.01<br>F: 4.12 |

Example 41

Preparation of compound No. 1g-124 in the Table 1g

1) 2.0 g of 2-n-hexyloxy-6-ethynylnaphthalene obtained under 1) and 2) of the Example 24 and 1.7 g of 2-chloro-5-heptylpyrimidine obtained under 1) and 2) of the Example 39 were allowed to react for 6 hrs. within 60 ml of triethylamine in the presence of 100 mg of bistriphenylphosphinepalladiumdichloride, 25 mg of cuprous iodide and 150 mg of triphenylphosphine as catalysts under nitrogen atmosphere at reflux temperature. Upon completion of reaction, the reactant caused triethylamine to be removed for subsequent toluene extraction. Toluene layer produced thereby was washed with hydrochloric acid solution in water and then with water for subsequent treatment through a silicagel-col,umn. Through subsequent recrystallization repeated twice using ethanol, 1.05 g of white crystalline compound No. 1g-124 in the Table 1g as the compound proposed in the present invention were thus yielded. IR and H-NMR spectra of the compound are illustrated in Figs. 63 and -64 respectively.

Result from elemental analysis

| Calculated values of components(%) | Observed values of components(%) |
|---|---|
| C: 81.31<br>H: 8.41<br>N: 6.54 | C: 81.19<br>H: 8.53<br>N: 6.45 |

Example 42

Preparation of compound No. 1i-16 in the Table 1i

1) p-iodobenzoic acid 57.0 g and 130 ml of thionyl chloride were subjected to reflux for 8 hrs. to obtain of p-iodobenzoyl chloride. Upon removal of excess thionyl chloride, the last mentioned compound was made dissolved in 400 ml of dry toluene for subsequent cooling down to below 10 °C. Ammonium gas with a temperature below 20 °C was allowed to be blown onto the surface of said toluene solution being kept stirred. Precipitate produced thereby was subjected to filtration and subsequent drying followed by washing with 1 N sodium hydroxide, then with water and subsequent drying. Through recryatallization using methanol, 50.6 g of p-iodobenzamide was thus yielded.

2) 300 ml of dry dioxane containing 50.0 g of p-iodobenzamide suspended therein were cooled down to below 10 °C. 51.0 g of trifluoroacetic anhydride was allowed to drop thereon at a temperature below 20 °C. Upon completion of dropping process the solution was kept stirred overnight with the temperature restored to room temperature. Solution produced thereby in transparent red color was poured into ice water, whereupon precipitate developed was subjected to filtration and subsequent drying. Through recrystallization using hexane, 33.2 g of p-cyanoiodobenzene was thus yielded.

3) 12 g of dry hydrogen chloride was allowed to be blown into 400 ml of dry ethanol containing therein 33.2 g of p-cyanoiodobenzene being kept stirred at a temperature below 10 °C. The solution resulted was kept stirred for 3 days at room temperature, caused solvent to be removed, and then added slowly with 500 ml of dry ethanol containing therein approximately 30 g of dry ammonium under ice cooled condition with the following stirrer kept for another 3 days at room temperature. Upon removal of ethanol and ammonium, the solution was added with dry ether whereupon precipitate devloped was subjected to filtration. 20.8 g of solid p-iodobenzamidine salt of hydrochloride was thus yielded.

4) Solution consisting of 150 ml of dry ethanol and 1.33 g of sodium metal was added with 5.3 g of p-iodobenzamidine salt of hydrochloride obtained under 3) and 4.3 g of $\beta$-n-nonyl-$\alpha$-dimethylaminoacrolein and then subjected to reflux for 20 hrs. Upon completion of reaction, the reactant caused solvent to be removed for subsequent toluene extraction. Toluene layer produced thereby was washed with 1 N hydrochlric acid solution in water, then with water for subsequent removal of toluene. Through recrystallization using methanol, 3.9 g of 2-p-iodophenyl-5-nonylpyrimidine were thus yielded.

5) 0.8 g of 2-p-iodophenyl-5-nonylpyrimidine and 0.5 g of p-n-hexyloxy-m-fluorophenylacetylene were allowed to react for 5 hrs. within 30 ml of triethylamine in the presence of 12 mg of bis-triphenylphosphinepalladiumdichloride and 3 mg of cuprous iodide as catalysts under nitrogen atmosphere at room temperature. Upon completion of reactive process, the reactant caused triethylamine to be removed for subsequent toluene extraction. Toluene layer produced thereby was washed with 1 N hydrochloric acid solution in water, then with water for subsequent treatment through a silicagel-column. Through recrystallization repeated twice using ethanol, 0.43 g of white crystalline compound No. 1i-16 under Table 1i as the compound proposed in the present invention were finally yielded. IR, H-NMR and F-NMR spectra of the compound are illustrated in Figs. 65, -66 and -67 respectively.

Result from elemental analysis

| Calculated values of components(%) | Observed values of components(%) |
|---|---|
| C: 79.20<br>H: 8.20<br>N: 5.60<br>F: 3.80 | C: 79.31<br>H: 8.07<br>N: 5.73<br>F: 3.65 |

Example 43

Preparation of compound No. 1h-2 in the Table 1h

20 ml of ethanol added with 0.2 g of compound No.1g-104 in the Table 1g of the Example 40 and 10 mg of 5% palladium-carbon were subjected to hydrogenation at reflux temperature and atmospheric pressure, Upon comfirmation that hydrogen absorption became found no longer taking place, the solution caused catalyst to be removed through filtration for subsequent cooling down to room temporature. Through recrystallization of crystal obtained above developed using ethanol, 0.15 g of white crystalline compound No. 1h-2 in the Table 1h as the compound proposed in the present invention was finally yielded. IR, H-NMR and F-NMR spectra of the compound are illustrated in Figs. 68, -69 and -70 respectively.

Result from elemental analysis:

| Calculated values of components(%) | Observed values of components(%) |
|---|---|
| C: 78.16<br>H: 8.61<br>N: 5.88<br>F: 3.99 | C: 78.31<br>H: 8.43<br>N: 5.97<br>F: 4.10 |

Example 44

Preparation of compound No. 1k-8 in the Table 1k

1) 1.4 l of water containing 210 g of sodium bicarbonate were added with 187 g of o-fluorophenol and allowed to be cooled down to temperature below 10 °C. The mixture was added with 423 g of grain iodine in five aliquot parts. Upon confirmation that iodine color has disappeared, extraction of mixture took place using toluene. The resulted toluene layer was washed with water, then with sodium hydrogen sulfite solution in water, and again with water. And then toluene was evaporated therefrom. The fluid in

reddish purpul color obtained through distillation under reduced pressure (b.p. 124 to 145 °C/18mmHg) was subjected to a recrystallization using hexane, whereupon 120 g of o-fluoro-p-iodophenol was finally yielded in white crystal.

120 ml of dimethylsulfoxide, into which 15.0 g of said o- fluoro-p-iodophenol has been dissolved, was added with sodium hydroxide solution in water (sodium hydroxide 3.0 g and water 10 ml) and stirred into a homogenous state. The mixture was then added with 10.4 g of n-hexylbromide and stirred for 3 days at room temperature. The reactant was poured into 1 ℓ of ice water so as to be extracted thrice using hexane. The resulted hexane layer was washed with water and the hexan was removed therefrom, 16.7 g of oily p-n-hexyloxy-m-fluoroiodobenzene was finally obtained.

2) 350 ml of dimethylsulfoxide, into which 34.0 g of p-iodophenol has been dissolved, was added with sodium hydroxide solution in water (sodium hydroxide 7.4 g and water 50 ml) and stirred into a homogeneous state. The mixture was added with 32.4 g of n-decylbromide and stirred for 3 days at room temperature. The reactant was poured into 1 ℓ of ice water and then subjected to extraction repeated thrice using hexane. The resulted hexane layer was washed with water and hexane was removed therefrom, then 46.6 g of oily p-n-decyloxy-iodobenzene were finally yielded.

3) 30 g of p-n-decyloxy-iodobenzene and 8.4 g of 3-methyl-1-butyne-3-ol were allowed to react overnight within 200 ml of triethylamine in the presence of 320 mg of bistriphenylhosphinepalladiumdichloride and 80 mg of cuprous iodide as catalysts under nitrogen atmosphere at room temperature. Upon completion of reaction, the reactant caused triethylamine to be removed and was subjected to an extraction using hexane. The resulted hexane layer was washed with 1 N hydrochloric acid solution in water, then with water and caused hexane to be removed, whereupon 22.8 g of solid compound with the structure represented by the following formula (x) was thus yielded.

$n-C_{10}H_{21}O-$ 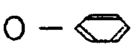 $-C\equiv C-C(CH_3)_2-OH$ (x)

4) 22.8 g of compound (x) yielded under 3) dissolved in 400 ml of dry toluene, into which 9.9 g of sodium hydroxide in powder form was added. The mixture was then subjected to a reflux for 1 hr. It was allowed to be cooled, washed with water and caused toluene to be removed. The residual black oil was subjected to extraction using methanol. Through removal of methanol, 13.2 g of oily p-n-decyloxyphenylacetylen were yielded.

5) 1.6 g of p-n-hexyloxy-m-fluoroiodobenzen obtained under 1) and 1.3 g of p-n-decyloxyphenylacetylen obtained under 4) were allowed to react overnight within 30 ml of triethylamine in the presence of 40 mg of bistriphenylhosphinepalladiumdichloride and 10 mg of cuprous iodide as catalysts under nitrogen atmosphere at room temperature. Upon completion of reaction the reactant caused triethylamine to be removed so as to be subjected to an extraction using hexane. The resulted hexan layer was washed with 1 N hydrochloric acid solution in water and then with water. It was further subjected to a treatment using a silicagel-column followed by recrystallization repeated twice using ethanol, whereupon 1.7 g of white crystalline compound No. 1k-8 under the Table 1k proposed in the present invention were thus yielded. IR , H-NMR and F-NMR spectrum of said compound are illustrated in Fig. 71, -72 and -73 respectivery.

Result from elemental analysis:

| Calculated values of components(%) | Observed values of components(%) |
|---|---|
| C: 79.65<br>H: 9.07<br>F: 4.20 | C: 79.38<br>H: 9.21<br>F: 4.09 |

Example 45

Preparation of compound No. 1k-25 in the Table 1k

1) 350 ml of dimethylsulfoxide, into which 34.0 g of p-iodophenol has been dissolved, was added with sodium hydroxide solution in water (sodium hydroxide 7.4 g and water 50 ml) and stirred into a homogeneous state. The mixture was added with 32.4 g of n-decylbromide and stirred for 3 days at room temperature. The reactant was poured into 1 l of ice water and then subjected to extraction repeated thrice using hexane. The resulted hexane layer was washed with water and hexane was removed therefrom, then 46.6 g of oily p-n-decyloxy-iodobenzene was finally yielded.

30 g of p-n-decyloxy-iodobenzene obtained above and 8.4 g of 3-methyl-1-butyne-3-ol were allowed to react overnight within 200 ml of triethylamine in the presence of 320 mg of bistriphenylhosphinepalladiumdichloride and 80 mg of cuprous iodide as catalysts under nitrogen atmosphere at room temperature. Upon completion of reaction, the reactant caused triethylamine to be removed and was subjected to an extraction using hexane. The resulted hexane layer was washed with 1 N hydrochloric acid solution in water, then with water and caused hexane to be removed, whereupon 22.8 g of solid compound with the structure represented by the following formula (x) was thus yielded.

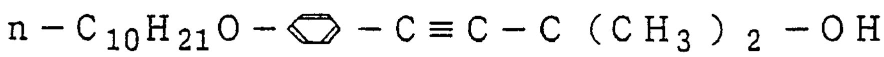 (x)

22.8 g of compound (x) yielded above was dissolved in 400 ml of dry toluene, into which 9.9 g of sodium hydroxide in powder form was added. The mixture was then subjected to a reflux for 1 hr. It was allowed to be cooled, washed with water and caused toluene to be removed. The residual black oil was subjected to extraction using methanol. Through removal of methanol, 13.2 g of oily p-n-decyloxyphenylacetylen were yielded.

2) 1000ml of water and 1000 ml of pyridine containing therein 109 g of p-iodo-o-fluorotoluene were added with 180 g of potassium permanganate in 5 aliquot parts under reflux. Upon completion of adding the last aliquot part, the solution was kept under reflux until color of potassium permangante disappeared. Upon completion of reaction, and of removal of unreacted p-iodo-o-fluorotoluene through distillation, the solution was made cooled down to room temperature. Upon completion of removal of manganese dioxide through filtration under reduced pressure, the filtrate was added with concentrated hydrochloric acid to make solution acidic. The white precipitate produced thereby was subjected to isolation through filtration under reduced pressure followed by water washing and subsequent drying. Through recrystallization using toluene 42 g of p-iodo-o-fluorobenzoic acid was yielded.

3) 5.33 g of p-iodo-o-fluorobenzoic acid yielded under 2) was added with 10 ml of thionyl chloride and subject to reflux for 5 hrs. Subsequent removal of excess thionyl chloride gave p-iodo-o- fluorobenzoyl chloride which was, upon dissolved in 20 ml of dry toluene without further purification, made in use in subsequent reactive process. Said acid chloride which was dissolved in toluene was allowed to drop for 30 min. onto 30 ml of dry toluene containing 1.50 g of 1-butanol and 3.2 g of dry pyridine which were separately prepared and kept cooled using ice water. The dropping proces was carried out under stirring and upon completion of dropping process the stirring continued for susequent 5 hrs. on 90 °C water bath. The reactant was upon cooled and washed with 1 N hydrochloric acid solution in water, with water, with sodium bicarbonate solution in water and again with water, caused toluene to be removed under reduced pressure. Through a silicagel-column purification of such a part of the matter which was soluble in hexane, 6.09 g of oily p-iodo-o-fluorobenzoic acid butyl ester was then yielded.

4) 1.3 g of p-n-decyloxyphenylacetylene yielded under 1) and 1.6 g of p-iodo-o-fluorobenzoicacid butyl ester yielded under 3) were allowed to react overnight within 30 ml of triethylamine in the presence of 40 mg of bistriphenylphosphinepalladiumdichloride and 10 mg of cuprous iodide as catalysts under nitrogen atmosphere at room temperature. Upon completion of reaction, the reactant caused triethylamine to be removed for subsequent hexane extraction. The hexane layer produced thereby was washed with 1 N hydrochloric acid solution in water, then with water and subjected to treatment through a silicagel-column. Through recystallization repeated twice using methanol, 1.7 g of white crystalline compound No. 1k-25 under Table 1k proposed in the present invention were thus yielded. IR, H-NMR, F-NMR spectra are illustrated in Figs. 74, -75 and -76 respectively.

Result from elemental analysis:

| Calculatedvalues of components(%) | Observed values of components(%) |
|---|---|
| C: 76.99<br>H: 8.19<br>F: 4.20 | C: 77.13<br>H: 8.02<br>F: 4.04 |

Example 46

Preparetion of compound No. 2a-12 in the Table 2a

120 ml of dry ether solution containing Grignard reagent which was prepared from 15.0 g of p-n-hexyloxy-m-fluoroiodobenzene and magnesium metal and 13.2 g of 2,5-dibromopyridine solution in dry tetrahydrofuran were allowed to react for 5 hrs. in the presence of 338 mg of dichlorobisdiphenylphosphinobutanepalladium as catalyst under nitrogen atmosphere at room temperature. The on-going reaction was suspended with water and the solution was subjected to ether extraction followed by washing with 1-N hydrochloric acid, and then with water for subsequint removal of ether. Through recrystallization of solid matter resulted using methanol, 10.0 g of 2-p-hexyloxy-m-fluorophenyl-5-bromopyridine was thus yielded.

2) 2.0 g of 2-p-hexyloxy-m-fluorophenyl-5-bromopyridine obtained under 1) and 1.6 g of p-n-hexyloxy-m-fluorophenylacetylene were subjected to reflux for 5 hrs. within 50 ml of triethylamine in the presence of 122 mg of dichlorobistriphenylphosphinepalladium, 31 mg of cuprous iodide and 160 mg of triphenylphosphine as catalysts under nitrogen atmosphere. The reactant, upon cooled, caused triethylamine to be remved for subsequent toluene extraction. Toluene layer produced thereby was washed with 1 N hydrochloric acid, and then with water for subsequent purification through a silicagel-column. Through recrystallization repeated twice of the solid crystal obtained thereby using ethanol, 2.0 g of compound No. 2a-12 in the Table 2a as the compound proposed in the present invention was finally yielded. IR,H-NMR and F-NMR spectra of the compound are illustrated in Figs. 77, -78 and 79 respectively.

Result from elemental analysis

| Calculatedvalues of components(%) | Observed values of components(%) |
|---|---|
| C:75.76<br>H: 7.13<br>F: 7.74<br>N: 2.85 | C:75.70<br>H: 7.23<br>F: 7.82<br>N: 2.83 |

Example 47

Prepration of compound No. 2a-6 in the Talbe 2a

1) 2.0 g of 2-p-hexyloxy-m-fluorophenyl-5-bromopyridine obtained under 1) of the Example 46 and 1.5 g of p-n-hexyloxyphenylacetylene were subjected to reflux for 5 hrs. within 50 ml of triethylamine in the presence of 122 mg of dicholrobistriphenylphosphinepalladium, 31 mg of cuprous iodide and 160 mg of triphenylphosphine as catalysts under nitrogen atmosphere. The solution, upon cooled caused triethylamine to be removed for subsequent toluene extraction. Toluene layer produced thereby was washed with 1 N hydrochloric acid, and then with water for subsequent purification through a silicagel-column chromatography. Through recrystallization repeated twice of the solid matter obtained thereby

using ethanol, 1.7 g of compound No. 2a-6 under Table 2a as the compound proposed in the present invention was finally yielded. IR,H-NMR and F-NMR spectra of the compound are illustrated in Figs. 80, -81 and -82 respectively.

Result from elemental analysis

| Calculatedvalues of components(%) | Observed values of components(%) |
|---|---|
| C:78.65<br>H: 7.61<br>F: 4.02<br>N: 2.96 | C:78.71<br>H: 7.60<br>F: 4.00<br>N: 2.93 |

Example 48

Preparation of compound No. 2a-15 in the Table 2a

1) 30.0 g of p-n-hexyliodobenzene and 10.5 g of 3-methyl-1-butyne-3-ol were allowed to react overnight within 200 ml of triethylamine in the presence of 330 mg of dichlorobistriphenylphosphinepalladium and 80 mg of cuprous iodide under nitrogen atmosphere at room temperature. Upon completion of reaction, the reactant caused triethylamine to be removed for subsequent hexane extraction. Hexane layer produced thereby was washed with 1 N hydrochloric acid, then with water for subsequent remaval of hexane. 25.4 g of oily compound (y) represented by the following formula was thus yielded.

$n-C_6H_{13}-$ 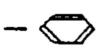 $-C\equiv C-C(CH_3)_2-OH$ (y)

2) 800 ml of toluene, upon dissolved therein 25.4 g of compound(y) obtained under 1) were added with 12.5 g of sodium hydroxide in powder form for subsequent heating reflux for 1 hr. The solution upon cooled and water washing caused toluene to be removed. Through extration of black colored oil obtained thereby using methanol followed by removal of methanol, 15.1 g of oily p-n-hexylphenylacetylene was thus obtained.

3) 2.0 g of 2-p-hexyloxy-m-fluorophenyl-5-bromopyridine obtained under 1) of the Example 46 and 1.4 g of p-n-hexylphenylacetylene obtained under 2) were subjected to reflux for 5 hrs. within 50 ml of triethylamine in the presence of 122 mg of dichlorobistriphenylphosphinepalladium, 31 mg of cuprous iodide and 160 mg of triphenylphosphine as catalysts under nitrogen atmosphere. The solution, upon cooled, caused triethylamine to be removed for subsequent toluene extraction. Toluene layer produced thereby was washed with 1 N hydrochloric acid,. and then with water for subsequent purification through a silicagel-column. Through recrystallization repeated twice of the solid matter obtained using ethanol, 1.6 g of compound No. 2a-15 under Table 2a as the compound proposed in the present invention were finally yielded. IR, H-NMR and F-NMR spectra of the compound are illustrated in Figs. 83, -84 and -85 respectively.

Result from elemental analysis

| Calculatedvalues of components(%) | Observed values of components(%) |
|---|---|
| C:83.22<br>H: 8.05<br>F: 4.25<br>N: 3.13 | C:83.20<br>H: 8.07<br>F: 4.20<br>N: 3.20 |

Example 49

Preparation of compound No. 2a-9 the Table 2a

1) 120 ml of Grignard reagent solution in dry ether prepared from 15.0 g of p-n-hexyloxyiodobenzene and magnesium, and 120 ml of 14.0 g 2,5-dibromopyridine solution in dry tetrahydrofuran were allowed to react for 5 hrs. in the presence of 357 mg of dichlorobisdiphenylphosphinobutanepalladium under nitrogen atmosphere at room temperature. The on-going reaction was quenched with water. The reactant mixture, upon completion ether extraction, was washed with 1 N hydrochloric acid, then with water for subsequent remaval of ether. Through recrystallization of solid matter obtained using methanol, 8.9 g of 2-p-hexyloxyphenyl-5-bromopyridine was thus yielded.

2) 1.9 g of 2-p-hexyloxyphenyl-5-bromopyridine obtained under 1) and 1.6 g of p-n-hexyloxy-m-fluorophenylacetylene were subjected to reflux for 5 hrs. within 50 ml of triethylamine in the presence of 122 mg of dichlorobistriphenylphosphinepalladium, 31 mg of cuprous iodide and 160 mg of triphenylphosphine under nitrogen atmosphere. The solution, upon cooled, caused triethylamine to be removed for subsequent toluene extraction. Toluene layer produced thereby was washed with 1 N hydrochloric acid, and then with water for subsequent purification through a silicagel-column. Through recrystallization repeated twice of the solid matter obtained, 1.5g of compound No. 2a-9 under Table 2a as the compound represented in the present invention was finally yielded. IR,H-NMR and F-NMR spectra of the compound are illustrated in Figs. 86, -87 and -88 respectively.

Result from elemental analysis

| Calculatedvalues of components(%) | Observed values of components(%) |
|---|---|
| C:78.65<br>H: 7.61<br>F: 4.02 | C:78.55<br>H: 7.53<br>F: 4.00 |

Example 50

Preparation of compound No. 2b-9 in the Table 2b

20 ml of ethanol containing 500 mg of compound No. 2a-9 under Table 2a obtained in the Example 49 suspended therein were added with 100 mg of 5% palladium-carbon and subjected to hydrogenation under hydrogen atmosphere of atmospheric pressure at reflux temperature. Upon comfirmation that hydrogen absorption became found no longer taking place, the solution caused catalyst to be removed through filtration for subsequent removal of ethanol. Through purification of the solid matter obtained through a silicagel-column followed by recrystallization repeated twice using ethanol 350 mg of compound No. 2b-9 under Table 2b as the compound proposed in the present invention were finally yielded. IR-, H-NMR and F-NMR spectra of the compound are illustrated in Figs. 89, -90 and -91 respectively.

Result from elemental analysis

| Calculatedvalues of components(%) | Observed values of components(%) |
|---|---|
| C:77.99<br>H: 8.39<br>F: 3.98<br>N: 2.94 | C:77.70<br>H: 8.45<br>F: 4.01<br>N: 3.01 |

Example 51

Preparation of compound Nc. 2c-12 in the Table 2c

2.5 g of 3-fluoro-4-hexyloxy-4′-iodobiphenyl obtained under 3) of the Example 40 and 1.7 g of p-n-hexyloxy-m-fluorophenylacetylene were allowed to react for 5 hrs. within 100 ml of triethylamine in the presence of 40 mg dichlorobistriphenylphosphinepalladium and 10 mg of cuprous iodide as catalysts under nitrogen atmosphere at room temperature. Upon completion of reactive process, the reactant caused triethylamine to be removed for subsequent toluene extraction. Toluene layer was washed with 1 N hydrochloric acid, and then with water for subsequent purification through a silicagel-column. Through recrystallization repeated twice of the solid matter obtained using ethanol, 2.0 g of compound No. 2c-12 under Table 2c as the compound proposed in the present invention were finally yielded. IR-, H-NMR and F-NMR spectra of the compound are illustrated in Figs. 92, -93 and -94 respectively.

Result from elemental analysis

| Calculatedvalues of components(%) | Observed values of components(%) |
|---|---|
| C:78.37<br>H: 7.35<br>F: 7.76 | C:78.22<br>H: 7.33<br>F: 7.82 |

Example 52

Preparation of compound No. 2c-6 in the Table 2c

2.5 g of 3-fluoro-4-n-hexyloxy-4′-iodobiphenyl obtained under 3) of the Example 40 and 1.5g of p-n-hexyloxyphenylacetylene were allowed to react for 5 hrs. within 100 ml of triethylamine in the presence of 40 mg of dichlorobistriphenylphosphinepalladium and 10 mg of cuprous iodide under nitrogen atmosphere at room temperature. Upon completion of reaction, the reactant caused triethylamine to be removed for subsequent toluene extraction.

Toluene layer produced thereby was washed with 1 N hydrochloric acid, and then with water for subsequent purification through a silicagel-column. Through recrystallization repeated twice of the solid matter obtained using ethanol, 1.8 g of compound No. 2c-6 under Table 2c as the compound proposed in the present invention was finally yielded. IR-, H-NMR- and F-NMR spectra of the compound are illustrated in Figs. 95, -96 and -97 respectively.

Result from elemental analysis

| Calculatedvalues of components(%) | Observed values of components(%) |
|---|---|
| C:81.36<br>H: 7.84<br>F: 4.03 | C:81.53<br>H: 7.80<br>F: 4.00 |

Example 53

Preparation of compound No. 2c-15 in the Table 2c

2.5 g of 3-fluoro-4-n-hexyloxy-4′-iodobiphenyl obtained under 3) of the Example 40 and 1.4 g of p-n-hexylphenylacetylene obtained under 1) and 2) of the Example 48 were allowed to react for 5 hrs. within 100 ml of triethylamine in the presence of 40 mg of dichlorobistriphenylphosphinepalladium and 10 mg of cuprous iodide under nitrogen atmosphere at room temperature. Upon completion of reaction, the reactant caused triethylamine to be removed for subsequent toluene extraction. Toluene layer produced thereby was washed with 1 N hydrochloric acid, and then with water for subsequent purification through a silicagel-column. Through recrystallization repeated twice of the solid matter obtained using ethanol, 1.8 g of compound No. 2c-15 under Table 2c as the compound proposed in the present invention was finally yielded. IR, H-NMR and F-NMR spectra of the compound are illustrated in Figs. 98, -99 and -100 respectively.

Result from elemental analysis

| Calculatedvalues of components(%) | Observed values of components(%) |
|---|---|
| C:84.21<br>H: 8.11<br>F: 4.17 | C:84.20<br>H: 8.07<br>F: 4.20 |

Example 54

Preparation of compound No. 2c-9 in the Table 2c

1) 50 ml of dimethylsulfoxide with 5.0 g of 4-hydroxy-4′-bromobiphenyl dissolved therein were added with sodium hydroxide solution in water (2.4 g/20 ml) and 4.0 g of n-hexylbromide for subsequent stirring kept for 3 days at room temperature. The reactant mixture was subjected to hexane extraction followed by water washing repeated thrice. Through removal of hexane, 5.0g of solid 4-n-hexyloxy-4′-bromobiphenyl were thus yielded.

2) 1.5g of 4-n-hexyloxy-4′-bromobiphenyl obtained under 1) and 1.2 g of p-n-hexyloxy-m-fluorophenylacetylene were subjected to heating reflux for 5 hrs. within 100 ml of triethylamine in the presence of 40 mg of dichlorobistriphenylphosphinepalladium, 10 mg of cuprous iodide and 80 mg of triphenylphosphine were subjected to reflux under nitrogen atmosphere. Upon completion of reaction, the reactant caused triethylamine to be removed for subsequent toluene extraction. Toluene layer produced thereby was washed with 1 N hydrochloric acid, and then with water for subsequent purification through a silicagel-column. Through recrystallization repeated twice of the solid matter obtained using ethanol, 1.7 g of compound No. 2c-9 under Table 2c as the compound proposed in the present invention was finally yielded. IR, H-NMR and F-NMR spectra of the compound are illustrated in Figs. 101, -102 and -103 respectively.

Result from elemental analysis :

| Calculatedvalues of components(%) | Observed values of components(%) |
|---|---|
| C:81.32<br>H: 7.90<br>F: 4.02 | C:81.53<br>H: 7.80<br>F: 4.00 |

Example 55

Preparation of compound No. 2d-12 in the Table 2d

500 mg of compound No. 2c-12 under Table 2c obtained in the Example 51 were suspended in 20 ml of ethanol and then they were added with 100 mg of 5% palladium-carbon and subjected to hydrogenation under hydrogen atmosphere of atmospheric pressure at reflux temperature. Upon confirmation that hydrogen absorption became found no longer taking place, the solution caused catalyst to be removed for subsequent removal of ethanol. Through purification of the solid matter obtained through a silicagel-column followed by recrystallization repeated twice using ethanol, 350 mg of compound No. 2d-12 under Table 2d as the compound proposed in the present invention was finally yielded. IR, H-NMR and F-NMR spectra of the compound are illustrated in Figs. 104, -105 and -106 respectively.

Result from elemental analysis

| Calculatedvalues of components(%) | Observed values of components(%) |
|---|---|
| C:77.73<br>H: 8.10<br>F: 7.69 | C:77.70<br>H: 8.05<br>F: 7.72 |

Example 56

Preparation of compound No. 2e-12 in the Table 2e

1) 100 ml of Grignard reagent solution in dry ether prepared from 11.0 g of p-n-hexyloxy-m-fluoroiodobenzene and magnesium, and 100ml of 7.6 g 3,6-dichloropyridazine solution in dry tetrahydrofuran were allowed to react for 5 hrs. in the presence of 250 mg of dichlorobisdiphenylphosphinobutanepalladium as catalyst under nitrogen atmosphere at room temperature. The on-going reaction was quenched with water for subsequent ether extraction. The reactant was washed with 1 N hydrochloric acid, then with water and caused ether to be removed. Through recrystallization of the solid matter obtained using methanol, 7.3 g of 3-p-n-hexyloxy-m-fluorophenyl-6-chloropyridazine was thus yielded.

2) 1.0 g of 3-p-n-hexyloxy-m-fluorophenyl-6-chloropyridazine and 850 mg of p-n-hexyloxy-m-fluorophenylacetylene were subjected to reflux for 5 hrs. within 50 ml of triethylamine in the presence of 24 mg of dichlorobistriphenylphosphinepalladium, 6 mg of cuprous iodide and 168 mg of triphenylphosphine as catalysts under nitrogen atmosphere. The reactant, upon cooled, caused triethylamine to be removed for subsequent toluene extraction.

Toluene layer produced thereby was washed with 1 N hydrochloric acid, and then with water for subsequent purification through a silicagel-column. Through recrystallization repeated twice of the solid matter obtained using ethanol, 920 mg of compound No. 2e-12 under Talbe 2e as the compound proposed in the present invention was finally yielded. IR, H-NMR and F-NMR spectra of the compound are illustrated in Figs. 107, -108 and -109 respectively

Result from elemental analysis

| Calculatedvalues of components(%) | Observed values of components(%) |
|---|---|
| C:73.17<br>H: 6.91<br>F: 7.72<br>N: 5.69 | C:73.30<br>H: 7.00<br>F: 7.82<br>N: 5.59 |

Example 57

Preparation of compound No. 2f-12 in the Table 2f

500 mg of compound No. 2e-12 under Table 2e obtained in the Example 56 was suspended in 20 ml of ethanol, then they were added with 100 mg of 5% palladium-carbon and then subjected to hydrogenation under hydrogen atmosphere of atomospheic pressure at reflux temperature. Upon confirmation that hydrogen absorption became found no longer taking place, the reactant caused catalyst through filtration and also ethanol to be removed. Through recrystallization repeated twice using ethanol, 300 mg of compoud No. 2f-12 under Table 2f as the compound proposed in the present invention was finally yielded. IR-, H-NMR and F-NMR spectra of the compound are illustrated in Figs. 110, -111 and -112 respectively.

Result from elemental analysis

| Calculatedvalues of components(%) | Observed values of components(%) |
|---|---|
| C:72.58<br>H: 7.66<br>F: 7.66<br>N: 5.65 | C:72.39<br>H: 7.63<br>F: 7.60<br>N: 5.72 |

Example 58

Preparation of compound No. 2g-11 in the Table 2g

700 mg of the compound yielded under 3) of the Example 40 and 500 mg of 1-decyne were allowed to react overnight within 10 ml of triethylamine in the presence of 12 mg of dichlorobistriphenylphosphinepalladium and 3 mg of cuprous iodide as catalysts under nitrogen atmosphere at room temperature. Upon completion of reaction, the reactant caused triethylamine to be removed for subsequent hexane extraction. The hexane layer produced thereby was washed with 1 N hydrochloric acid, then with water and subjected to purification through a silicagel-column. Through recrystallization repeated twice of the solid matter developed thereby using methanol 400 mg of compound No. 2g-11 under Table 2g proposed in the present invention was yielded. IR, H-NMR and F-NMR spectra of said compound are illustrated in Figs. 113, 114 and 115 respectively.

Result from elemental analysis:

| Calculated values of components(%) | Observed values of components(%) |
|---|---|
| C: 82.31<br>H: 9.31<br>F: 4.65 | C: 82.11<br>H: 9.46<br>F: 4.75 |

Example 59

Preparation of compound No. 2h-21 in the Table 2h

270 mg of compound No. 2g-11 under Table 2g yielded in the Example 58 are made solved in 15 ml of

ethanol. 100 mg of 5% palladium-carbon were added thereto, whereupon the solution was subjected to hydrogenation under hydrogen atmosphere of atmospheric pressure at room temperature. Upon confirmation that hydrogen became found no longer absorbed, the reactant caused catalysts and then ethanol to be removed. Through purification of solid matter yielded through a silicagel-column, 80 mg of compound No. 2h-21 under Table 2h proposed in the present invention was yielded. IR H-MNR and F-NMR spectra of the compound are illustrated in Figs. 116, 117, and 118 respectively.

Result from elemental analysis:

| Calculated values of components(%) | Observed values of components(%) |
|---|---|
| C: 81.50<br>H: 10.02<br>F: 4.60 | C: 81.37<br>H: 10.22<br>F: 4.53 |

Example 60

Preparation of compound No. 2i-2 in the Table 2i

1) 20 ml of Grignard reagent solution in dry ether prepared from 3.2 g of p-n-heptyloxy-m-fluoroiodobenzene and magnesium, and 20 ml of 1.5g 2,5-dibromopyridine in dry tetrahydrofuran were allowed to react for 5 hrs. in the presence of 57 mg of dichlorobisdiphenylphosphinobutanepalladium as catalyst under nitrogen atmosphere at room temperature. The on-going reaction was quenched with water for subsequent ether extraction of solution. The solution resulted was washed with 1 N hydrochloric acid and then with water for subsequent removal of ether. Through recrystallization of the solid matter obtained using methanol, 1.4 g of 2-p-n-heptyloxy-m-fluorophenyl-5-bromopyridine was finally yielded.

2) 1.4 g of 2-p-n-heptyloxy-m-fluorophenyl-5-bromopyridine obtained under 1) and 540 mg of 1-octyne were subjected to reflux for 5 hrs. within 50 ml of triethylamine in the presence of 40 mg of dichlorobistriphenylphosphinepalladium, 10 mg of cuprous iodide and 60 mg of triphenylphosphine as catalysts under nitrogen atmosphere. The reactant, upon cooled, caused triethylamine to be removed for subsequent hexane extraction. Hexane layer was washed with hydrochloric acid, and then with water for subsequent purification through a silicagle-column. Through recrystallization repeated twice of the solid matter obtained using ethanol, 1.1g of compound of No. 2i-2 under Talbe 2i as the compound proposed in the present invention was finally yielded.

Result from elemental analysis

| Calculated values of components(%) | Observed values of components(%) |
|---|---|
| C:78.99<br>H: 8.61<br>F: 4.81<br>N: 3.54 | C:79.00<br>H: 8.55<br>F: 4.80<br>N: 3.60 |

Example 61

Preparation of compound No. 2i-3 in the Table 2i

1.5 g of 2-p-n-octyloxy-m-fluorophenyl-5-bromopyridine obtained using p-n-octyloxy-m-fluoroiodobenzene instead of p-n- heptyloxy-m-fluoroiodobenzene in the same way as in 1) of the Example 60 and 550 mg of 1-octyne were subjected to heating reflux for 5 hrs. within 50 ml of triethylamine in the presence of 40 mg of dichlorobistriphynylphosphinepalladium, 10 mg of cuprous iodide and 60 mg of triphenylphosphine as catalysts under nitrogen atmosphere. The solution, upon cooled, caused triethylamine to be removed for subsequent hexane extraction. Hexane layer produced thereby was washed with 1 N hydrochloric acid, and then with water for subsequent purification through a silicagel-column chromatography. Through recrystallization repeated twice of the solid matter obtained using ethanol, 1.2 g of compound No. 2i-3 under Table 2i as the compund proposed in the present invention were finally yielded.

Result from elemental analysis

| Calculated values of components(%) | Observed values of components(%) |
|---|---|
| C:79.22<br>H: 8.80<br>F: 4.65<br>N: 3.42 | C:79.16<br>H: 8.85<br>F: 4.55<br>N: 3.50 |

Example 62

Preparation of compound No. 2i-8 in the Table 2i

1) 80 ml of dimethylsulfoxide, into which 22.0 g of o-fluoro-p-iodophenol had been dissolved, was added with sodium hydroxide solution in water (sodium hydroxide 7.4 g and water 30 ml) and stirred into a homogeneous state. The mixture was added with 21.2 g of n-octylbromide and stirred for 4 days at room temperature. The reactant was poured into ice water and then subjected to extraction using hexane. The resulted hexane layer was washed with water thrice and hexane was removed therefrom, then 25.0 g of oily p-n-octyloxy-m-fluoroiodobenzene was finally yielded.

2) 20 ml of Grignard reaent solution in dry ether prepared from 3.3 g of p-n-octyl-m-fluoroiodobenzene and magnesium, and 20 ml of 1.5 g 2,5-dibromopyridine solution in dry tetrahydrofuran were allowed to react for 5hr. in the presence of 57 mg of dichlorobisdiphenylphosphinobutanepalladium under nitrogen atmosphere at room temperature. The on-going reaction was quenched with water. The reactant mixture, upon completion ether extraction, was washed with 1N hydrochloric acid, then with water for subsequent removal of ether. Through recrystalization of solid matter obtained using methanol, 1.5 g of 2-p-octyloxy-m-fluorophenyl-5-bromopyridine was thus yielded.

3) 1.5 g of 2-p-n-octyloxy-m-fluorophenyl-5-bromopyridine yielded under 2), and 680 mg of 1-nonyne were subjected to reflux for 5 hrs. within 25 ml of triethylamine in the presence of 25 mg of dichlorobistriphenylphosphinepalladium, 6 mg of cuprous-iodide and 60 mg of triphenylphosphin as catalysts under nitrogen atmosphere. The reactant produced, upon cooled, caused triethylamine to be removed for subsequent hexane extraction. The hexane layer was washed with 1 N hydrochloric acid, and then with water for subsequent purification through a silicagel-column. Through recrystallization repeated twice of solid matter produced using ethanol 520mg of compound No. 2i-8 under Table 2i proposed in the present invention was thus yielded. IR, H-NMR and F-NMR spectra of the compound are illustratecl in 119, 120 and 121 respectively.

Result from elemental analysis

| Calculated values of components(%) | Observed values of components(%) |
|---|---|
| C:79.38<br>H: 9.04<br>F: 4.49<br>N: 3.31 | C:79.16<br>H: 9.15<br>F: 4.55<br>N: 3.30 |

Example 63

Preparation of compound No. 2i-36 in the Table 2i

1) 50 ml of Grignard reagent solution in dry ether prepared form 6.0 g of p-n-heptyloxyiodobenzene and magnesium, and 50 ml of 3.0 g 2,5-dibromopyridine solution in dry tetrahydrofuran were allowed to react for 5 hrs. in the presence of 115 mg of dichlorobisdiphenylphosphinobutanepalladium as catalysts under nitrogen atmosphere at room temperature. The reactant mixture, after when the on-going reaction was quenched with water, was subjected to ether extraction followed by washing with 1 N hydrochloric acid and then with water and finally caused ether to be removed by distillation. Through recrystallization of the solid matter obtained using methanol, 2.0 g of 2-p-heptyloxyphenyl-5-bromopyridine was yielded.
2) 1.3 g of 2-p-heptyloxyphenyl-5-bromopyridine obtained under 1) and 540 mg of 1-octyne were subjected to reflux for 5 hrs. within 50 ml of triethlylamine in the presence of 40 mg of dichlorobistriphenylphosphinepalladium, 10 mg of cuprous iodide and 60 mg of triphenylphosphine as catalysts under nitrogen atmosphere, The solution, upon cooled, caused triethylamine to be removed for subsequent hexane extraction. Hexane layer produced thereby was washed with 1 N hydrochloric acid, then with water for subsequent purification through a silicagel-column. Through recrystallization repeated twice of the solid matter obtained using ethanol, 0.9 g of compound No. 2i-36 under Table 2i as the compuond proposed in the present invention were finally yielded. IR and H-NMR spectra of the compund are illustrated in Figs. 122, and 123 respectively.
Result from elemental analysis

| Calculated values of components(%) | Observed values of components(%) |
|---|---|
| C:82.76<br>H: 9.28<br>N: 3.71 | C:82.70<br>H: 9.38<br>N: 3.60 |

Example 64

Preparation of compound No. 2j-18 in the Table 2j

1) 30 ml of Crignard reagent solution in dry ether prepared from 2.0 g of p-n-octyloxy-m-fluoroiodobenzene and magnesium, and 50 ml of 2.5 g 2-n-nonyl-5-iodopyridine solution in dry tetrahydrofuran obtained using 1-nonyne instead of 1 decyne in the same way as in 1) to 3) of the Example 1 were subjected to heating reflux for 8 hrs. in the presence of 45 mg of dichlorobisdiphenylphosphinbutanepalladium as catalst under nitrogen atmosphere. On-going reaction of the solution was, upon cooled, quenched with water. The resulted reactant was subjected to ether extraction followed by washing with 1 N hydrochloric acid, and then with water, whereupon it caused ether to be removed by evaporation. Through purification of the solid matter obtained through a silicagel-column followed by recrystallization

repeated twice using methanol 0.7 g of compound No. 2i-18 under Table 2i as the compound proposed in the present invention were finally yielded. IR, H-NMR and F-NMR spectra of the compound are illustrated in Figs. 124, -125 and -126 respectively.

| Calculated values of components(%) | Observed values of components(%) |
|---|---|
| C: 78.69<br>H: 9.84<br>F: 3.28<br>N: 4.45 | C: 78.60<br>H: 9.90<br>F: 3.30<br>N: 4.44 |

Example 65

Preparation of compound No. 2k-8 in the Table 2k

1) 80 ml of dimethylsulfoxide, into which 22.0 g of o-fluoro-p-iodophenol had been dissolved, was added with sodium hydroxide solution in water (sodium hydroyide 7.4 g and water 30 ml) and stirred into a homogeneous state. The mixture was added. with 21.2 g of n-octylbromide and stirred for 4 days at room temperature. The reactant was poured into ice water and then subjected to extraction using hexane. The resulted hexane layer was washed water thrice and hexane was removed therefrom, then 25.0 g of oily p-n-octhyoxy-m- fluoroiodobenzene was finally yielded.

2) 20 ml of Grignard reagent solution in dry ether made of 3.3 g of p-n-octyloxy-m-fluoroiodobenzene and magnesium, and 20 ml of dry tetrahydrofuran solution with 1.5 g of 3,6-dichloropyridadine dissolved therein were allowed to react for 5 hrs. in the presence of 57 mg of dichlorobisdiphenylphosphinobutanepalladium as catalyst under nitrogen atmosphere at room temperature. The on-going reaction was quenched using water. The reactant mixture was subjected to ether extraction, washed with 1 N hydrochloric acid, then with water and caused ether to be removed by evaporation. Through recrystallization of solid substance produced thereby using methanol, 0.5 g of 3-p-n-octyloxy-m-fluorolphenyl-6-cholopyridazine was yielded.

3) 0.5 g of 3-p-n-octyloxy-m-fluorolphenyl-6-cholopyridazine yielded under 1) and 220 mg of 1-nonyne were subjected to reflux process for 5 hrs. within 25 ml of triethylamine in the presence of 13 mg of dichlorobistriphenylphosphinepalladium and 5 mg of cuprous iodide as catalysts under nitrogen atmosphere. The reactant was allowed to be cooled, and caused triethylamine to be removed for subsequent hexane extraction. The hexane layer produced thereby was washed with 1 N hydrochloric acid, then with water for subsequent purification through a silicagel-column. The solid substance developed thereby was subjected to recrystallization repeated twice using methanol. 130 mg of compound No. 2k-8 under the Table 2k proposed in the present invention were thus yielded. IR, H-NMR and F-NMR spectra of said compound are illustrated in Figs. 127, - 128 and -129 respectively.

Result from elemental analysis:

| Calculated values of components(%) | Observed values of components(%) |
|---|---|
| C: 76.38<br>H: 8.78<br>F: 4.47<br>N: 6.60 | C: 76.52<br>H: 8.66<br>F: 4.52<br>N: 6.58 |

Example 66

Preparation of compound No. 21-18 in the Table 21

100 mg of compound No. 2k-8 under Table 2k obtained in the Example 65 was suspended in 20 ml of ethanol, then they were added with 10 mg of 5% palladium-carbon and then subjected to hydrogenation under hydrogen atmosphere of atmospheric pressure at reflux temperature. Upon confirmation that hydrogen absorption became found no longer taking place, the reactant caused catalyst to be removed. Through purification of the solid matter obtained through a silicagel-column followed by recrystallization repeated twice using ethanol 70 mg of compound No. 21-18 under Table 21 as the compound proposed in the present invention were finally yielded. IR, H-NMR and F-NMR spectra of the compound are illustrated in Figs. 130, -131 and -132 respectively.

Result from elemental analysis

| Calculated values of components(%) | Observed values of components(%) |
|---|---|
| C:75.70<br>H: 9.58<br>F: 4.44<br>N: 6.54 | C:75.70<br>H: 9.45<br>F: 4.41<br>N: 6.62 |

Example 67

Preparation of compound No. 2m-26 in the Table 2m

1) 130 ml of thionyl chloride containing therein 57.0 g of p-iodobenzoic acid were subjected to reflux for 8 hrs. under heated condition, so that p-iodobenzoyl Cholride was yielded. Upon completion of removal of excess thionyl chloride it was made dissolved in dry toluene of 400 ml for subsequent cooling at temperature below 10 ° C. Ammonium gas having temperature below 20 ° C was allowed to blown onto the surface of toluene solution being kept stirred. The precipitate produced thereby was subjected to filtration followed by drying. Through washing with 1 N sodium hydroxide solution in water, then with water followed by drying and subsequent recrystallization using methanol, 50.6 of p-iodobenzamide was thus yielded.

2) 50.0 g of p-iodobenzamide were made suspended within 300ml of dry dioxane so as to be cooled down blelow 10 ° C.

51.0 g of trifluoroacetic anhydride was allowed to drop thereon at temperature below 20 ° C. Upon completion of dropping process, the temperture was restored to room temperature for subsequent stirrer being kept overnight. The reactant with reddish transparent appearance was poured into ice water. The precipitate produced thereby was subjected to filtration, and drying followed by recrystallization using hexane, whereupon 33.2 g of p- cyanoiodobenzene was yielded.

3) Approxmatly 12 g of dry hydrogen chloride were allowed to be blown into 400 ml of dry ethanol containing 33.2 g of p-cyanoiodobenzene being stirred under the temperature below 10 ° C. The resultant solution was kept stirred for 3 days at room temperature. It caused solvent to be removed and was added slowly with 500ml of dry ethanol containing approxmatly 30 g of dry ammonium under cooled condition by ice so as to be kept stirred for another 3 days at room temperature. Through subsequent removal of ethanol and ammonia followed by filration of precipitate caused by addition of dry ether, 20.8 g of solid p-iodobenzamidinhydrochloride salt was yielded.

4) Onto 800 ml of dry dimethylformamide containing therein 25.0 g of sodium hydride (60% content) 100.0 g of n-decylalcohol were allowed to drop at such a rate that hydrogen evolved not so vigorously under temperature of 70 to 80 ° C. Upon confirmation that the evolution of hydrogen was completed, 185.0 g of bromoacetoaldehydediethylacetal were allowed to drop thereon for 1 hr. with the temperature of solution restored to room temperature. The solution was kept stirred for subsequent. 3 days. It was, upon completion of reaction, poured into ice water for subsequent toluene extraction. The toluene layer produced thereby was washed with water, caused toluene to be removed for subsequent distillation under reduced pressure. 58.3 g of n-decyloxyacetoaldehydediethylacetal (b.p.: 93 to 94 ° C/0.4 mmHg)

was thus yielded.

5) 82.5 g of dry diemethylformamide was dissolved into 350 ml of dry 1,2-dichloroethane so as to be cooled down below 5 °C 56.0 g of trichloromethylchloroformate were allowed to drop onto said solution below 10 °C. The solution was, upon completion of dropping process, further kept stirred for 30 min. n-decyloxyacetoaldehydediethylacetal solution in 1,2-dichloroethane (n-decyloxyacetoaldehydediethylacetal 58.0 g, 1,2-dichloroethane 75 ml) separately prepared was allowed to drop onto the said solution. Upon completion of dropping process the solution was subjected to reflux for 3 hrs. With the temperature restored to room temperature the solution was added with sodium hydroxide solution in water (sodium hydroxide 40.0 g, water 500 ml) and kept stirred for 3 hrs. 30 to 50 °C. The organic layer produced thereby was washed with water, and caused 1,2-dichloroethan to be removed for subsequent distillation under reduced pressure. 16.8 g of β-n-decyloxy-α-dimethlaminoacrolein was thus yielded.

6) A solution made of 300 ml of dry ethanol and 2.65 g of sodium metal was added with 10.6 g of p-iodobenzamidinehydrochloride yielded under 3) of example 42 and 9.0 g of β-n-decyloxy-dimethylaminoacrolein yielded under 5) and then subjected to reflux for 18 hrs. Upon completion of reaction, the solution caused solvent to be removed for subsequent toluene extraction. The toluene layer produced thereby was washed with 1 N hydrochloric acid solution in water, then with water, and caused toluene to be removed for subsequent recrystallization using methanol. 5.1 g of 2-p-iodophenyl-5-decyloxypyrimidine was thus yielded.

7) 2.0 g of 2-p-iodophenyl-5-decyloxypyrimidine yielded under 6) and 1.0 g of 1-octyne were allowed to react overnight within 40 ml of triethylamine in the presence of 48 mg of bis-triphenylphosphinepalladiumdichloride and 12 mg of cuprous iodide as catalysts under nitrogen atmosphere at room temperature. Upon completion of reaction, the solution caused triethylamine to be removed for subsequent hexane extraction. The hexane layer was washed with 1 N hydrochloric acid solution in water, then with water and subjected to treatment through a silicagel-column. Through recrystallization repeated twice using ethanol 1.2 g of white crystalline compound No. 2m-26 under the Table 2m proposed in the present invention was yielded. IR and H-NMR spectra are illustrated in Figs. 133 and -134 respectively.

Result from elemental analysis:

| Calculated values of components(%) | Observed values of components(%) |
|---|---|
| C: 80.00<br>H: 9.52<br>N: 6.67 | C: 79.83<br>H: 9.65<br>N: 6.81 |

Example 68

Preparation of compound No. 3a-2 in the Table 3a

1) 15.5 g of n-decylalcohol was allowed to drop onto 250 ml of dry dimethylformamide containing 3.8 g of sodium hydride(60% content) at such a rate that hydrogen evolved not so vigorousl 70 to 80 °C. Where the evolution of hydrogen was completed, the solution was allowed to be cooled down to the room temperature, added with 15.4 g of 2-chloro-5-nitropyridine and then subjected to reflux for 2 hrs. Upon completion of reaction, the solution was poured into ice water. The resulted precipitate was filtrated and dried. Of the solid matter resulted such part which is soluble to hexane was made recrystallized using methanol, whereupon 14.4 g of 2-n-decyloxy-5-nitropyridine were thus obtained.

2) 150 ml of ethanol containing 14.4 g of 2-n-decyloxy-5-nitropyridine obtained under 1) were added with 1.0 g of 5% palladium carbon and subjected to reduction process under hydrogen atmosphere of atmospheric pressure. Upon completion of reaction, the solution caused catalyst to be removed through filtration and then ethanol to be removed, whereupon 12.1 g of oily 2-n-decyloxy-5-aminopyridine was finally obtained.

3) 450 ml of water containing 50 g of 36% hydrochloric acid was added with 12.1 g of 2-n-decyloxy-5-aminopyridine and allowed to be cooled down to below 5 °C. Onto the solution sodium nitrite solution in water (sodium nitrite 3.3 g and water 20 ml) was allowed to drop below 5 °C. Upon completion of

dropping process, the solution was stirred for 1 hr. and then added with potassium iodide solution in water (potassium iodide 40 g, water 40 ml) so as to be restored up to the room temperature. The solution was then stirred until nitrogen was no longer evolved. Upon completion of reaction, the solution was subjected to hexane extraction; the hexan layer resulted was washed by water, then by sodium hydrogen sulfite solution in water and again by water; the solution was finally subjected to a treatment using a silicagel column, whereupon 9.4 g of oily 2-n-decyloxy-5-iodopyridine were thus obtained.

4) 3.0 g of 2-n-decyloxy-5-iodopyridine and 0.9 g of 3-methyl-1 butyne-3-ol were allowed to react overnight within 30 ml of triethylamine in the presence of 72 mg of bistriphenylphosphinepalladiumdichloride and 18 mg of cuprous iodide as catalysts under nitrogen atmosphere at room temperature. Upon completion of reaction the solution caused triethylamine to be removed and then subjected to hexane extraction. The resulted hexane layer was washed by 1 N hydrochloric acid and then by water. Through a silicagel-column treatment, it produced 1.9 g of oily compound (z) with the structure represented by the following formula.

$n-C_{10}H_{21}O-$ 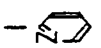 $-C\equiv C-C(CH_3)_2-OH$ (z)

5) 40 ml of dry toluene containing 1.9 g of compound (z) yielded under 4) were added with 0.7 g of sodium hydroxide in powder form and then subjected to reflux for 1 hr. Upon completion of cooling and washing the solution by water, the solution caused toluene to be removed, was dissolved in hexane and then subjected to a silicagel-column treatment. 0.95 g of 2-n-decyloxy-5-ethynylpyridine was thus yielded.

6) 0.45 g of 2-n-decyloxy-5-ethynylpyridine under 5) and 0.72 g of optically active p-(1-trifluoromethylheptyloxycarbonyl) iodobenzene (this compound was obtained in such a manner that p-iodobenzoic acid was transformed into p-iodobenzoyl acid chloride through thionyl chloride and then allowed to react with optically active 1-trifluoromethylheptanol) was allowed to react overnight within 20 ml of triethylamine in the presence of 24 mg of bistriphenylphosphinepalladiumdichloride and 6 mg of cuprous iodide as catalysts under nitrogen atmosphere at room temperature. Upon completion of reaction process, the solution caused triethylamine to be removed and was subjected to hexane extraction. The resulted hexane layer was washed by hydrochloric acid solution in water and then by water. It was then subjected to a silicagel-column treatment followed by recrystallization repeated twice using ethanol, resulting in 0.42 g of white crystalline compound as No. 3a-2 in the Table 3a proposed in the present invention. IR, H-NMR and F-NMR spectra are shown in Fig. 135, -136 and -137 respectively.

Result from elemental analysis:

| Calculated values of components(%) | Observed values of components(%) |
|---|---|
| C: 70.46<br>H: 7.71<br>N: 2.57<br>F: 10.46 | C: 70.22<br>H: 7.83<br>N: 2.66<br>F: 10.51 |

Example 69

Preparation of compound No. 3a-1 in the Table 3a

1) Onto dry dimethylformamide of 200 ml containing 2.0 g of sodium hydride (60% content), 9.2 g of optically active (s)-1-trifluoromethylheptanol was allowed to drop at room temperature in such a rate that hydrogen evolved not so vigorously, so that sodium salt of optically active (s)-1-trifluoromethylheptanol was yielded. Upon completion of dropping process, the solution was subjected to stir for 1 hr. followed by reflux for 2hrs., 12.4 g p-iodonitrobenzene being added thereto. The reactant was, upon cooled, poured into ice water for hexane extraction. The resulted hexane layer was washed with water, then with

1 N hydrochloric acid solution in water and again with water. It was subjected to a separation and purification using a silicagel-column to finally obtain 7.7 g of optically active, oily p-((s)-1-trifluoromethylheptyloxy) nitrobenzene.

2) 100 ml of ethanol containing 7.7 g of optically active p-((s)-1-trifluoromethylheptyloxy) nitrobenzene were added with 0.5 g of 5% Pd-C (5% palladium-carbon) as catalyst for the purpose of reduction, the solution being stirred under hydrogen atmosphere of atmospheric pressure at room temperature. The reduction continued until hydrogen became no longer found absorbed. The reactant caused catalyst to be removed through filtraion and ethanol to be removed through evaporation, whereupon 6.7 g of optically active, oily p-((s)-1-trifluoromethylheptyloxy) aniline was finally yielded.

3) 200 ml of water containing 25 g of 36% hydrochloric acid was added with 6.7 g of optically active p-((s)-1-trifluoromethylheptyloxy) aniline and allowed to be cooled down to below 5°C. A sodium nitrite solution in water (sodium nitrite 1.7 g, water 10 ml) prepared separately was allowed to drop thereon below 5 °C. Upon completion of dropping process the solution was kept stirred for 1 hr. and added with potassium iodide solution in water (potassium iodide 20 g, water 20 ml) with the temperature slowly restored up to the room temperature, the solution being kept stirred until the evolution of nitrogen was completed. Upon completion of reaction, the reactant was subjected to hexane extraction. The hexane layer produced thereby was washed with water, then with sodium hydrogen sulfite solution in water and again with water and then hexan was removed therefrom, whereupon 7.7 g of optically active, oily p-((s)-1-trifluoromethylheptyloxy) iodobenzene were finally yielded.

4) 0.50 g of 2-n-decyloxy-5-ethynylpyridine synthesized under 5) of the Example 68 and 0.75 g of optically active p-((s)-1-trifluoromethylheptyloxy) iodobenzene were allowed to react within 20 ml of triethylamine in the presence of 24 mg of bistriphenylphosphinepalladiumdichloride and 6 mg of cuprous iodide as catalysts under nitrogen atmosphere at room temperature. Upon completion of reaction process the reactant caused triethylamine to be removed for subsequent hexane extraction. The hexane layer produced thereby was washed with hydrochloric acid solution in water and then with water for subsequent separation and purification through a silicagel-column. 0.57 g of compound No. 3a-1 under the Table 3a was thus yielded. IR, H-NMR, and F-NMR spectra of said compound are illustrated in Figs, 138 ,-139, and -140 respectively.

Results from elemental analysis:

| Calculated values of components(%) | Observed values of components(%) |
|---|---|
| C: 71.95 | C: 72.10 |
| H: 8.12 | H: 8.01 |
| N: 2.71 | N: 2.63 |
| F: 11.03 | F: 10.95 |

Example 70

Preparation of compound No. 3a-12 in the Table 3a

1) Onto 200 ml of dry dimethylformamide containing 2.0 g of sodium hydride (60% content), 9,2 g of optically active (R)-1-trifluoromethylheptanol were allowed to drop at such a rate that hydrogen evolved not so vigorously so as to obtain sodium salt of optically active (R)-1-trifluoromethylheptanol . Upon completion of dropping process, the yielded solution was kept stirred for 1 hr, and then added with 8.0 g of 2-chloro-5-nitropyridine for subsequent reflux for 2 hrs. The solution was, upon cooled, poured into ice water for subsequent hexane extraction. The hexane layer produced thereby washed with water, then with hydrochloric acid solution in water and again with water for subsequent separation and purification through a silicagel-column. 11.9 g of optically active, oily 2-((R)-1′-trifluoromethylheptyloxy)-5-nitropyridine was thus yielded.

2) 150 ml of ethanol containing 11.9 g optically active 2-((R)-1′-trifluoromethylheptyloxy)-5-nitropyridine were added with 1.0 g of 5% Pd-C (5% palladium-carbon) as catalyst for subsequent reduction, being kept stirred under hydrogen atmosphere of atmospheric pressure at room temperature. Upon confirmation that hydrogen was found no longer absorbed, the solution caused catalyst to be removed through filtration and ethanol to be removed by evaporation, whereupon 10.5 g of optically active, oily 2-((R)-1'-trifluoromethylheptyloxy)-5-aminopyridine was finally yielded.

3) 500 ml of water containing 40 g of 36% hydrochloric acid therein were added with 10.5 g of optically active 2-((R)-1'-trifluoromethylheptyloxy)-5-aminopyridine so as to be cooled down to below 5 °C. Sodium nitrite solution in water (sodium nitrite 2.6 g, water 20ml) separately prepared was allowed to drop onto said solution below 5°C. Upon completion of dropping process, the solution was kept stirred for 1 hr. and then added with potassium iodide soution in water (potassium iodide 31 g, water 30 ml) with the temperature slowly restored to the room temperature, stirring being kept until the evolution of nitrogen was completed. Upon completion of reaction the solution was subjected to hexane extraction. The hexane layer produced thereby was washed with water, then with sodium hydrogen sulfite solution in water and again with water, whereupon 9.5 g of optically active, oily 2-((R)-1'-trifluoromethylheptyloxy)-5-iodopyridine was finally yielded.

4) 350 ml of dimethylsulfoxide containing 34.0 g of p-iodophenol therein were added with sodium hydroxide solution in water (sodium hydroxide 7.4 g, water 50 ml) and stirred to a homogeneous solution. It was then added with 32.4 g of n-decylbromide and kept stirred for 3 days at room temperature. The reactant was poured into 1 ℓ of ice water for the subsequent hexane extraction to be repeated thrice. The hexane layer produced thereby was washed by water and caused hexane to be removed so as to finally yield 46.6 g of oily p-n-decyloxy-iodobenzene.

5) 30 g of p-n-decyloxyiodobenzene and 8.4 g of 3-methyl-1-butyen-3-ol were allowed to react overnight within 200 ml of triethylamine in the presence of 320 mg of bistriphenylphosphinepalladiumdichloride and 80 mg of cuprous iodide as catalysts under nitrogen atmosphere at room temperature Upon completion of reactive process the solution caused triethylamine to be removed for subsequent hexane extraction. The hexane layer produced thereby was washed by 1 N hydrochloric acid solution in water, then with water and caused hexane to be removed so as to finally yield 22.8 g of solid compound with a structure represented by the following formula.

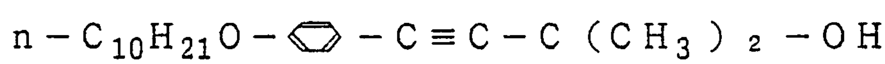

6) 400 ml of dry toluene containing 22.8 g of compound(yielded) under 5) were added with 9.9 g of sodium hydroxide in powder form and subjected to reflux for 1 hr. Upon completion of cooling process it was washed by water and then caused toluene to be removed. The black oil yielded thereby was subjected to methanol extraction, whereupon 13.2 g of oily p-n-decyloxyphenylacetylen was yielded after methanol had been removed therefrom.

7) 1.5 g of optically active 2-((R)-1'-trifluoromethylbutyloxy)-5-iodopyridine and 1.0 g of p-n-decyloxyphenylacetylene were allowed to react overnight within 50 ml of triethylamine in the presence of 32 mg of bistriphenylphosphinepalladiumdichloride and 8 mg of cuprous iodide as catalysts under nitrogen atmosphere at room temperature. Upon completion of reaction, the solution caused triethylamine to be removed for subsequent hexane extraction. The hexane layer produced thereby was washed with hydrochloric acid solution in water, then with water for the subsequent separation and purification through a silicagel-column, whereupon 0.63 g of oily compound No. 3a-12 in the Table 3a proposed in the present invention was finally yielded. IR, H-NMR and F-NMR spectra of the said compound are illustrated in Figs. 141, -142 and -143.

Results from elemental analysis:

| Calculated values of components(%) | Observed values of components(%) |
|---|---|
| C: 71.95<br>H: 8.12<br>N: 2.71<br>F: 11.03 | C: 71.66<br>H: 8.23<br>N: 2.91<br>F: 10.85 |

Example 71

Preparation of compound No. 3b-1 in the Table 3b

1) 17.6 g of n-decylalcohol were allowed to drop onto 400 ml of dry dimethylformamide containing therein 2.67 g of sodium hydride at such a rate that hydrogen evolved not so vigorously under 70 to 80 °C. Upon confirmation that the evolution of hydrogen was completed, the temperature of the solution was restored to room temperature, the solution was added with 25.0 g of 3,6-dichloropyridazine and subjected to reflux for 2 hrs. Upon completion of reaction, the solution was poured into ice water and precipitate produced thereby was subjected to filtration followed by drying. Through a silicagel-column treatment of such part which is soluble to hexane of the solid matter yielded, followed by recrystallization using methanol 19.4 g of 3-n-decyloxy-6-chloropyridadine was thus yielded.

2) 11.7 g of 3-n-decyloxy-6-chloropyridadine yielded under 1) and 5.5 g of 3-methyl-1-butyne-3-ol were subjected to reflux for 5 hrs. within 150 ml of triethylamine in the presence of 200 mg of bis-triphenylphosphinepalladiumdichloride, 50 mg of cuprous iodide and 300 mg of triphenylphosphine as catalysts under nitrogen atmosphere. Upon completion of reactive process the solution caused triethylamine to be removed for subsequent hexane extraction. The hexane layer produced thereby was washed with hydrochloric acid solution in water, then with water. Through subsequent removal of hexane, 12.3 g of black oil was thus yielded. The substance was made in use for the subsequent process without further purification.

3) 12.3 g of black oil yielded under 2) was dissolved in 450 ml of dry toluene. 3.3 g of sodium hydroxide m powder form were added to the solution for subsequent reflux for 1 hr. Upon completion of reaction, the reactant caused toluene to be removed and was added with ether. The ether layer developed thereby was washed with water for subsequent removal of ether. Through dissolving in hexane followed by purification through a silicagel-column, 3.4 g of white solid 3-n-decyloxy-6-ethynylpyridadine was yielded.

4) Onto dry dimethylformamide of 200 ml containing 2.0 g of sodium hydride (60% content), 9.2 g of optically active (s)-1-trifluoromethylheptanol were allowed to drop at room temperature in such a rate that hydrogen evolved not so vigorously, so that sodium salt of optically active (s)-1-trifluoromethylheptanol was yielded. Upon completion of dropping process, the solution was subjected to stir for 1 hr. followed by reflux for 2hrs., 12.4 g p-iodonitrobenzene being added thereto. The reactant was, upon cooled, poured into ice water for hexane extraction. The resulted hexane layer was washed with water, then with 1 N hydrochloric acid solution in water and again with water. It was subjected to a separation and purification using a silicagel-column to finally obtain 7.7 g of optically active, oily p-((s)-1-trifluoromethyl-heptyloxy) nitrobenzene.

5) 100 ml of ethanol containing 7.7 g of optically active p-((s)-1-trifluoromethylheptyloxy) nitrobenzene were added with 0.5 g of 5% Pd-C (5% palladium-carbon) as catalyst for the purpose of reduction, the solution being stirred under hydrogen atmosphere of atmospheric pressure at room temperature. The reduction continued until hydrogen became no longer found absorbed. The reactant caused catalyst to be removed through filtraion and ethanol to be removed through evaporation, whereupon 6.7 g of optically active, oily p-((s)-1-trifluoromethylheptyloxy) aniline was finally yielded.

6) 200 ml of water containing 25 g of 36% hydrochloric acid was added with 6.7 g of optically active p-(-(s)-1-trifluoromethylheptyloxy) aniline and allowed to be cooled down to below 5 °C. A sodium nitrite solution in water (sodium nitrite 1.7 g, water 10 ml) prepared separately was allowed to drop thereon below 5 °C. Upon completion of dropping process the solution was kept stirred for 1 hr. and added with potassium iodide solution in water (potassium iodide 20 g, water 2o ml) with the temperature slowly restored up to the room temperature, the solution being kept stirred until the evolution of nitrogen was completed. Upon completion of reaction, the reactant was subjected to hexane extraction. The hexane layer produced thereby was washed with water, then with sodium hydrogen sulfite solution in water and again with water and then hexan was removed therefrom, whereupon 7.7 g of optically active, oily p-((s)-1-trifluoromethylheptyloxy) iodobenzene were finally yielded.

7) 0.72 g of 3-n-decyloxy-6-ethynylpyridadine yielded under 3) and 1.06 g of optically active p-((S)-1-trifluoromethylheptyloxy) iodobenzene yielded under 6 were allowed to react overnight within 30 ml of triethylamine in the presence of 40 mg of bistriphenylphosphinpalladiumdichloride and 10 mg of cuprous iodide as catalysts under nitrogen atmosphere of room temperature. Upon completion of reaction, the reactant caused triethylamine to be removed for subsequent hexane removal. The hexane layer produced thereby was washed with hydrochloric acid solution in water, then with water for subsequent treatment through a silicagel-column. Through recrystallation repeated twice using ethanol, 0.53 g of

white crystalline compound No. 3b-1 under Table 3b proposed in the present invention was thus yielded. IR, H-NMR and F-NMR spectra of the compound are illustrated in Figs. 144, -145 and -146 respectively.

Result from elemental analysis:

| Calculated values of components(%) | Observed values of components(%) |
|---|---|
| C: 69.49<br>H: 7.92<br>N: 5.41<br>F: 11.00 | C: 69.25<br>H: 8.13<br>N: 5.43<br>F: 11.25 |

Example 72

Preparation of compound No. 3b-2 in the Tabel 3b

0.64 g of compound yielded under 3) of the Example 71 and 1.02 g of p-(1-trifluoromethylheptyloxycarbonyl) iodobenzene (obtainable through preparation of p-iodobenzoyl chloride from p-iodobenzoic acid using thionyl chloride followed by subsequent reaction with optically active 1-trifluoroheptanol) were allowed to react overnight within 30 ml of triethylamine in the presence of 36 mg of bistriphenylphosphinepalladiumdichloride and 9 mg of cuprous iodide as catalysts under nitrogen atmosphere at room temperature. Upon completion of reactive process, the reactant caused triethylamine to be removed for subsequent hexane extraction. The hexane layer produced thereby was washed with hydrochloric acid solution in water, then with water for subsequent treatment through a silicagel-column. Through recrystallization repeated twice using ethanol, 0.44 g of white crystalline compound No. 3b-2 under Table 3b proposed in the present invention were thus yielded. IR, H-NMR, and F-NMR spectra of the compound are illustrated in Figs. 147, 148 and 149 respectively.

Result from elemental analysis:

| Calculated values of components(%) | Observed values of components(%) |
|---|---|
| C: 68.13<br>H: 7.51<br>N: 5.13<br>F: 10.44 | C: 67.87<br>H: 7.63<br>N: 5.31<br>F: 10.58 |

Example 73

Preparation of compound No. 3b-12 in the Table 3b

1) 2.76 g of optically active (R)-1-trifluoromethylheptanol was allowed to drop onto 100 ml of dry diemethylformamide containing therein 0.36 g of sodium hydride at such a rate that hydrogen evolved not so vigorously at room temperature so as to obtain sodium salt of optically active (R)-1-trifluoromethylheptanol. Upon completion of dropping process the solution was kept stirred for subsequent 1 hr., added with 4.5 g of 3,6-dichloropyridazine and subjected to reflux for subsequent 1 hr. The reactant, upon cooled, was poured into ice water for subsequent hexane extraction. The hexane layer was washed with water, then with hydrochloric acid and again with water for subsequent separation and purification through a silicagel-column. 3.82 g of optically active, oily 3-((R)-1′-trifluoromethylheptyloxy)-6-chloropyridazine was thus yielded.

2) 350 ml of dimethylsulfoxide containing 34.0 g of p-iodophenol therein were added with sodium hydroxide solution in water (sodium hydroxide 7.4 g, water 50 ml) and stirred to a homogeneous solution. It was then added with 32.4 g of n-decylbromide and kept stirred for 3 days at room temperature. The reactant was poured into 1 ℓ of ice water for the subsequent hexane extraction to be repeated thrice. The hexane layer produced thereby was washed by water and caused hexane to be removed so as to finally yield 46.6 g of oily p-n-decyloxy-iodobenzene.

3) 30 g of p-n-decyloxyiodobenzene and 8.4 g of 3-methyl-1-butyen-3-ol were allowed to react overnight within 200 ml of triethylamine in the presence of 320 mg of bistriphenylphosphinepalladiumdichloride and 80 mg of cuprous iodide as catalysts under nitrogen atmosphere at room temperature Upon completion of reactive process the solution caused triethylamine to be removed for subsequent hexane extraction. The hexane layer produced thereby was washed by 1 N hydrochloric acid solution in water, then with water and caused hexane to be removed so as to finally yield 22.8 g of solid compound with a structure represented by the following formula.

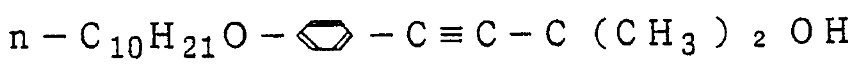

4) 400 ml of dry toluene containing 22.8 g of compound(yielded) under 3) were added with 9.9 g of sodium hydroxide in powder form and subjected to reflux for 1 hr. Upon completion of cooling process it was washed by water and then caused toluene to be removed. The black oil yielded thereby was subjected to methanol extraction, whereupon 13.2 g of oily p-n-decyloxyphenylacetylen was yielded after methanol had been removed therefrom.

5) 1.15 g of optically active 3-((R)-1′-trifluoromethylheptyloxy)-6-chloropyridazine yielded under 1) and 1.15 g of p-n-decyloxyphenylacetylene were subjected to reflux for 5 hrs. within 30 ml of triethylamine yielded under 4 in the presence of 44 mg of bis-triphenylphosphinepalladiumdichloride, 11 mg of cuprous iodide and 66 mg of triphenylphosphine as catalysts under nitrogen atmosphere. Upon completion of reaction, the reactant caused triethylamine to be removed for subsequent hexane extraction. The hexane layer was washed with hydrochloric acid solution in water, then with water for subsequent treatment through a silicagel-column. Through recrystallization repeated twice using ethanol, 0.63 g of compound No. 3b-12 under Table 3b proposed in the present invention was thus yielded. IR, H-NMR, and F-NMR spectra of the compound are illustrated in Figs. 150, 151 and 152 respectively.

Result from elemental analysis:

| Calculated values of components(%) | Observed values of components(%) |
|---|---|
| C: 69.49<br>H: 7.92<br>N: 5.41<br>F: 11.00 | C: 69.25<br>H: 8.04<br>N: 5.23<br>F: 11.13 |

Example 74

Preparation of compond No. 3d-12 in the Table 3d

1) 30 ml of ethanol containing therein 0.52 g of compound yielded of the Example 73 were added with 0.1 g of 5 % palladium-carbon for subsequent hydrogenation under hydrogen atmosphere of atmospheric pressure. Upon completion of reaction, the reactant caused catalyst to be removed through filtration and also ethanol to be removed. The reactant was dissolved in hexane. Through separation and purification using a silicagel-column 0.33 g of compound No. 3d-12 under Table 3d proposed in the present invention was thus yielded. IR, H-NMR and F-NMR spectra of the compund are illustrated in Figs 153, 154 and 155 respectively.

Result from elemental analysis:

| Calculated values of components(%) | Observed values of components(%) |
|---|---|
| C: 68.97<br>H: 8.62<br>N: 5.36<br>F: 10.92 | C: 69.12<br>H: 8.44<br>N: 5.51<br>F: 10.87 |

Example 75

Preparation of compound No. 4c-5 in the Table 4c

1) p-toluenesulfonic acid chloride solution in toluene (dry toluene 250 ml, p-toluenesulfonic acid chloride 130.0 g) was allowed to drop onto 250 ml pyridine with 50.0 g of optically active (S) -(-)-2-methyl-1-butanol dissolved therein under ice-cooled condition for the duration of 2 hrs. Upon completion of dropping process, the solution was allowed to be kept stirred for 27 hrs. with the temperature restored to room temperature. Upon completion of reaction, the solution was subjected to washing with water, then with 1 N hydrochloric acid solution in water, further with 1 N sodium hydroxide solution in water and again with water for subsequent removal of toluene. 128.0 g of optically active (s)-2-methylbutyl p-toluensulfonate was yielded.

2) 500 ml of dry ethanol with 40.0 g of o-fluoro-p-iodophenol and 41.0 g of optically active (s)-2-methylbutyl p-toluensulfonate dissolved therein were added with 10.4 g of potassium hydroxide for subsequent reflux for 7 hrs.

Upon completion of reaction, the solution caused ethanol to be removed by evaporation for subsequent toluene extraction. Through subsequent water washing of incidental toluene layer followed by removal of toluene, 50.0 g of oily optically active (s)-p-2-methylbutyloxy-m-fluoroiodobenzene was yielded.

3) 20.0 g of optically active (s)-p-2-methylbutyloxy-m-fluoroiodobenzene and 6.0 g of 3-methyl-1-butyne-3-ol were kept stirred for 7 hrs. within 150 ml of triethylamine in the presence of 137 mg of dichlorobistriphenylphosphinepalladium and 34 mg of cuprous iodide as catalysts under nitrogen atmosphere at room temperature. Upon completion of reaction, the solution caused triethylamine to be removed for subsequent hexane extraction. Through washing of hexane layer with hydrochloric acid sloution in water, and then with water followed by removal of hexane, 16.4 g of compound represented by the following formula were yielded.

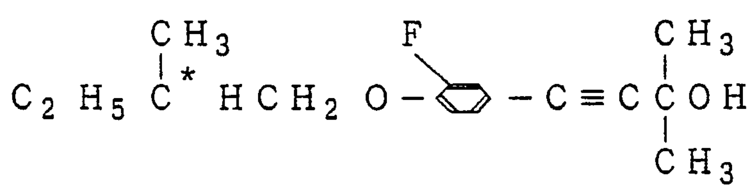

4) 350 ml of dry toluene with 16.4g of the said compound dissolved therein was added with 2.5 g of sodium hydroxide in powder form and subjected to reflux for 1 hr. The solution, upon cooled and washed with water, caused toluene to be removed. Through subsequent methanol extraction with the product of black colored oil followed by removal of methanol, 11.3 g of optically active, oily (s)-p-2-methylbutyloxy-m-fluorophenylacetylene were yielded.

5) 120 ml of Grignard reagent solution in dry ether prepared from 15.0 g of p-n-hexyloxy-m-fluoroiodobenzene and magnesium, and 120 ml of 13.2 g 2,5-dibromopyridine solution in dry tetrahydrofuran were kept stirred for 5 hrs. in the presence of 338 mg of dichlorobisdiphenylphosphinobutanepalladium as catalyst under nitrogen atomsphere and at room temperature. The sulution had then on-going reactive process quenched with water for subsequent ether extraction. The ether layer produced thereby was washed with 1 N hydrocholoric acid and then with water for

subsequent removal of ether. Through recrystallization of the solid matter produced using methanol, 9.0 g of 2-p-hexyloxy-m-fluorophenyl-5-bromopyridine was yielded.

6) 0.88 g of optically active, oily p-2-methylbutyloxy-m-fluorophenylacetylene obtained under 4) and 1.5g of 2-p-hexyloxy-m-fluorophenyl-5-bromopyridine obtained under 5) were kept stirred for 4 hrs. within 50 ml of triethylamine in the presence of 15 mg of dichlorobistriphenylphosphinepalladium, 4 mg of cuprous iodide and 23 mg of triphenylphosphine as catalysts under nitrogen atmosphere at reflux temperature. Upon completion of reaction, the solution caused triethylamine to be removed for subsequent extraction. The toluene layer obtained thereby was washed with 1 N hydrochloric acid solution in water, and then with water. Through purification through a silicagel-column followed by quadruplicate recrystallization using ethanol, 0.56 g of white crystalline compound No. 4c-5 under Table 4c as the compound proposed in the present invention was finally yielded. IR, H-NMR and F-NMR spectra of the compound are illustrated in Figs. 156, -157 and -158 respectively.

Result from elemental analysis:

| Calculated values of components(%) | Observed values of components(%) |
|---|---|
| C: 75.47<br>H: 6.92<br>N: 2.94<br>F: 7.97 | C: 75.31<br>H: 7.03<br>N: 2.81<br>F: 7.95 |

Example 76

Preparation of compound No. 4c-6 in the Table 4c

Same reaction as described of the example 75 took place here wherein used racemic-form 2-methyl-1-butanol intead of optically active (S) -(-)-2-methyl-1-butanol. The consequence was yielded compound No. 4c-6 under Table 4c.

Result from elemental analysis:

| Calculated values of components(%) | Observed values of components(%) |
|---|---|
| C: 75.47<br>H: 6.92<br>N: 2.94<br>F: 7.97 | C: 75.23<br>H: 7.12<br>N: 2.99<br>F: 8.03 |

Example 77

Preparation of compound No. 4d-5 in the Table 4d

1) 20 ml of ethanol with 0.25 g of compound No. 4c-5 under Table 4c obtained in the Example 75 dissolved therein were added with 0.1 g of 5% palladium-carbon and subjected to hydrogenation under hydrogen atmosphere of atmospheric pressure at reflux temperature. Upon comletion of reaction, the solution caused catalyst to be removed for subsequet removal of ethaol. Through subsequent dissolving in hexane followed by separation and purification through a silicagel-column, 0.19 g of No. 4d-5 under Table 4d as the compound proposed in the present invention was thus yielded. IR, H-NMR and F-NMR

spectra of the compound are illustrated in Figs. 159, -160 and -161 respectively.
Result from elemental analysis:

| Calculated values of components(%) | Observed values of components(%) |
|---|---|
| C: 74.85<br>H: 7.69<br>N: 2.91<br>F: 7.90 | C: 74.71<br>H: 7.73<br>N: 3.03<br>F: 8.11 |

Example 78

Preparation of compound No. 4g-1 in the Table 4g

1) 50 ml of dry ethanol with 5.0 g of 4-hydroxy-3-fluoro-4′-iodobiphenyl obtained under 1) to 4) of the Example 23, 3.8 g of optically active (s)-2-methylbutyl-p-toluenesulfonate and 1.1 g of potassium hydroxide dissolved therein were subjected to reflux for 22 hrs. Upon comletion of reaction, the solution caused ethanol to be removed for subsequent toluene extraction. Through subsequent washing with 1 N sodium hydroxide solution in water, and then with water; removal of toluene; and following recrystallization using methanol, 4.7 g of compound represented by the following formula were yielded.

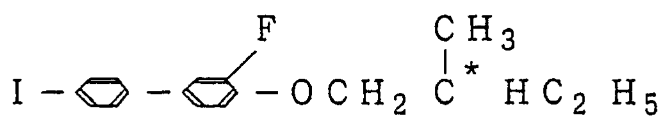

2) 1.5 g of the said compound and 0.9 g of p-n-hexyloxy-m-fluorophenylacetylene were kept stirred for 3 hrs. within 50 ml of triethylamine in the presence of 14 mg of dichlorobistriphenylphosphinepalladuim and 3 mg of cuprous iodide under nitrogen atmosphere at room temperature. Upon completion of reaction, the soltion caused triethylamine to be removed for subsequent toluene extraction. The toluene layer washed with 1 N hydrochloric acid solution in water, and then with water for subsequent purification through a silicagel-column. Through subsequent quadruplitate recrystallization using ethanol, 0.69 g of white crystalline compound No. 4g-1 under Table 4g as the compound proposed in the present invention was finally yielded. IR, H-NMR and F-NMR spectra of the compound are illustrated in Figs. 162, -163 and -164 respectively.
Result from elemental analysis:

| Calculated values of components(%) | Observed values of components(%) |
|---|---|
| C: 78.16<br>H: 7.14<br>F: 7.98 | C: 78.27<br>H: 7.01<br>F: 8.04 |

Example 79

Preparation of compound No. 4g-5 in the Table 4g

1) 0.78 g of optically active (s)-p-2-methylbutyloxy-m- fluorophenylacetylene and 1.50 g of 4-n-hexyloxy-3-fluoro-4′-iodobiphenyl were kept stirred for 3 hrs. within 50 ml of triethylamine in the presence of 14 mg of dichlorobistriphenylphosphinepalladium and 3 mg of cuprous iodide as catalysts under nitrogen atmosphere at room temperature. Upon completion of reaction, the solution caused triethylamine to be removed for subsequent toluene extraction. The toluene layer was washed with 1 N hydrochloric acid solution in water, and then with water for subsequent purification through a silicagel-column. Through subsequent quadruplicate recrystallization using ethanol, 0.68 g of white crystalline compound No. 4g-5 under Table 4g as the compound proposed in the present invention was finally yielded. IR, H-NMR and F-NMR spectra of the compound are illustrated in Figs. 165, -166 and -167 respectively.

Result from elemental analysis:

| Calculated values of components(%) | Observed values of components(%) |
|---|---|
| C: 78.16<br>H: 7.14<br>F: 7.98 | C: 78.09<br>H: 7.23<br>F: 8.12 |

Example 80

Preparation of compound No. 4g-6 in the Table 4g

In the essentially same way as was in the Example 79, compound No. 4g-6 under Table 4g was yielded using 2-methyl-1-butanol as racemic compound instead of optically active (s)-2-methyl-1-butanol.

Phase transition temperatures of those compounds obtained in the Example 1 to 67 as well as Example 75 to 80 are disclosed below in the Table 5.

Table 5 (1)

| EXAMPLE No. | The phase transition temperatures (℃) Cr | $S_I$ | Sc (Sc*) | $S_A$ | N | I |
|---|---|---|---|---|---|---|
| 1 | +35.1 | - | +38.0 | +43.7 | +45.0 | + |
| 2 | +26.1 | - | +27.5 | - | +41.6 | + |
| 3 | +27.1 | - | +29.7 | +33.3 | +40.8 | + |
| 4 | +26.4 | - | +35.9 | +40.9 | +46.5 | + |
| 5 | +47.5 | - | (+39.4) | (+46.8) | +47.6 | + |
| 6 | +26.8 | - | +40.9 | +51.2 | - | + |
| 7 | +27.4 | - | +31.7 | +35.1 | +40.9 | + |
| 8 | +38.4 | - | (+36.1) | +39.4 | +41.4 | + |
| 9 | +35.9 | - | +40.8 | +45.7 | +46.4 | + |
| 10 | +46.3 | - | (+42.6) | +47.3 | - | + |
| 11 | +38.5 | - | +45.5 | +51.1 | - | + |
| 12 | +34.5 | - | +38.8 | +41.8 | +46.4 | + |
| 13 | +37.0 | - | +41.1 | +44.6 | +46.3 | + |
| 14 | +37.4 | - | +46.2 | +51.5 | - | + |
| 15 | +47.9 | - | (+47.7) | +51.8 | - | + |
| 16 | +33.1 | - | +39.1 | +45.0 | - | + |
| 17 | +39.1 | - | +44.9 | +49.8 | - | + |
| 18 | +47.5 | +57.6 | - | +69.0 | - | + |
| 19 | +43.9 | - | - | - | +44.5 | + |
| 20 | +65.5 | - | - | (+60.5) | +66.6 | + |
| 21 | +48.3 | (+39.7) | - | (+39.8) | - | + |
| 22 | +51.7 | - | (+48.5) | - | +52.6 | + |
| 23 | +127.9 | - | +140.5 | +184.5 | - | + |
| 24 | +81.6 | - | - | +102.6 | +110.3 | + |
| 25 | +74.8 | (+54.5) | - | +93.5 | +109.5 | + |
| 26 | +66.9 | +103.2 | - | +121.9 | - | + |
| 27 | +100.8 | - | (+92.7) | - | - | + |
| 28 | +106.0 | - | (+105.5) | - | - | + |
| 29 | +79.3 | - | +84.5 | - | - | + |
| 30 | +77.4 | - | +97.9 | - | +100.4 | + |
| 31 | +83.0 | - | +106.1 | - | - | + |
| 32 | +90.7 | - | +112.6 | - | - | + |
| 33 | +141.2 | - | +200.0 | - | +201.0 | + |
| 34 | +133.4 | - | - | - | (+131.9) | + |
| 35 | +113.1 | - | - | +140.3 | - | + |
| 36 | +48.1 | - | (+41.3) | - | +54.2 | + |
| 37 | +54.3 | - | (+41.2) | - | +73.4 | + |
| 38 | +42.7 | - | (+36.2) | - | +51.9 | + |
| 39 | +81.0 | - | - | - | - | + |
| 40 | +153.1 | - | (+138.6) | - | +172.5 | + |

Table 5 (2)

| EXAMPLE No. | The phase transition temperatures (℃) Cr | $S_1$ | Sc (Sc*) | $S_A$ | N | I |
|---|---|---|---|---|---|---|
| 41 | +100.6 | − | − | − | +119.0 | + |
| 42 | +70.4 | − | +121.3 | − | +167.0 | + |
| 43 | +74.5 | (+58.7) | +92.7 | − | +102.9 | + |
| 44 | +49.0 | (+46.9) | +63.9 | − | +77.7 | + |
| 45 | +46.0 | − | (+30.4) | +62.0 | − | + |
| 46 | +101.7 | − | +171.7 | − | +188.6 | + |
| 47 | +93.2 | +118.2 | +155.6 | − | +214.0 | + |
| 48 | +104.2 | − | +158.5 | − | +183.2 | + |
| 49 | +93.4 | +120.1 | +183.1 | − | +209.5 | + |
| 50 | +71.5 | − | +121.1 | +156.8 | − | + |
| 51 | +149.5 | − | +164.4 | +175.9 | +185.4 | + |
| 52 | +100.0 | +150.0 | +156.4 | − | +189.9 | + |
| 53 | +123.5 | − | +125.1 | +134.2 | +153.0 | + |
| 54 | +155.9 | − | +182.3 | +200.5 | +211.0 | + |
| 55 | +68.5 | − | − | +128.7 | − | + |
| 56 | +152.2 | − | +204.0 | − | +209.6 | + |
| 57 | +133.7 | − | +137.7 | +158.0 | − | + |
| 58 | +45.8 | − | +46.8 | − | − | + |
| 59 | +46.9 | +51.5 | − | − | − | + |
| 60 | +44.5 | − | +51.7 | − | − | + |
| 61 | +38.4 | − | +53.8 | − | − | + |
| 62 | +38.6 | − | +59.5 | − | − | + |
| 63 | +29.6 | +96.0 | − | − | − | + |
| 64 | +46.4 | +83.0 | − | − | − | + |
| 65 | +85.1 | − | +105.6 | − | − | + |
| 66 | +68.0 | − | +94.3 | − | − | + |
| 67 | +49.9 | − | +91.3 | +95.0 | − | + |
| 75 | +87.1 | − | +150.0 | − | +161.5 | + |
| 76 | +86.8 | − | +150.5 | − | +161.9 | + |
| 77 | +31.8 | − | +90.0 | +121.3 | − | + |
| 78 | +138.6 | − | − | − | +152.5 | + |
| 79 | +136.6 | − | +143.9 | +157.2 | +160.1 | + |
| 80 | +136.8 | − | +143.5 | +156.9 | +160.5 | + |

Following symbols used in the table 5 under the following examples denote the respective phase enumerated hereunder;

Cr : Crystal phase

$S_1$ : Unidentified smectic phase

Sc* : Smectic C phase

$S_A$ : Smectic A phase

N : Nematic phase

I : Isotropic fluid phase

+ : existance

− : no existance

( ) : Monotropic phase

Chiral smectic C liquid crystal compositions (B) to (H) were obtained when the following smectic C liquid crystal composition (A) was added with optically active compound obtained in the Example 68 to 74 equivalent to 5 wt % thereof.

These compositions essentially displayed chiral smectic C phase at room temperature.

Each composition was injected into cell of 2µm thickness, provided with transparent electrodes, which had been coated with polyvinyl alcohol and surface-aligned by rubbing. Each device was placed between

two crossed polarizers, and response time was measured from change of intensity of transmitted light when voltage of ±10V was applied. The results obtained are shown in Table 6.

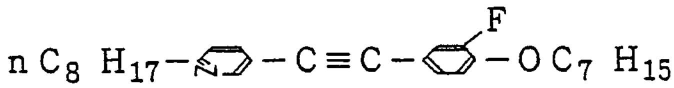

20wt%

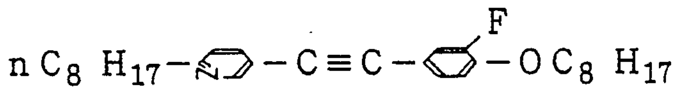

30wt%

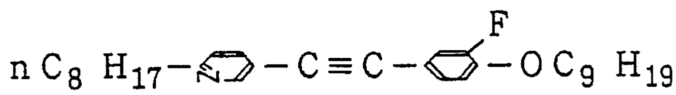

20wt%

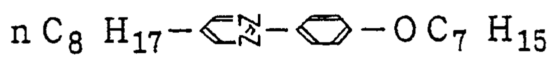

10wt%

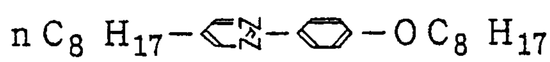

10wt%

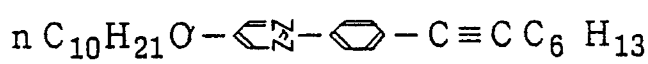

6wt%

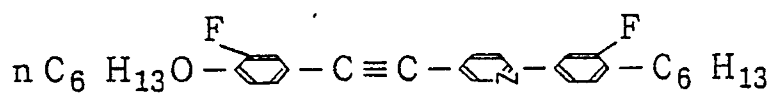

4wt%

Table 6

| EXAMPLE No. | 68 | 69 | 70 | 71 | 72 | 73 | 74 |
|---|---|---|---|---|---|---|---|
| Response Time (μ sec.) | 200 | 160 | 180 | 170 | 210 | 250 | 190 |

Comparative Example 1

The said smectic C liquid composition (A) was added with the following compound which is equivalent to the one under the Example 64, of which trifluoromethyl group is substituted by methyl group, in a quantity equivalent to 5 weight % of (A) so as to obtain chiral smectic C liquid crystal composition (I).

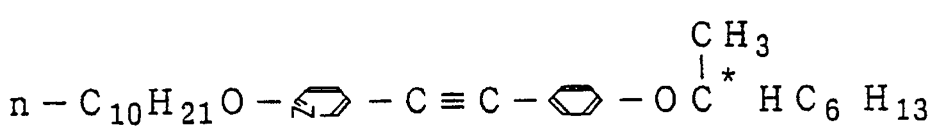

(I)

The last mentioned composition was transfused into the said cell, so that the response time may be determined. The time measured to approxmately 450μsec.

## Claims

1. A compound of one of the following formulae (1) or (2);

$R_1$ $-X_1$ $-A_1$ $-(A_2)_n$ $-Y_1$ $-A_3$ $-X_2$ $-R_2$ (1)

$R_1$ $-X_1$ $-(A_3$ $-Y_1)_n$ $-A_1$ $-A_2$ $-X_2$ $-R_2$ (2)

wherein $R_1$ and $R_2$ denote the same or different alkyl groups containing 1 to 18 carbon atoms, in which one or two $-CH_2$ -groups of the alkyl group may be substituted by oxygen atoms, sulfur atoms or $-CH=CH-$, provided that 2 oxygen atoms or 2 sulfur atoms are not directly bonded together; $X_1$ and $X_2$ denote a direct single bond, -O-, -S-, $-C \equiv C-$, -COO-, -OCO-. $-CH_2O-$ or $-OCH_2-$;
$Y_1$ denotes $-CH_2-CH_2-$ or $-C \equiv C-$; $A_1$ denotes: 1,4-phenylene group which may be substituted by 1 to 4 fluorine atoms, 1 or 2 chlorine atoms, 1 or 2 bromine atoms, 1 or 2 cyano groups, 1 or 2 nitro groups of 1 or 2 trifluoromethyl groups;

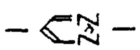, 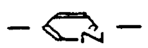, 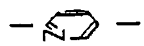, or 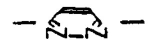 ;

$A_2$ denotes 1,4-phenylene group which may be substituted by 1 to 4 fluorine atoms, 1 or 2 chlorine atoms, 1 or 2 bromine atoms, 1 or 2 cyano groups, 1 or 2 nitro groups or 1 or 2 trifluoromethyl groups;
$A_3$ denotes: 1,4-phenylene group which may be substituted by 1 to 4 fluorine atoms, 1 or 2 chlorine atoms, 1 or 2 bromine atoms, 1 or 2 cyano groups, 1 or 2 nitro groups or 1 or 2 trifluoromethyl groups; 4,4'-biphenylene group (of which 1 or 2 CH groups may be substituted by nitrogen atoms); or 2,6-naphthylene group; and n is 0 or 1.

2. A compound according to claim 1, wherein $X_1$ is a single bond, -O- or $-C \equiv C-$.

3. A compound according-to claim 1 or 2, wherein $X_2$ is a single bond, -O- or $-C \equiv C-$.

4. A compound according to any preceding claim of formula (1), wherein $A_1$ is

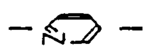.

5. A compound according to any of claims 1 to 3 of formula (1), wherein $A_1$ is

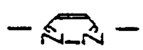.

6. A compound according to any of claims 1 to 3 of formula (1), wherein $A_1$ is

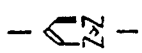.

7. A compound according to any of claims 1 to 3 of formula (1), wherein $A_1$ is 1,4-phenylene group which may be substituted by 1 to 4 fluorine atoms.

8. A compound according to any of claims 1 to 3 of formula (1), wherein $A_1$ is

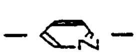.

9. A compound according to any preceding claim or formula (1), wherein n is 0 and $A_3$ is 1,4-phenylene group which may be substituted by 1 to 4 fluorine atoms.

10. A compound according to any of claims 1 to 8 of formula (1), wherein n is 0 and $A_3$ is 4,4'-biphenylene group which may be substituted by 1 to 4 fluorine atoms.

11. A compound according to any of claims 1 to 8 of formula (1), wherein n is 0 and $A_3$ is 2,6-naphthylene group.

12. A compound according to any of claims 1 to 9 of formula (1), wherein n is 1, and $A_1$ and $A_3$ are 1,4-phenylene groups which may be substituted by 1 to 4 fluorine atoms.

13. A compound according to claim 1, 7 or 9 of formula (1), wherein n is 0; $A_1$ and $A_3$ are 1,4-phenylene groups which may be substituted by 1 to 4 fluorine atoms: and $X_1$ and $X_2$ are -O-, -COO- or -OCO-.

14. A compound according to claim 1, 2 or 3 of formula (2), wherein $A_1$ is

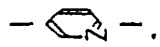

15. A compound according to claim 1, 2 or 3 of formula (2), wherein $A_1$ is

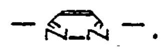

16. A compound according to claim 1, 2 or 3 of formula (2), wherein $A_1$ is 1,4-phenylene group which may be substituted by 1 to 4 fluorine atoms.

17. A compound according to claim 1, 2 or 3 of formula (2), wherein $A_1$ is

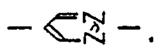

18. A compound according to any of claims 1, 2, 3, or 14 to 17 of formula (2), wherein n is 1 and $A_3$ is 1,4-phenylene group which may be substituted by 1 to 4 fluorine atoms.

19. A compound according to any of claims 1, 2, 3 or 14 to 18 of formula (2), wherein n is 1 and $A_3$ is 1,4-biphenylene group which may be substituted by 1 to 4 fluorine atoms.

20. A compound according to any of claims 1, 2, 3 or 14 to 19, wherein n is 0 and one of $X_1$ and $X_2$ is $-C \equiv C-$.

21. A compound of one of the following formulae (3) or (4);

$R_3 -X_1 -A_4 -Y_1 -A_3 -X_2 -R_4$ (3)

$R_3 -X_1 -A_5 -Y_1 -A_1 -A_2 -X_2 -R_4$ (3)

wherein $R_3$ and $R_4$ denote the same or different alkyl groups containing 1 to 18 carbon atoms, at least one of $R_3$ or $R_4$ preferably having asymmetric carbon atoms, in which one or two $-CH_2-$ groups of the alkyl group may be substituted by oxygen atoms, sulfur atoms and -CH=CH-, provided that 2 oxygen atoms or 2 sulfur atoms are not directly bonded together;

$X_1$ and $X_2$ denote a direct single bond, -O-, -S-, $-C \equiv C-$, -COO-, -OCO-, $-CH_2O-$ or $-OCH_2-$; $Y_1$ denotes $-CH_2-CH_2-$ or $-C \equiv C-$;

$A_1$ denotes: 1,4-phenylene group which may be substituted by 1 to 4 fluorine atoms, 1 or 2 chlorine atoms, 1 or 2 bromine atoms, 1 or 2 cyano groups, 1 or 2 nitro groups or 1 or 2 trifluoromethyl groups;

$A_2$ and $A_5$ denote 1,4-phenylene group which may be substituted by 1 to 4 fluorine atoms, 1 or 2 chlorine atoms, 1 or 2 bromine atoms, 1 or 2 cyano groups, 1 or 2 nitro groups or 1 or 2 trifluoromethyl groups;

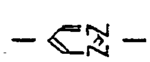 , 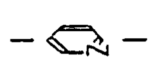 , 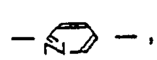 , or 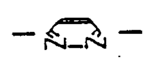 ;

$A_3$ denotes: 1,4-phenylene group which may be substituted by 1 or 4 fluorine atoms, or 1 or chlorine atoms, 1 or 2 bromine atoms, 1 or 2 cyano groups, 1 or 2 nitro groups or 1 or 2 trifluoromethyl groups; 4,4′-biphenylene group, of which 1 or 2 CH groups may be substituted by nitrogen atoms; or 2,6-naphthylene-group;

$A_4$ denotes

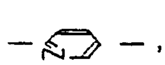 , 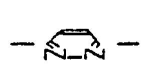 or 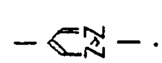

22. A compound according to claim 21, wherein $X_1$ is a direct single bond, -O-, -COO- or $-C \equiv C-$

23. A compound according-to claim 21 or 22, wherein $X_2$ is a direct single bond, -O-, -COO-, $-C \equiv C-$ or $-CH_2O-$.

24. A compound according to any of claims 21 to 23 of formula (3), wherein $A_4$ is

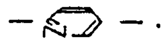

25. A compound according to any of claims 21 to 23 of formula (3), wherein $A_4$ is

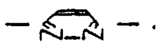

26. A compound according to any of claims 21 to 25 of formula (3), wherein $A_3$ is 1,4-phenylene group which may be substituted by 1 to 4 fluorine atoms.

27. A compound according to any of claims 21 to 26 of formula (4), wherein $A_1$ is 1,4-phenylene group which may be substituted by 1 to 4 fluorine atoms.

28. A compound according to any of claims 21 to 26 of formula (4), wherein $A_1$ is

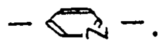

29. A compound according to any of claims 21 to 28 of formula (4), wherein $A_5$ is 1,4-phenylene group which may be substituted by 1 to 4 fluorine atoms.

30. A compound according to any of claims 21 to 28 of formula (4), wherein $A_2$ is 1,4-phenylene group which may be substituted by 1 to 4 fluorine atoms.